# EUROPEAN PATENT SPECIFICATION

(11) **EP 0 578 388 B1**
(45) Date of publication and mention of the grant of the patent: **04.11.1998**
(21) Application number: 93304599.9
(22) Date of filing: 14.06.1993
(51) Int. Cl.: C12P 17/04, C12P 7/64, C12N 9/20, C12N 1/20

(54) **Fermentation process for preparing 10-hydroxy-C18-carboxylic acid and gamma-dodecalactone derivatives**
Verfahren zur fermentativen Herstellung von 10-Hydroxy-C18-Carbonsäure und Gamma-Dodekalaktonderivaten
Procédé de fermentation pour la production de dérivés de l'acide 10-hydroxy-C18-carboxylique et du gamma dodecalactone

(30) Priority: 25.06.1992 US 904435
(43) Date of publication of application: 12.01.1994
(73) Proprietor: INTERNATIONAL FLAVORS & FRAGRANCES INC., New York New York 10019 (US)
(72) Inventor: Farbood, Mohamad I., Monmouth, New Jersey 07733 (US); Morris, James A., Monmouth, New Jersey 07728 (US); McLean, Lynda B., Monmouth, New Jersey 07747 (US)
(74) Representative: Brown, John David

(56) References cited:
- EP-A- 0 258 993
- EP-A- 0 371 568
- EP-A- 0 409 321
- EP-A- 0 412 880
- WO-A-83/01072
- JP-A- 3 198 787

## Description

### SUMMARY OF THE INVENTION

Our invention relates to a fermentation process for preparing 10-hydroxy-C₁₈-carboxylic acid derivative containing compositions and gamma-dodecalactone derivative-containing compositions, products produced thereby and organoleptic uses thereof. EP-A-258993 discloses a method for the production of gamma-dodecalactone by incubating a source of ricinoleic acid with sporobolomyces odorus or Rhodotorus glutinis. The reaction for preparing the 10-hydroxy-C₁₈-carboxylic acid derivative containing compositions concerns the carrying out of a fermentation of C₁₈-carboxylic acid derivatives defined according to the structure: 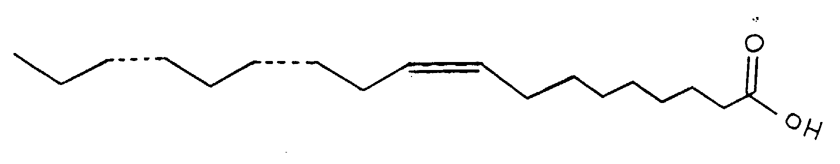 with the microorganism, Pseudomonas sp NRRL B-2994 or a 10-hydroxy-C₁₈-carboxylic acid derivative-producing mutant thereof for a period of time sufficient to produce one or more of such hydroxy carboxylic acids. Our invention also covers a process for carrying out the fermentation of such 10-hydroxy-C₁₈-carboxylic acid derivatives with microorganisms of the genus Candida and/or the genus Yarrowia or

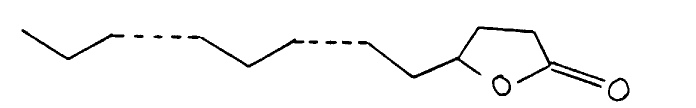

-producing mutants thereof for a period of time sufficient to produce one or more gamma-dodecalactone derivatives having the structure: 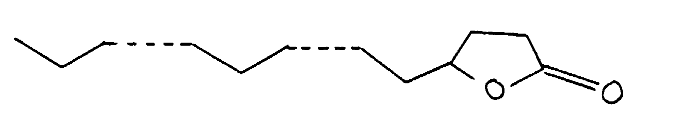 wherein the dashed lines represent the same or different carbon-carbon bonds; with each of the dashed lines representing a carbon-carbon single bond or a carbon-carbon double bond. Our invention also relates to products produced according to such processess as well as organoleptic uses of such products.

### DETAILED DESCRIPTION OF THE INVENTION

Our invention relates to a method using fermentation techniques to produce and recover certain naturally occurring C₁₂ lactones found to be useful for their organoleptic properties in augmenting or enhancing the aroma or taste of consumable materials such as foodstuffs, chewing gums, toothpastes, medicinal products, chewing tobaccos, smoking tobaccos, perfume compositions, colognes and perfumed articles, e.g., solid or liquid anionic, cationic, nonionic or zwitterionic detergents, perfumed polymers, fabric softener compositions, fabric softener articles, cosmetic powders, hair preparations and the like which lactones are defined according to the generic structure: 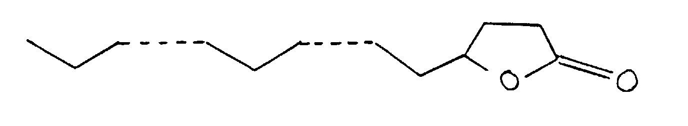 wherein the dashed lines represent the same or different carbon-carbon bonds; with each of the dashed lines representing a carbon-carbon single bond or a carbon-carbon double bond.

The lactone compositions of our invention preferably contain one or more of the following lactone compounds: 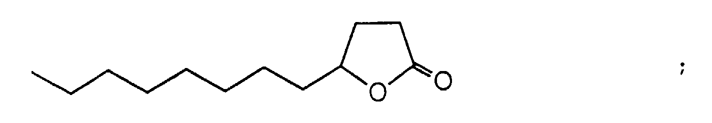 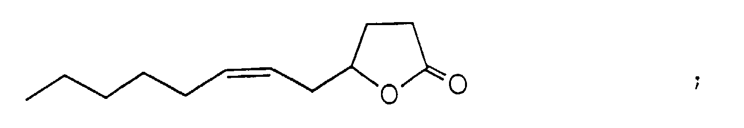 and/or

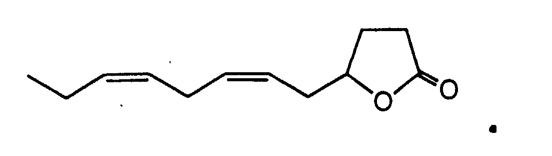

Our invention is also directed to processes for preparing precursor hydroxy carboxylic acid-containing compositions useful for, inter alia, the production of the above-mentioned lactone compositions. Such hydroxy carboxylic acid compositions are defined according to the generic structure: 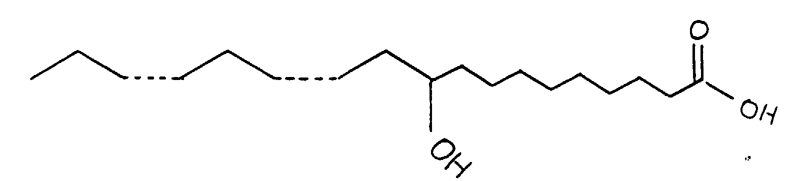 wherein the dashed lines represent the same or different carbon-carbon bonds; with each of the dashed lines representing a carbon-carbon single bond or a carbon-carbon double bond.

The hydroxy-carboxylic acid-containing compositions of our invention are produced by means of fermentation of one or more carboxylic acids defined according to the generic structure: 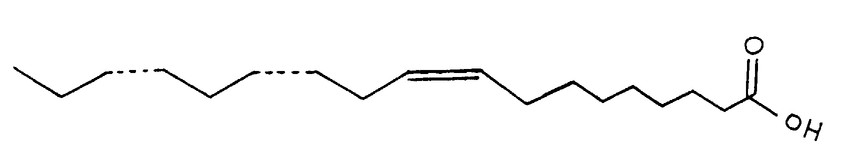 wherein the dashed lines represent the same or different carbon-carbon bonds; with each of the dashed lines representing a carbon-carbon single bond or a carbon-carbon double bond using the microorganism Pseudomonas sp. NRRL B-2994 according to the reaction: 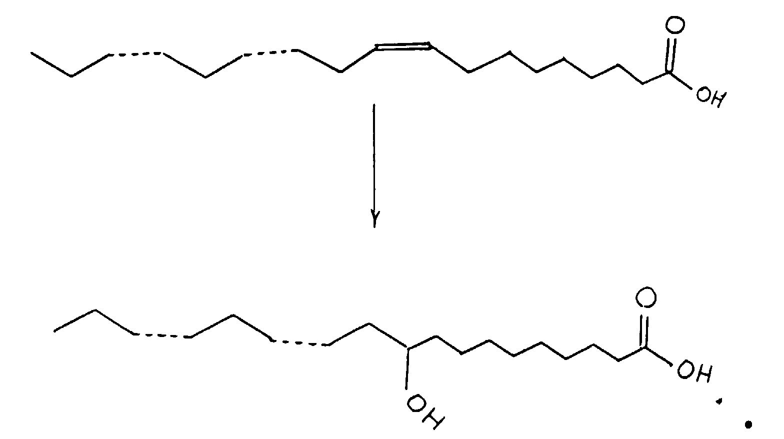 The fermentation reaction is carried out at a pH in the range of from about 5.5 up to about 9.0 with the most preferable pH range being from about 6.5 up to about 8.5. The reaction is carried out at a temperature of from about 20°C up to about 35°C and for a period of time of from about 48 hours up to about 150 hours. Triglycerides of such carboxylic acid reactants may be used in place of the free carboxylic acids with equally beneficial results. Thus, for example, triglyceride-containing oils such as sun flower oil (which contains a high percentage of oleic acid) may be utilized in creation of hydroxy acids such as those having the structures: 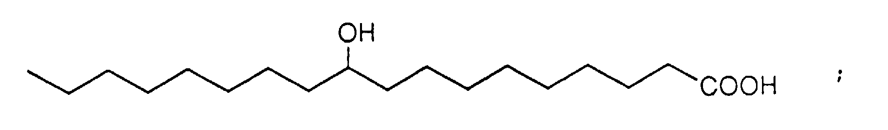 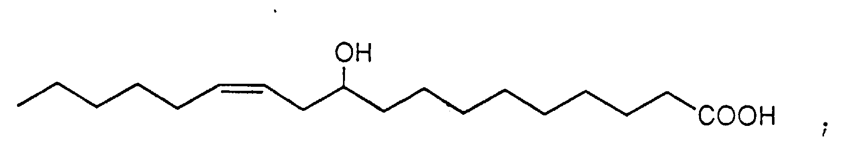 and/or

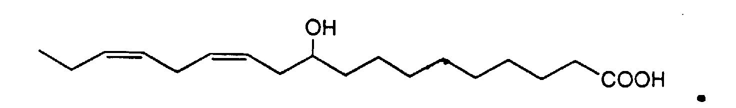

Such hydroxy carboxylic acids are produced according to the reactions: 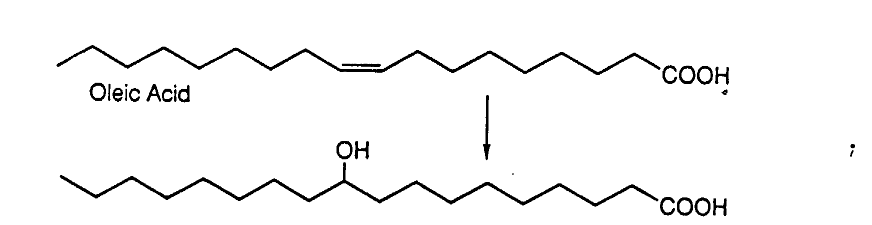 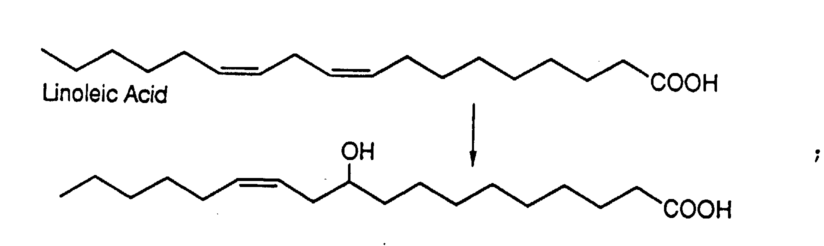 and

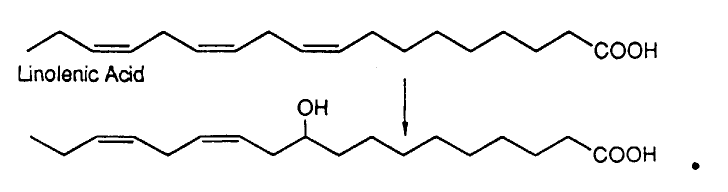

As stated, supra, the reaction, to wit: 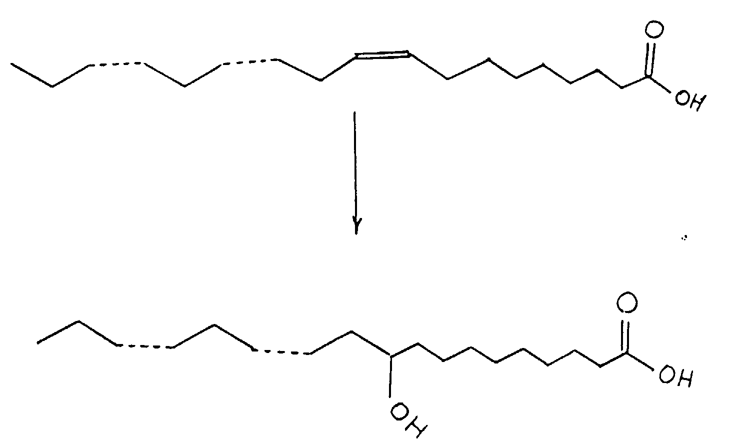 which will produce "Z,Z" isomers and which will produce optically active compounds is carried out using the microorganism Pseudomonas sp. NRRL B-2994 or a 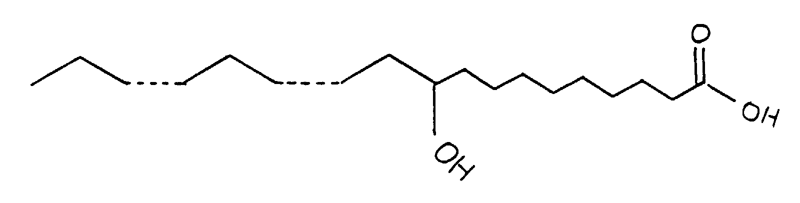 -producing mutant strain thereof. By the same token the triglyceride of the acid having the structure: 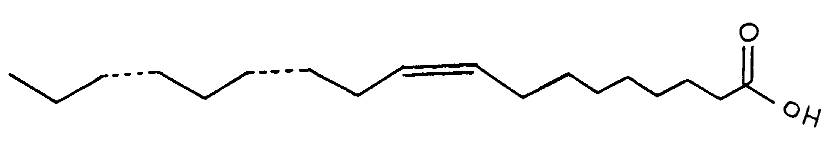 can also be reacted with such microorganism under the above conditions to yield the hydroxy carboxylic acids defined according to the generic structure:

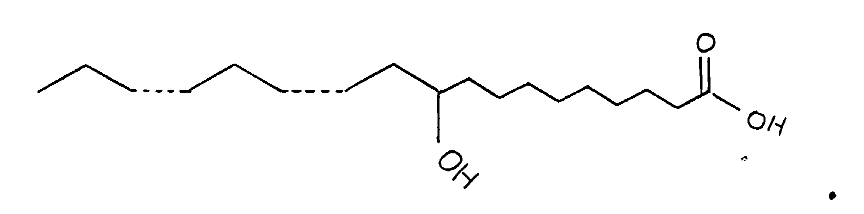

The lactone compositions having the generic structure: 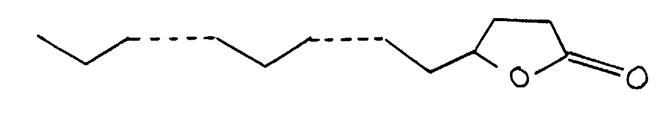 are produced from the hydroxy carboxylic acid compositions defined according to the generic structure: 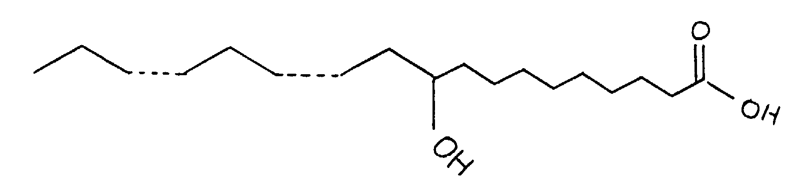 using a microorganism of the Candida genus or the Yarrowia genus. Specific examples of such microorganisms are as follows:
Candida brumptii IFO 0731;
Candida deformans ATCC 22969;
Candida ethanothermophilum ATCC 20380;
Candida iberica ATCC 26318;
Candida kefyr ATCC 42265;
Candida oleophila ATCC 20177;
Candida rugosa ATCC 14830;
Candida sp. ATCC 20473;
Candida sp. ATCC 20504;
Candida utilis ATCC 9226;
Candida utilis ATCC 15239;
Candida utilis ATCC 20248;
Candida utilis ATCC 42181;
Candida zeylanoides ATCC 15585;
Yarrowia lipolytica ATCC 8661;
Yarrowia lipolytica ATCC 8662;
Yarrowia lipolytica ATCC 16617;
Yarrowia lipolytica ATCC 16618;
Yarrowia lipolytica ATCC 20255;
Yarrowia lipolytica ATCC 20324;
Yarrowia lipolytica ATCC 20341;
Yarrowia lipolytica ATCC 46483;
Candida petrophilum ATCC 20226; and
Yarrowia lipolytica ATCC 34088;
according to the reaction: 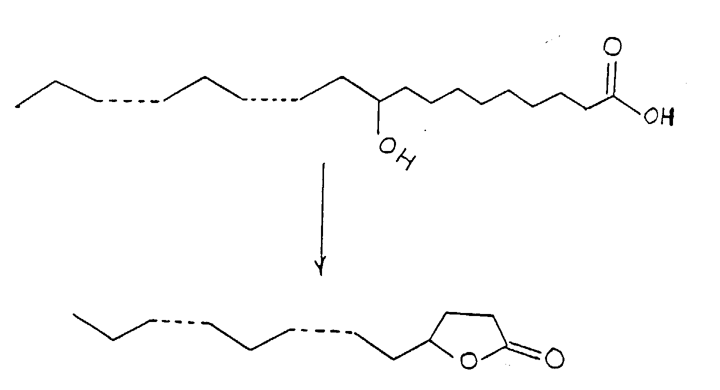 wherein the dashed lines represent the same or different carbon-carbon double bonds; with each of the dashed lines representing a carbon-carbon single bond or a carbon-carbon double bond. The reaction is carried out at a pH in the range of from about 5 up to about 7.5, with the most preferable pH range being from about 6.5 up to about 7.0. The reaction is carried out at a temperature in the range of from about 20°C up to about 35°C. The reaction is carried out for a time of from about 18 hours up to about 30 hours. The reaction is carried out with or without a "co-oxidant" which can be olive oil, coconut oil, palm oil or any other "co-oxidant" with which one having ordinary skill in the art is familiar. A preferred "co-oxidant" is olive oil. However, the reaction need not be carried out with such "co-oxidant" although the use of the "co-oxidant" is highly preferable.

Examples of the reaction shown generically, thusly: 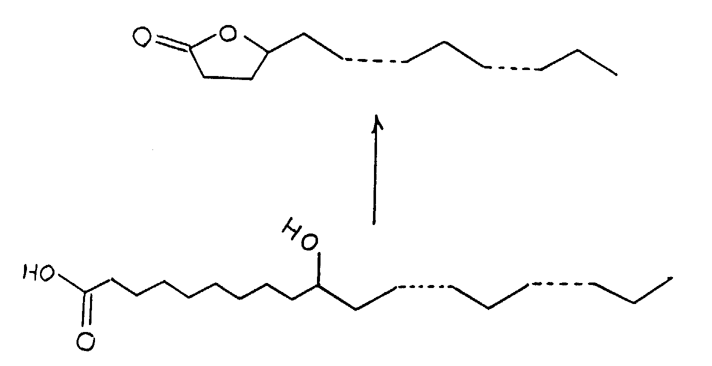 are as follows: 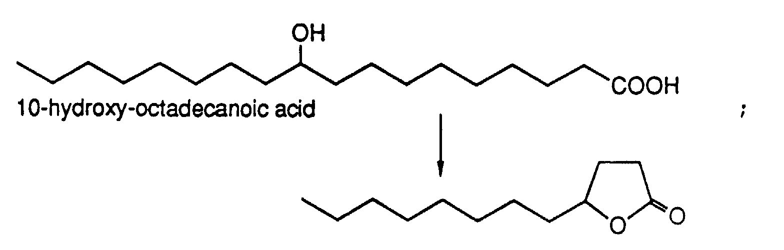 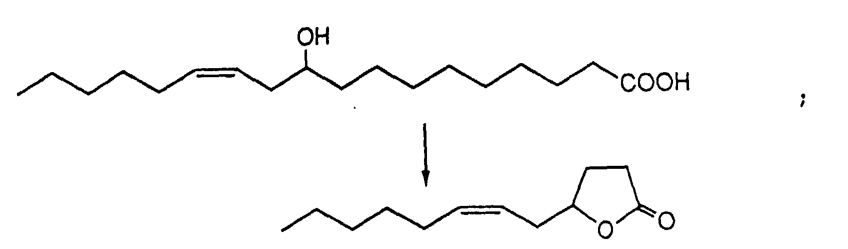 and 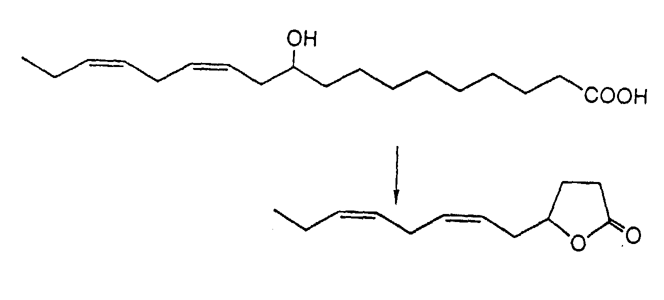 The foregoing reactions can be carried out within the same mixture, simultaneously or they can be carried out on an individual basis, using substantially pure hydroxy carboxylic acid reactants.

In addition to the microorganisms used for carrying out the reactions: 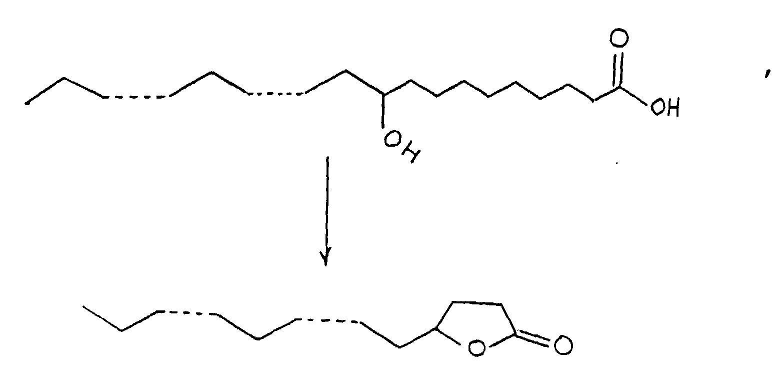 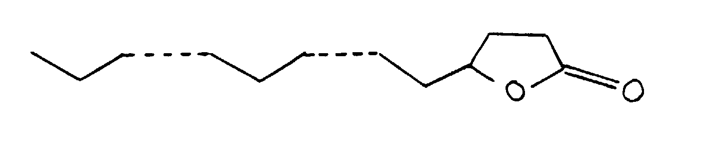 -producing mutant strains of such organisms can also be used, in many instances creating yields higher than that of the above-listed microorganisms.

The fermentation is terminated by adjusting the pH to a range of about 2 up to about 4 using such acid compositions as aqueous 50% sulfuric acid or citric acid. The fermentation broth is then heated to approximately 100°C for a period of between about 10 minutes and about 60 minutes. The broth is then extracted with a suitable solvent, e.g., ethyl acetate and the crude extract is then evaporated and fractionally distilled to yield a composition of matter containing a substantial quantity of at least one of the lactones defined according to the generic structure: 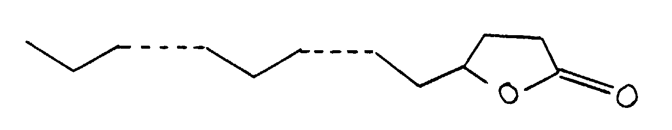 wherein the dashed lines represent the same or different carbon-carbon bonds; with each of the dashed lines representing a carbon-carbon single bond or a carbon-carbon double bond.

When producing the lactone compositions of our invention directly from the carboxylic acids, the reaction sequence is as follows: 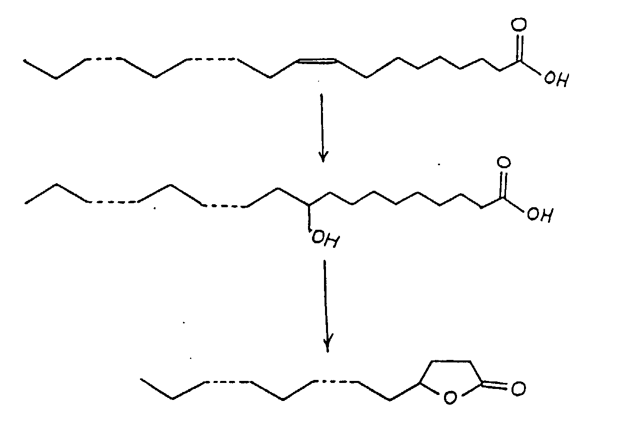 wherein the following reactions, for example, can be carried out either separately or in combination:

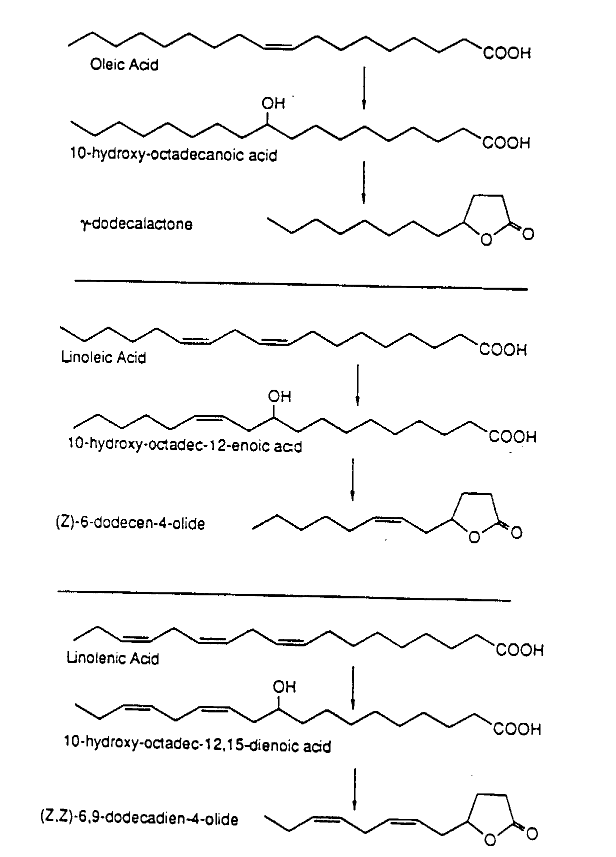

Prior to either of the foregoing reactions taking place, a culture suspension is prepared by inoculation of a suitable medium with the desired microorganism.

A suitable medium is one which contains carbon sources, nitrogen sources, inorganic salts and growth factors. Among the suitable carbon sources are, for example, glucose, galactose, L-sorbose, maltose, sucrose, cellobiose, trehalose, L-arabinose, L-rhamnose, ethanol, glycerol, L-erythritol, D-mannitol, lactose, melibiose, raffinose, melezitose, starch, D-xylose, D-sorbitol, alpha-methyl-D-glucoside, lactic acid, citric acid and succinic acid. Among the suitable nitrogen sources are, for example, nitrogen-containing organic substances such as peptone, meat extract, yeast extract, corn steep liquor, and casein, urea, amino acids, or nitrogen containing inorganic compounds such as nitrates, nitrites, and inorganic ammonium salts. Among the suitable inorganic salts are, for example, phosphates, magnesium, potassium, calcium, sodium. The above-mentioned nutrients in the culture medium may be supplemented with, for example, one or more vitamins of the B Group and/or one or more trace minerals such as Fe, Mo, Cu, Mn, B as described. However, the process can be performed in a vitamin-free medium, for example, when a small amount of yeast extract is added to the medium there is no need for vitamins or trace minerals.

The cultivation of the microorganism can be carried out as a stationary culture or as a submerged culture (e.g., shaking culture, fermentors) preferably under aerobic conditions. One suitably may work in the pH range of from about 3.5 to about 8.0, and preferably in the range of from about 4.0 to about 7.5. The pH may be regulated by addition of inorganic or organic bases, such as sodium hydroxide, potassium hydroxide, calcium hydroxide, calcium carbonate by ion-exchange resins, or by the additon of a buffer such as a phosphate. The incubation temperature is suitably maintained at between about 15°C and about 33°C, with a range from about 20°C to about 30°C being preferred.

In the production of the C₁₈-10-hydroxy carboxylic acids having the structure: 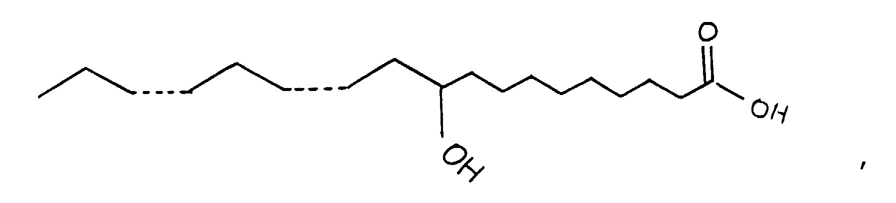 in place of using the carboxylic acids having the structure: 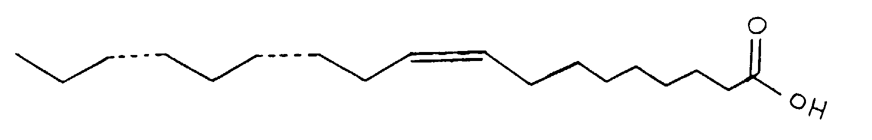 as precursors or as reactants, triglycerides thereof may be used having the structure: 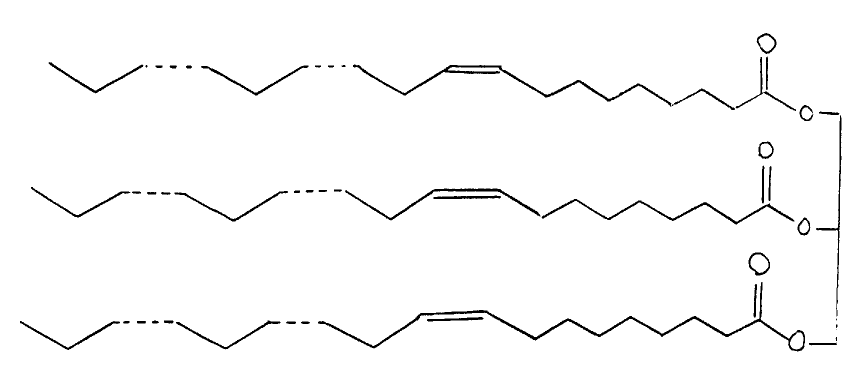 whereby such triglycerides may be reacted microbiologically using Pseudomonas sp NRRL B-2994 (as stated, supra) and also using Lipase according to the reaction: 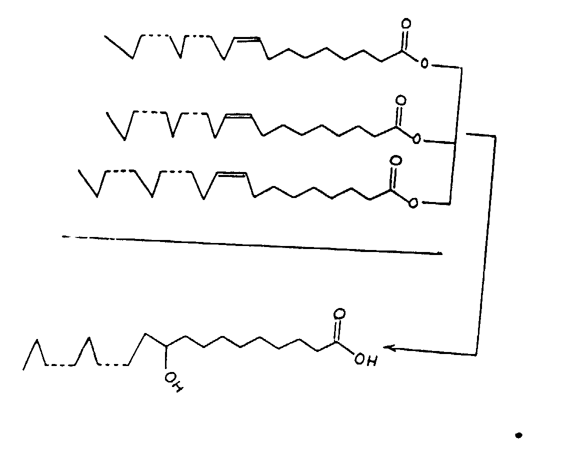 Thus, for example, the triglyceride having the structure: 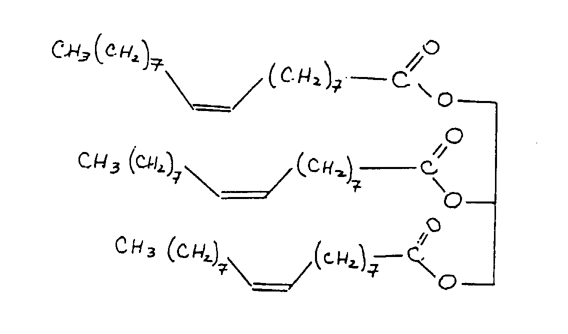 may be subjected to fermentation using Lipase MY and the Pseudomonas sp NRRL B-2994 (in accordance with the procedure of Example IX, infra), according to the reaction:

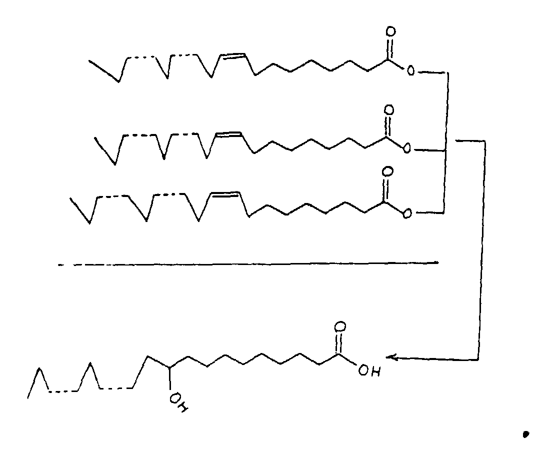

In the foregoing reactions the dashed lines represent the same or different carbon-carbon double bonds; with each of the dashed lines representing a carbon-carbon single bond or a carbon-carbon double bond. Thus, the triglycerides that can be used when the dashed lines are each carbon-carbon single bonds are, for example, sun flower oil (high oleic content). As desired, other triglyceride-containing oils may also be used.

The lactone derivative(s) and one or more auxiliary perfume ingredients, including, for example, hydrocarbons, alcohols, ketones, aldehydes, nitriles, esters other than the lactone derivatives of our invention ethers, synthetic essential oils, and natural essential oils may be admixed so that the combined odors of the individual components produce a pleasant and desired fragrance, particularly and preferably in the fruity area (e.g., peach and apricot aromas). Such perfume compositions usually contain (a) the main note or the "bouquet" or foundation stone of the composition; (b) modifiers which round off and accompany the main note, (c) fixatives which include odorous substances which lend a particular note to the perfume throughout all stages of evaporation and substances which retard evaporation; and (d) topnotes which are usually low-boiling, fresh-smelling materials.

In perfume compositions, it is the individual compositions which contribute to their particular olfactory characteristics, however, the overall sensory effect of the perfume composition will be at least the sum total of the effects of each of the ingredients. Thus, one or more of the lactone derivative(s) of our invention can be used to alter, modify or enhance the aroma characteristics of a perfume composition, for example, by utilizing or moderating the olfactory reaction contributed by another ingredient in the composition.

The amount of lactone derivative(s) of our invention which will be effective in perfume compositions as well as in perfumed articles and colognes depends upon many factors including the other ingredients, their amounts and the effects which are desired. It has been found that perfume compositions containing as little as 0.005% of lactone derivative(s) or even less (e.g., 0.002%) can be used to impart sweet, fruity (peach and apricot) aromas to soaps, cosmetics, detergents including solid or liquid anionic, cationic, nonionic or zwitterionic detergents, perfumed polymers and other products. The amount employed can range up to 70% of the fragrance components and will depend upon the consideration of cost, nature of the end product, the effect desired on the finished product and the particular fragrance sought.

The lactone derivative(s) of our invention are useful (taken alone or together with other ingredients in perfume compositions) in detergents, soaps, space odorants and deodorants, perfumes, colognes, toilet waters, bath preparations, hair preparations such as lacquers, brilliantines, pomades and shampoos; cosmetic preparations such as creams, deodorants, hand lotions and sun screens; powders such as talcs, dusting powders, face powders and the like.

As little as 0.25% of the lactone derivative(s) will suffice to impart an intense, sweet, fruity (peach and apricot) aroma to floral perfume formulations. Generally no more than 5% of the lactone derivative(s) based on the ultimate end product is required to be used in the perfume compositions.

Furthermore, as little as 0.25% of the lactone derivative(s) will suffice to impart such aromas to perfumed articles per se, whether in the presence of other perfume materials or whether used by themselves. Thus, the range of use of the lactone derivative(s) of our invention in perfumed articles, e.g., perfumed polymers and solid or liquid anionic, cationic, nonionic or zwitterionic detergents, may vary from 0.25% up to about 5% by weight based on the total weight of the perfumed article.

In addition, the perfume composition or fragrance composition of our invention can contain a vehicle or carrier for the lactone derivative(s). The vehicle can be a liquid such as a non-toxic alcohol, e.g., ethanol, a non-toxic glycol, e.g., propylene glycol, or the like. The carrier can also be an absorbent solid such as a gum (e.g., gum arabic or xanthan gum or guar gum) or components for encapsulating the composition by means of coacervation (such as by gelatin) or by means of formation of a polymer around a liquid center (as by using a urea formaldehyde prepolymer to form a polymeric capsule around a perfume composition center).

It will be appreciated from the present disclosure that the lactone derivatives according to the present invention can be used to alter, vary, fortify, modify, enhance or otherwise improve the flavor of a wide variety of materials which are ingested, consumed or otherwise organoleptically sensed.

### BRIEF DESCRIPTION OF THE DRAWINGS

Figure 1 is the GLC profile for the reaction product of Example I(a) produced by action of Candida brumpti IFO 0731 on 10-hydroxy stearic acid.

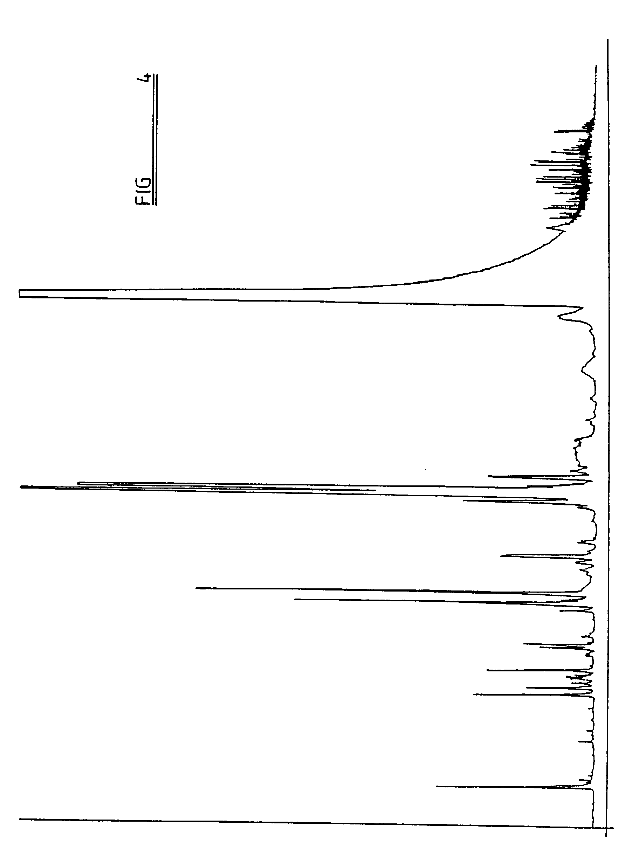

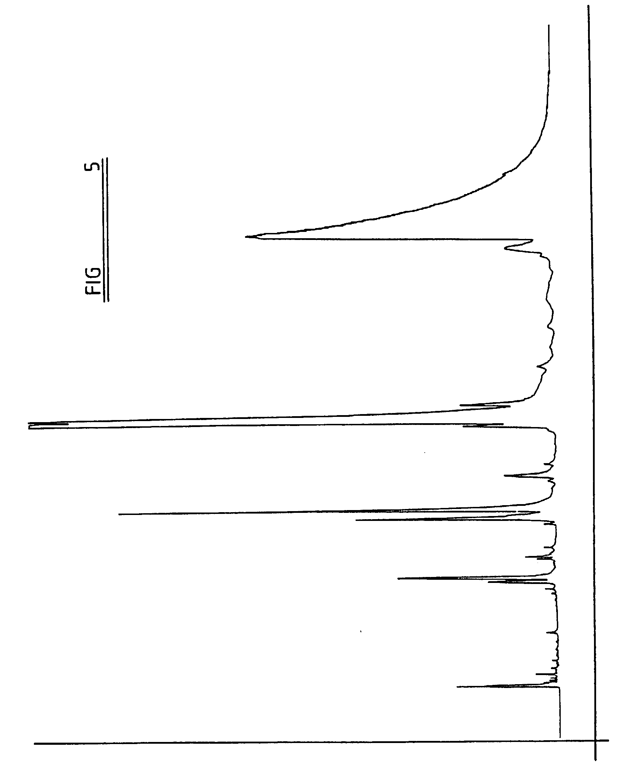

Figure 2 is the GLC profile for the reaction product of Example I(b) produced by action of Candida deformans ATCC 22969 on 10-hydroxy stearic acid.

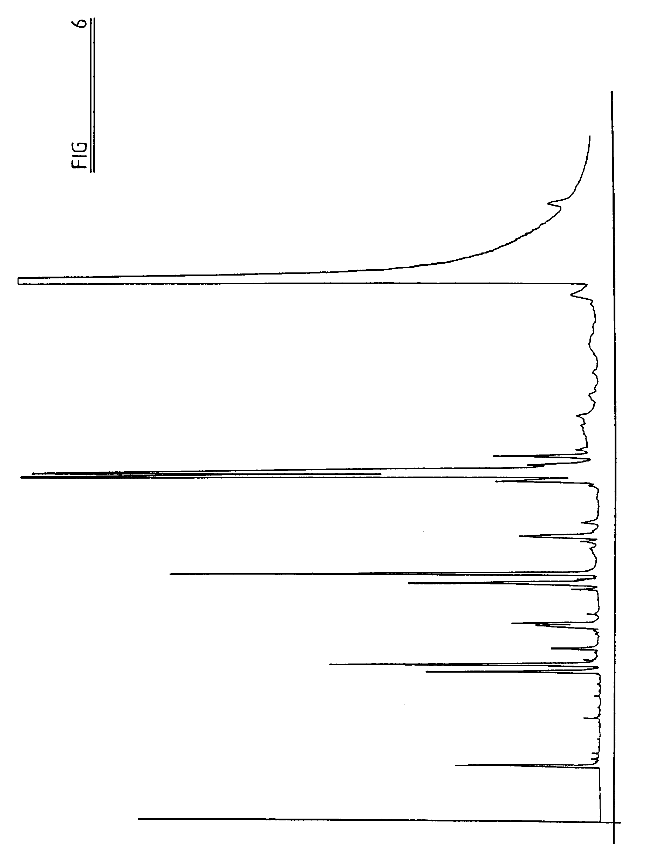

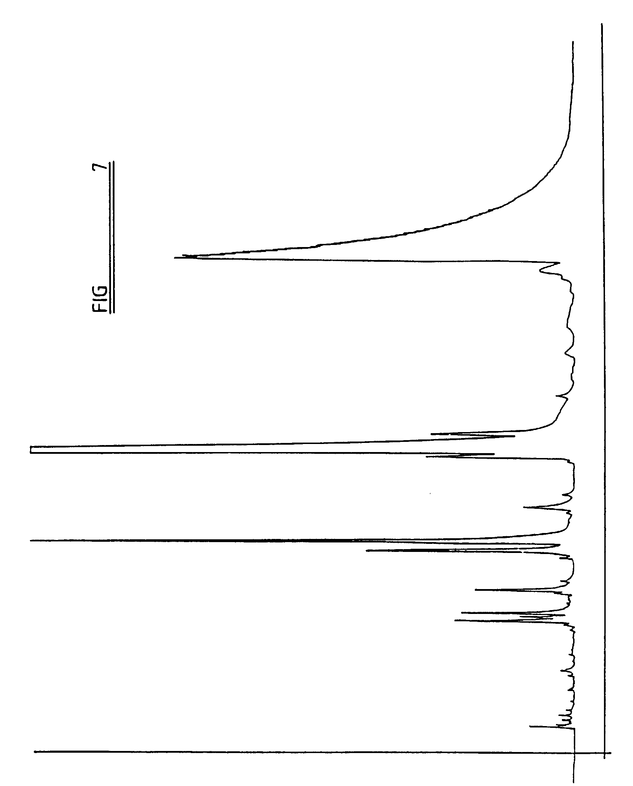

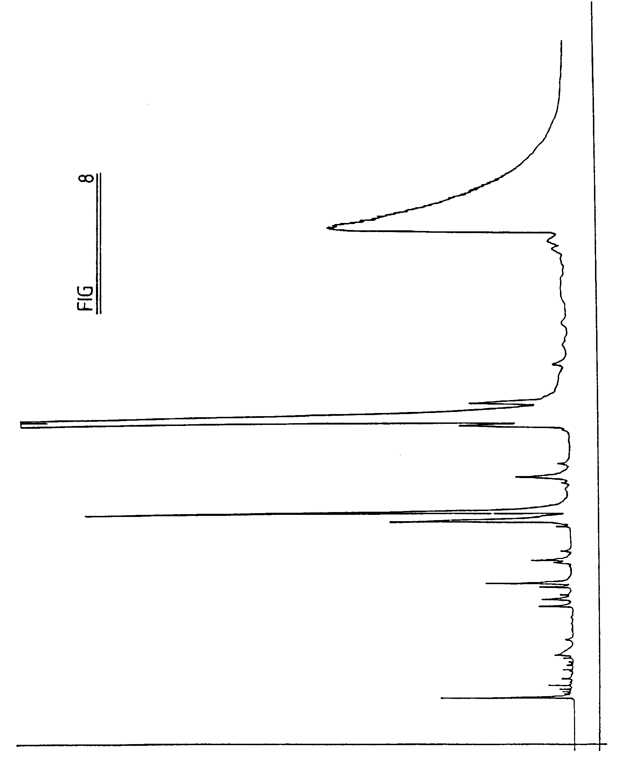

Figure 3 is the GLC profile for the reaction product of Example I(c) produced by action of Candida ethanothermophilum ATCC 20380 on 10-hydroxy stearic acid.

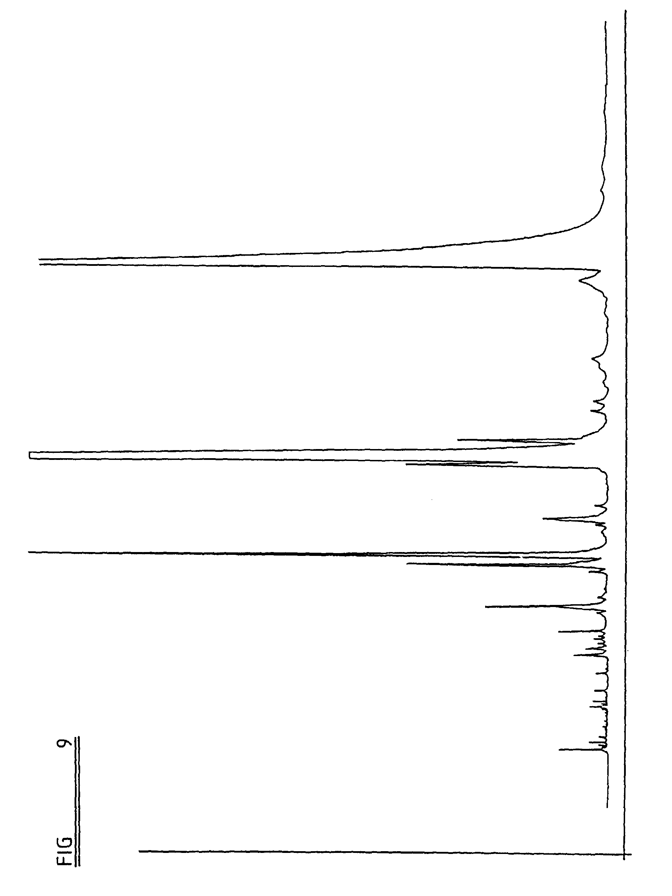

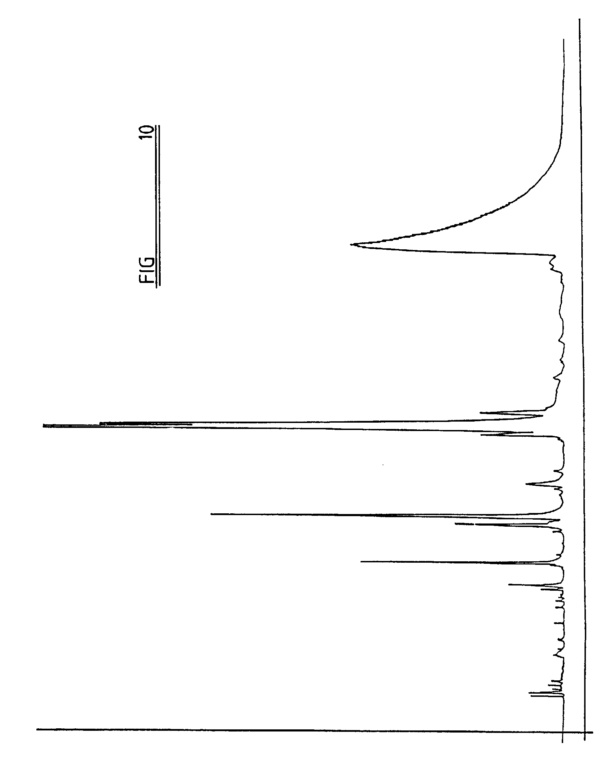

Figure 4 is the GLC profile for the reaction product of Example I(d) produced by action of Candida iberica ATCC 26318 on 10-hydroxy stearic acid.

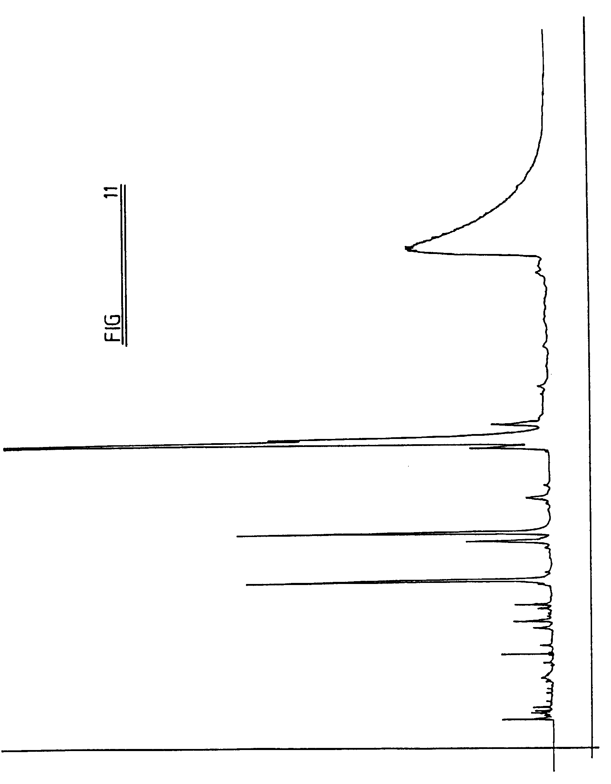

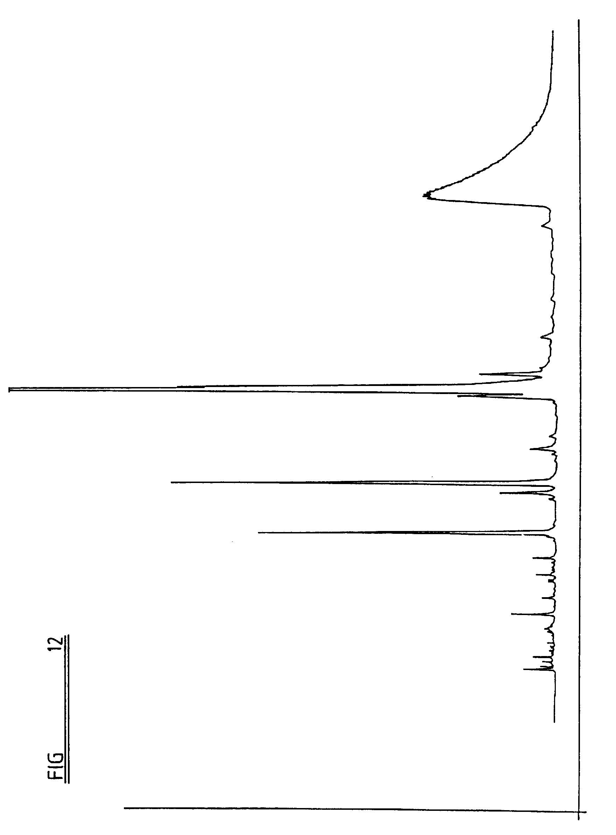

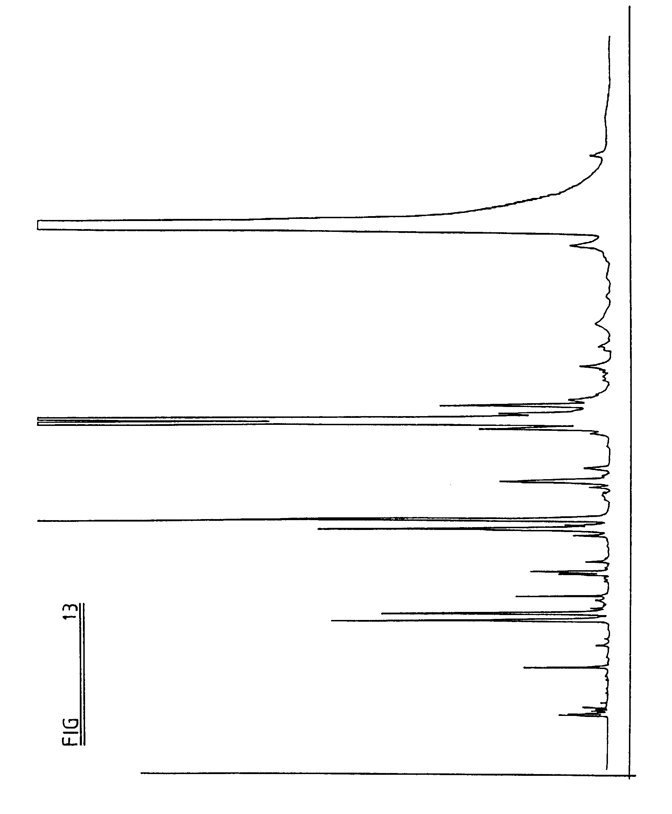

Figure 5 is the GLC profile for the reaction product of Example I(e) produced by action of Candida kefyr ATCC 42265 on 10-hydroxy stearic acid.

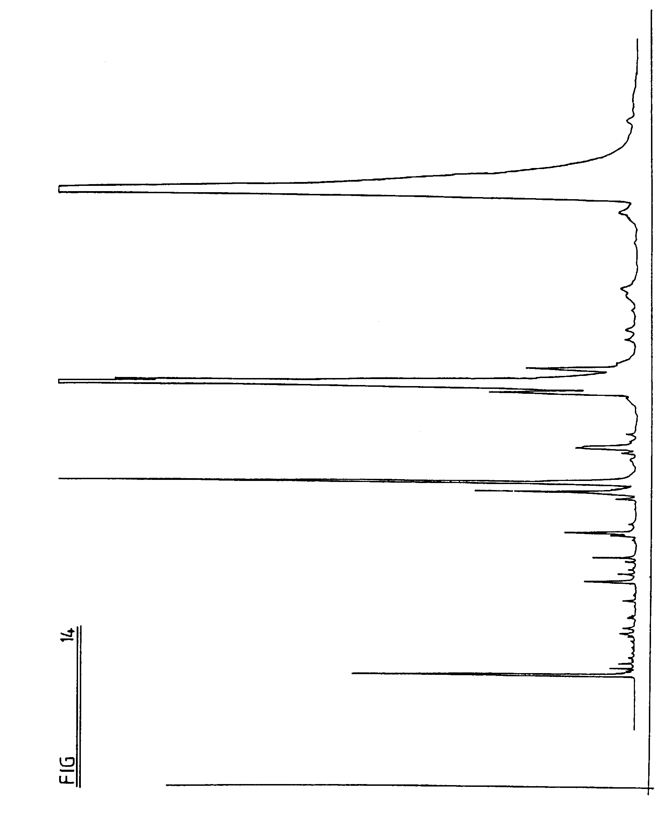

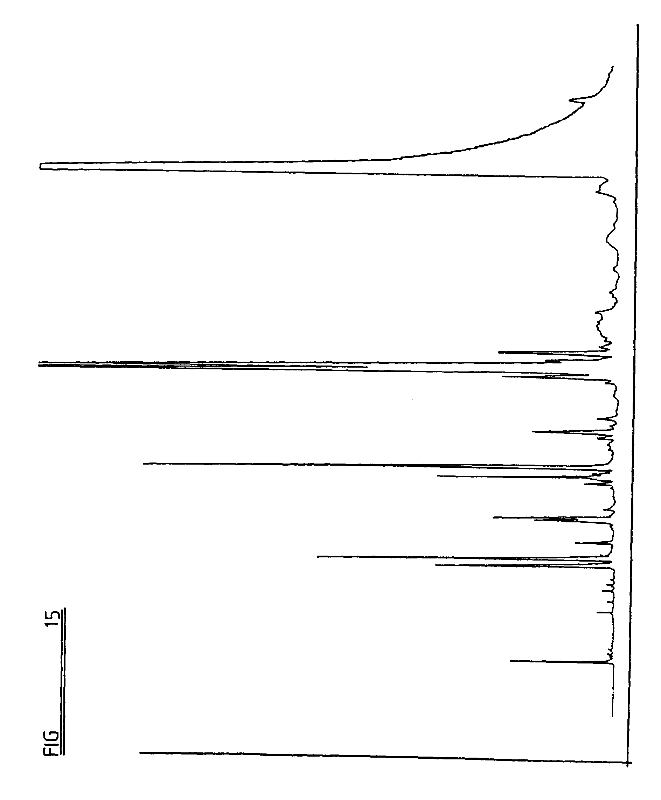

Figure 6 is the GLC profile for the reaction product of Example I(f) produced by action of Candida oleophila ATCC 20177 on 10-hydroxy stearic acid.

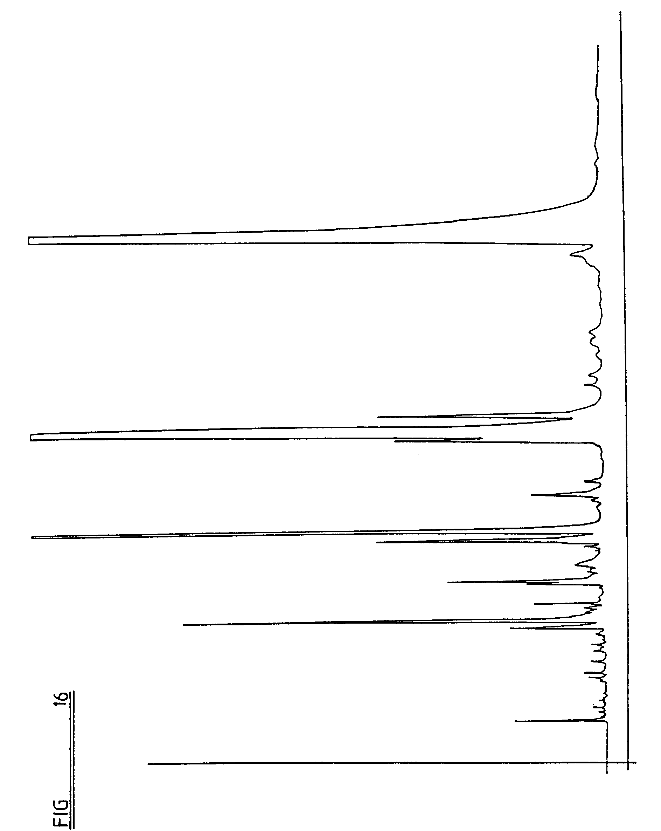

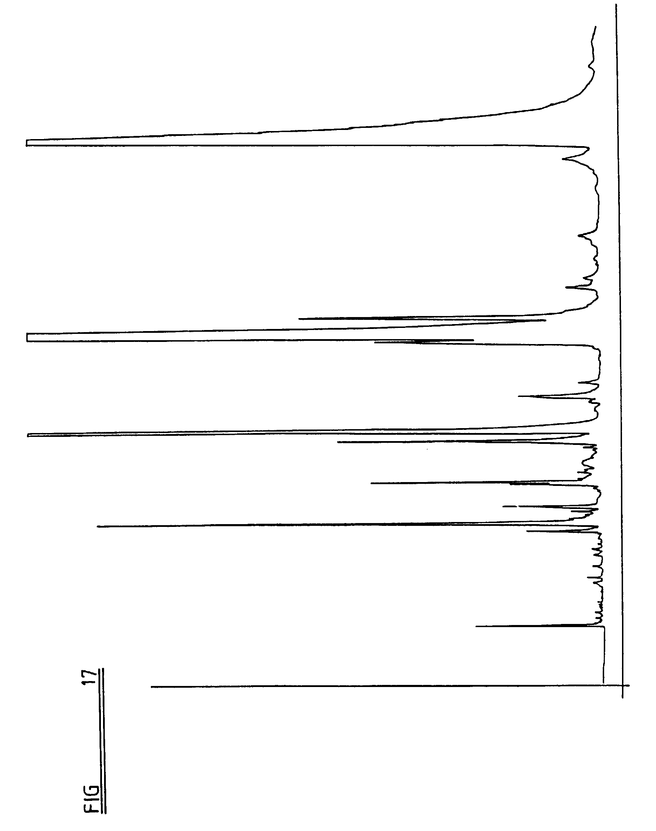

Figure 7 is the GLC profile for the reaction product of Example I(g) produced by action of Candida rugosa ATCC 14830 on 10-hydroxy stearic acid.

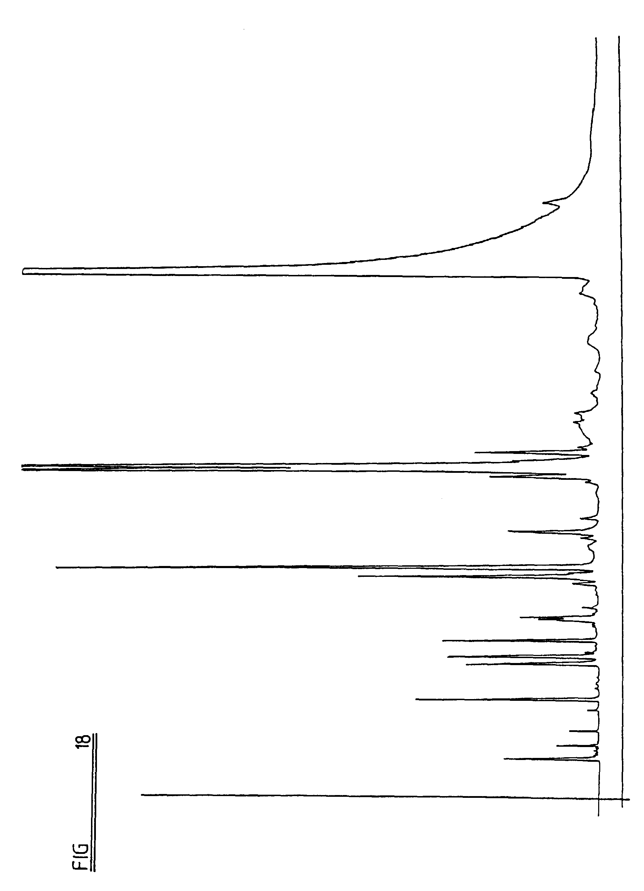

Figure 8 is the GLC profile for the reaction product of Example I(h) produced by action of Candida sp ATCC 20473 on 10-hydroxy stearic acid.

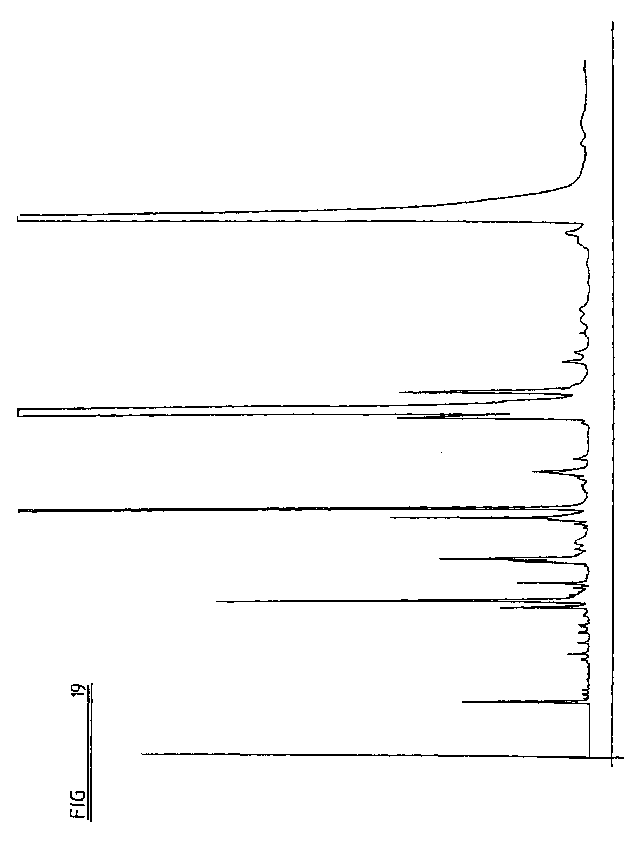

Figure 9 is the GLC profile for the reaction product of Example I(i) produced by action of Candida sp ATCC 20504 on 10-hydroxy stearic acid.

Figure 10 is the GLC profile for the reaction product of Example I(j) produced by action of Candida utilis ATCC 9226 on 10-hydroxy stearic acid.

Figure 11 is the GLC profile for the reaction product of Example I(k) produced by action of Candida utilis ATCC 15239 on 10-hydroxy stearic acid.

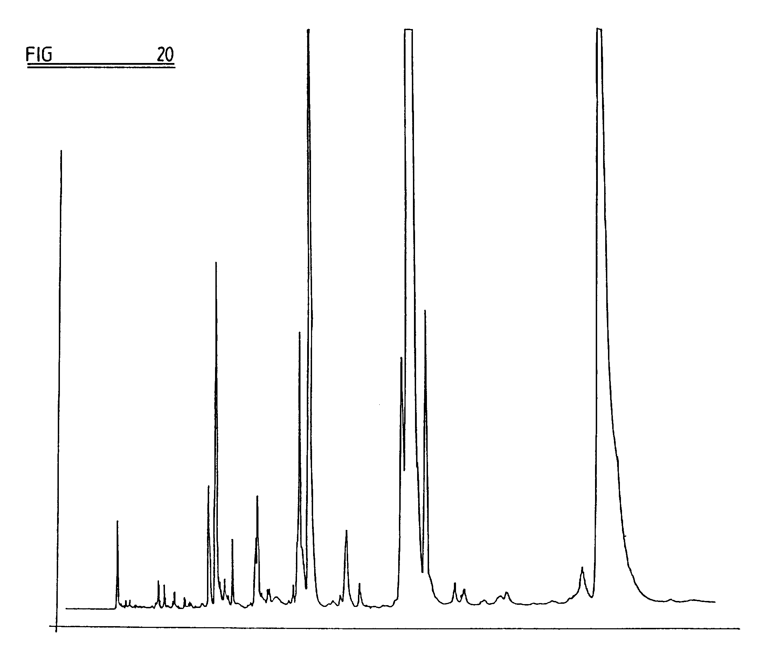

Figure 12 is the GLC profile for the reaction product of Example I(1) produced by action of Candida utilis ATCC 20248 on 10-hydroxy stearic acid.

Figure 13 is the GLC profile for the reaction product of Example I(m) produced by action of Candida utilis ATCC 42181 on 10-hydroxy stearic acid.

Figure 14 is the GLC profile for the reaction product of Example I(n) produced by action of Candida zeylanoides ATCC 15585 on 10-hydroxy stearic acid.

Figure 15 is the GLC profile for the reaction product of Example I(o) produced by action of Yarrowia lipolytica ATCC 8661 on 10-hydroxy stearic acid.

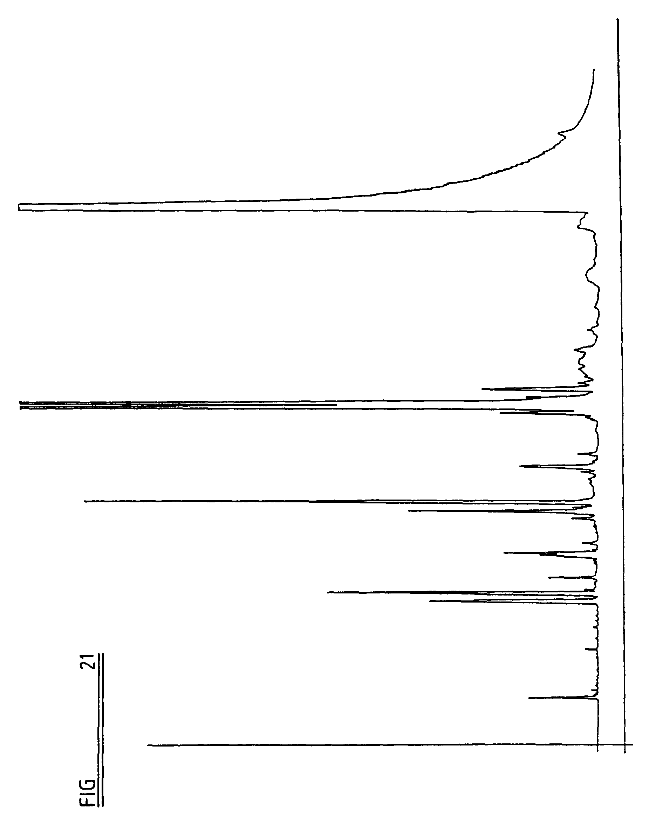

Figure 16 is the GLC profile for the reaction product of Example I(p) produced by action of Yarrowia lipolytica ATCC 8662 on 10-hydroxy stearic acid.

Figure 17 is the GLC profile for the reaction product of Example I(q) produced by action of Yarrowia lipolytica ATCC 16617 on 10-hydroxy stearic acid.

Figure 18 is the GLC profile for the reaction product of Example I(r) produced by action of Yarrowia lipolytica ATCC 16618 on 10-hydroxy stearic acid.

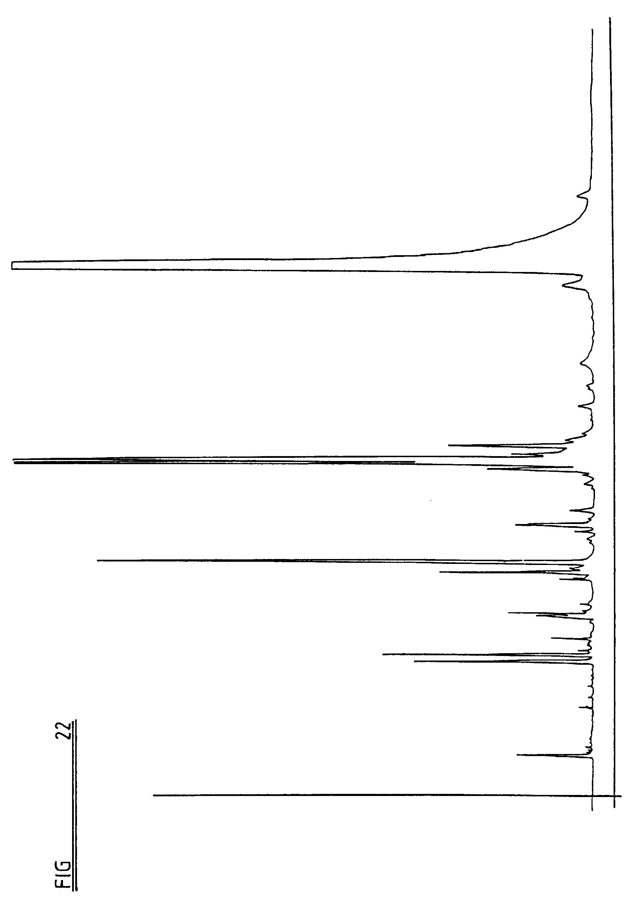

Figure 19 is the GLC profile for the reaction product of Example I(s) produced by action of Yarrowia lipolytica ATCC 20255 on 10-hydroxy stearic acid.

Figure 20 is the GLC profile for the reaction product of Example I(t) produced by action of Yarrowia lipolytica ATCC 20324 on 10-hydroxy stearic acid.

Figure 21 is the GLC profile for the reaction product of Example I(u) produced by action of Yarrowia lipolytica ATCC 20341 on 10-hydroxy stearic acid.

Figure 22 is the GLC profile for the reaction product of Example I(v) produced by action of Yarrowia lipolytica ATCC 46483 on 10-hydroxy stearic acid.

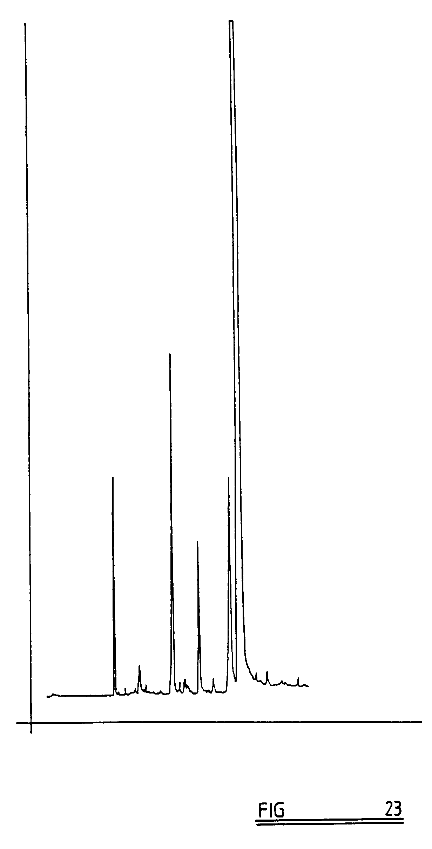

Figure 23 is the GLC profile for the reaction product of Example II, Step 2 containing the compound having the structure: 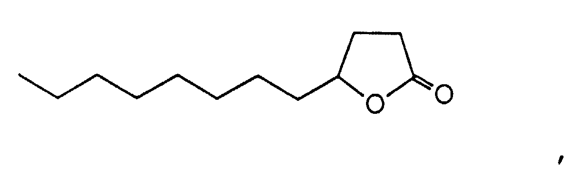 (bulked distillation fractions 3 and 4) produced by the action of Yarrowia lipolytica ATCC 34088 on 10-hydroxy stearic acid.

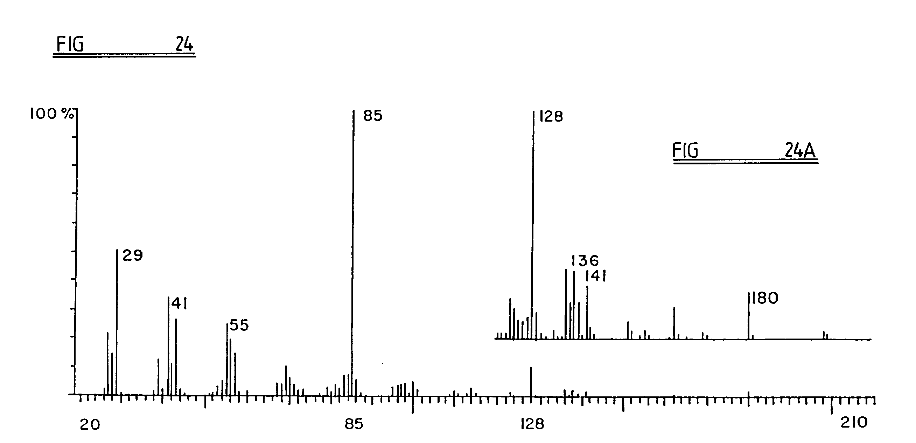

Figure 24 is the GC mass spectrum for gamma-dodecalactone having the structure: 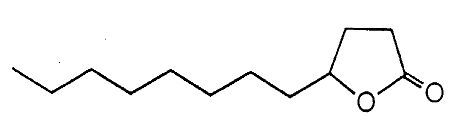 produced according to Example II, Step 2.

Figure 24A is an enlargement of the range from peaks 127-181 of the GC mass spectrum of Figure 24 for the compound having the structure:

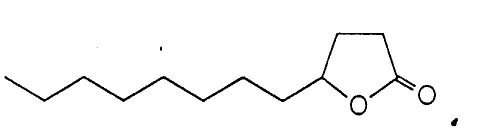

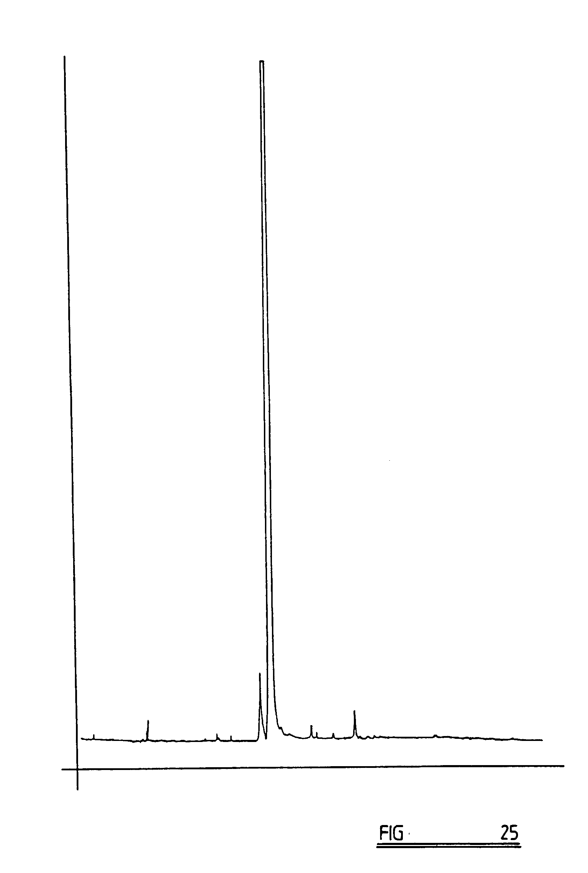

Figure 25 is the GLC profile for the reaction product of Example III, Step 2 containing the compound having the structure:

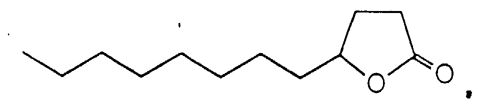

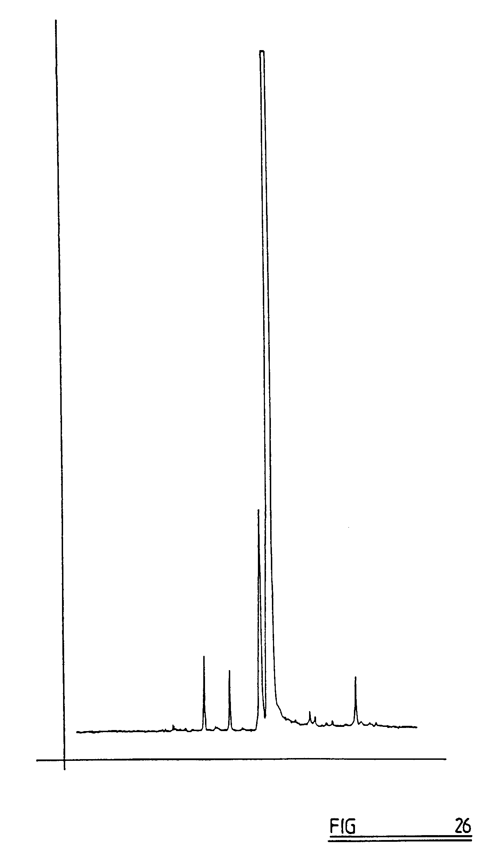

Figure 26 is the GLC profile for the reaction product of Example IV, Step 2 containing the compound having the structure:

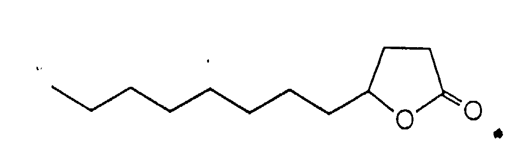

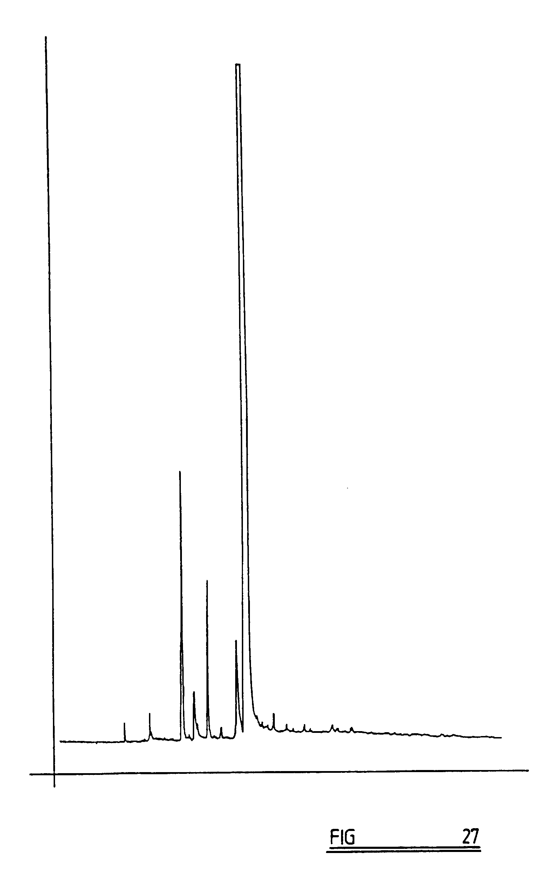

Figure 27 is the GLC profile for the reaction product of Example V, Step 2 containing the compound having the structure: 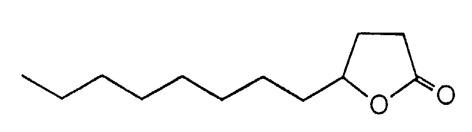 produced by action of Candida petrophilum ATCC 20226 on 10-hydroxy stearic acid.

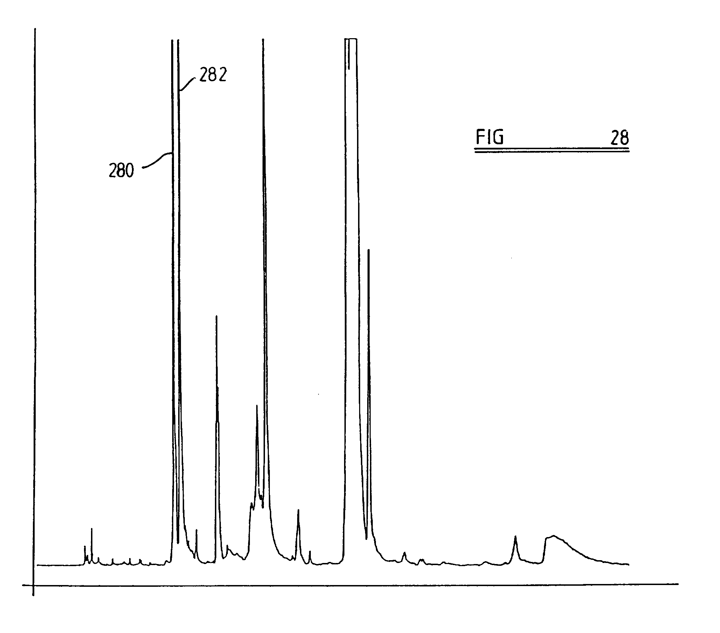

Figure 28 is the GLC profile for the crude reaction product of Example VI containing the compounds having the structures: 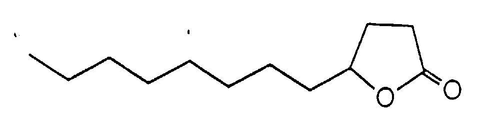 and 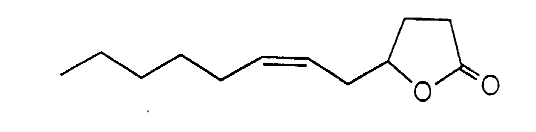 produced by action of Yarrowia lipolytica ATCC 34088 on a mixture of 10-hydroxy stearic acid and 10-hydroxy-octadec-12-enoic acid.

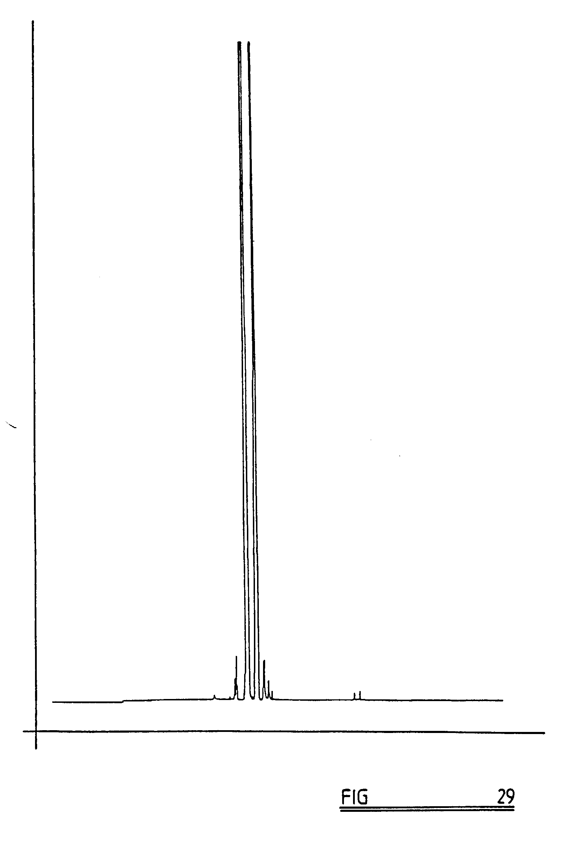

Figure 29 is the GLC fused silica capillary survey of the reaction product of Example VI containing the mixture of compounds having the structures: 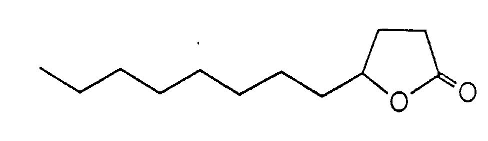 and

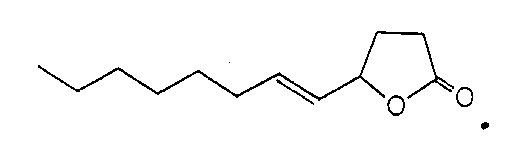

Figure 30 is the GLC profile for the reaction product of Example VIII, the reaction product of the crude extract of Step 2 of Example II with BF₃/methanol (whereby methyl esters are formed); containing inter alia the compound having the structure:

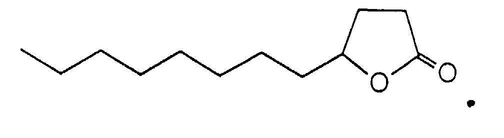

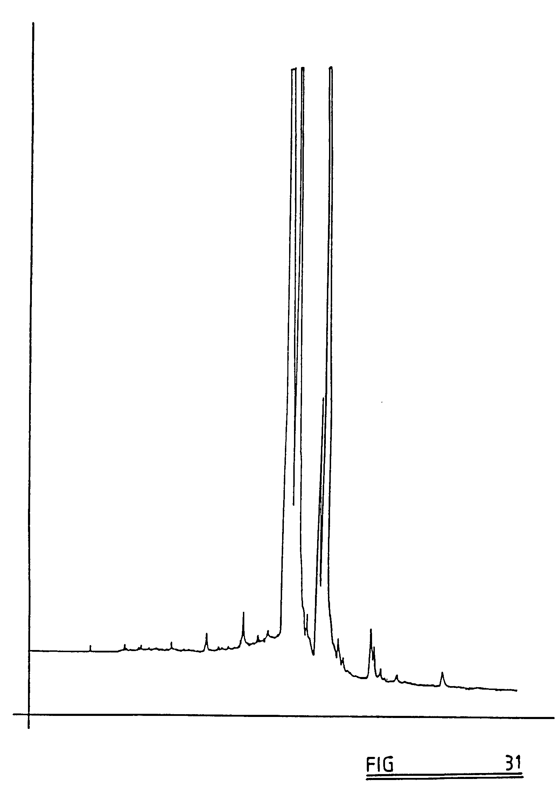

Figure 31 is the GLC profile for the reaction product of Example VII, Step 2 containing the compounds having the structures: 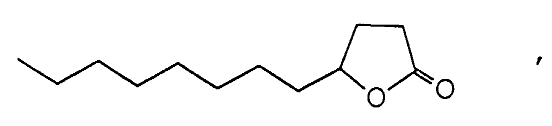 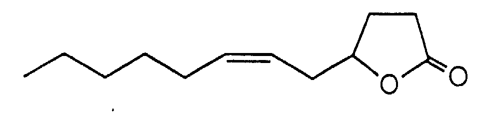 and 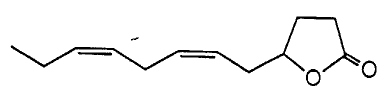 produced by action of Yarrowia lipolytica ATCC 34088 on the mixture of compounds having the structures: 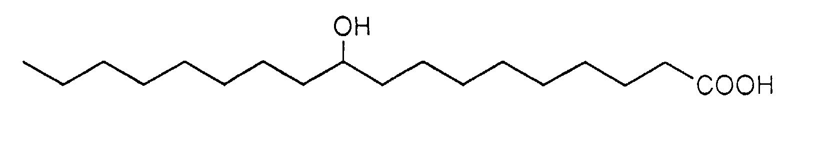 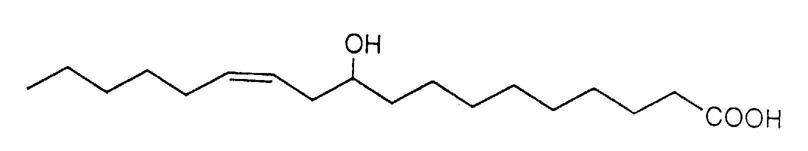 and 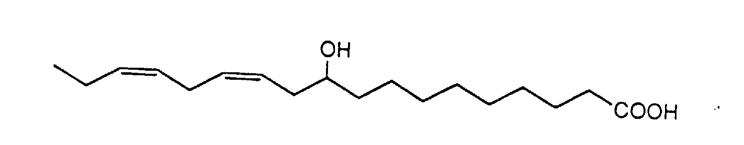 (GLC column programmed at 200°-225°C at 10°C per minute).

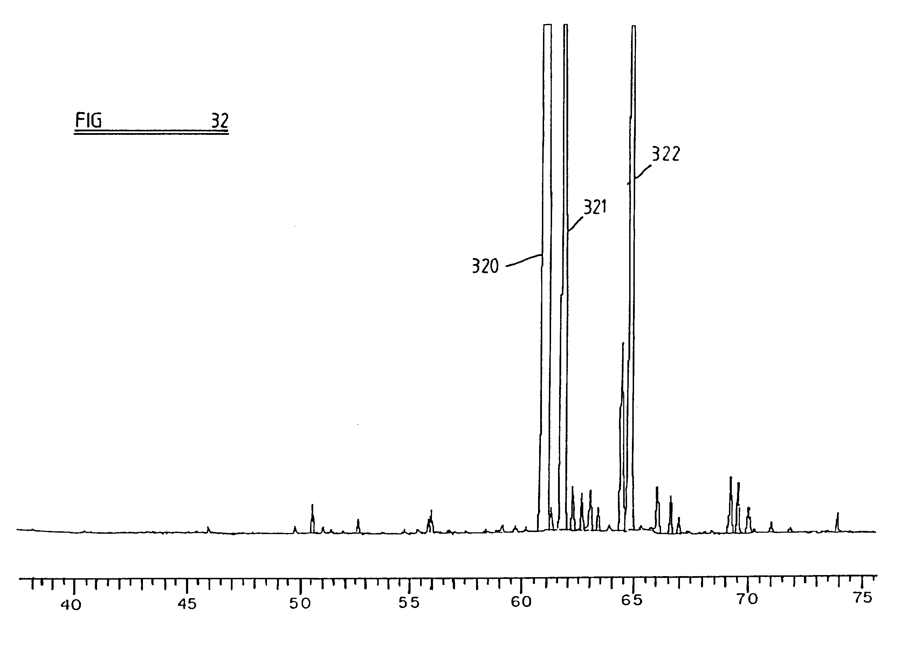

Figure 32 is the GC spectrum for the reaction product of Example VII containing the compounds having the structures: 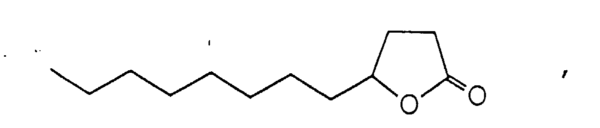 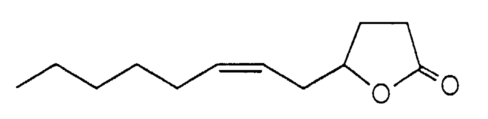 and

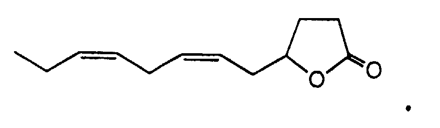

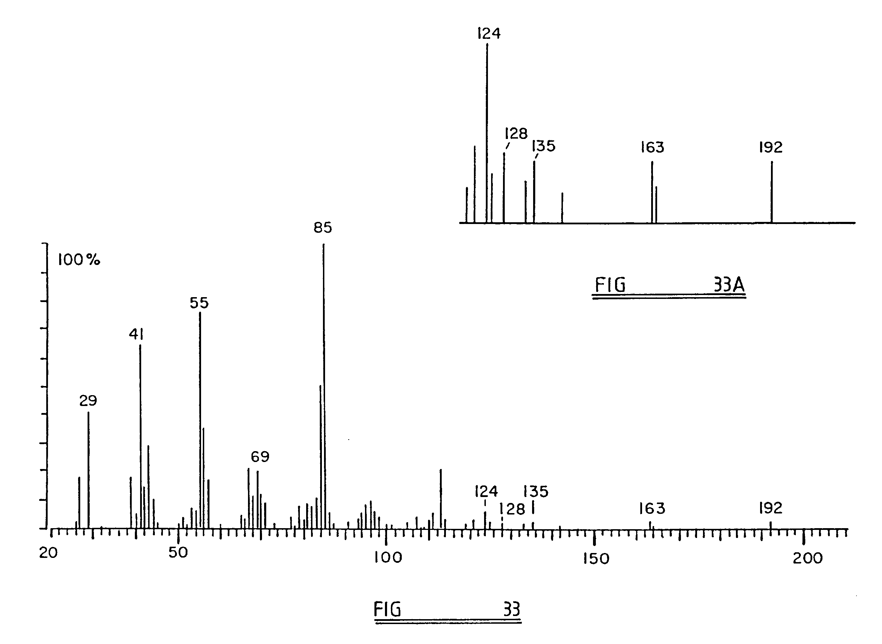

Figure 33 is the GC mass spectrum for the compound having the structure: 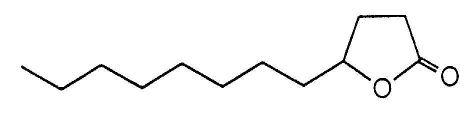 (gamma-dodecalactone) prepared according to Example VII.

Figure 33A is an enlargement of the section of Figure 33 from the peaks 123 to 192.

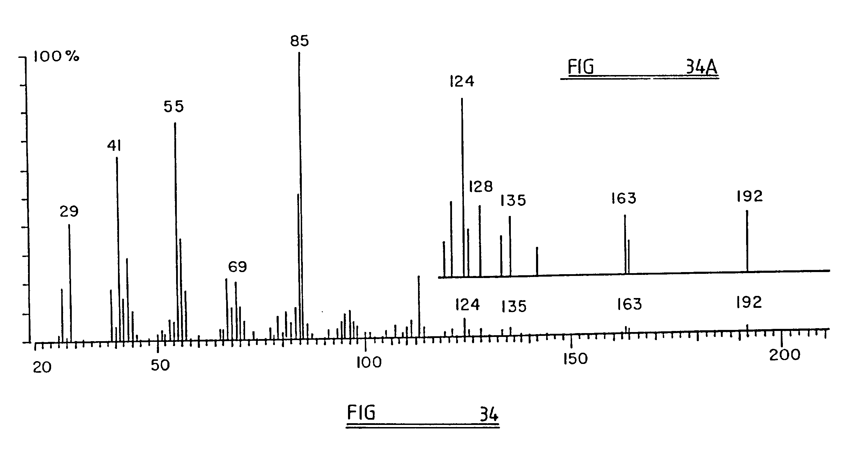

Figure 34 is the GC mass spectrum for the compound having the structure: 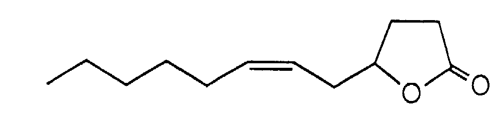 (Z)-6-dodecen-4-olide prepared according to Example VII.

Figure 34A is an enlargement of that part of the mass spectrum of Figure 34 containing peaks 123-192.

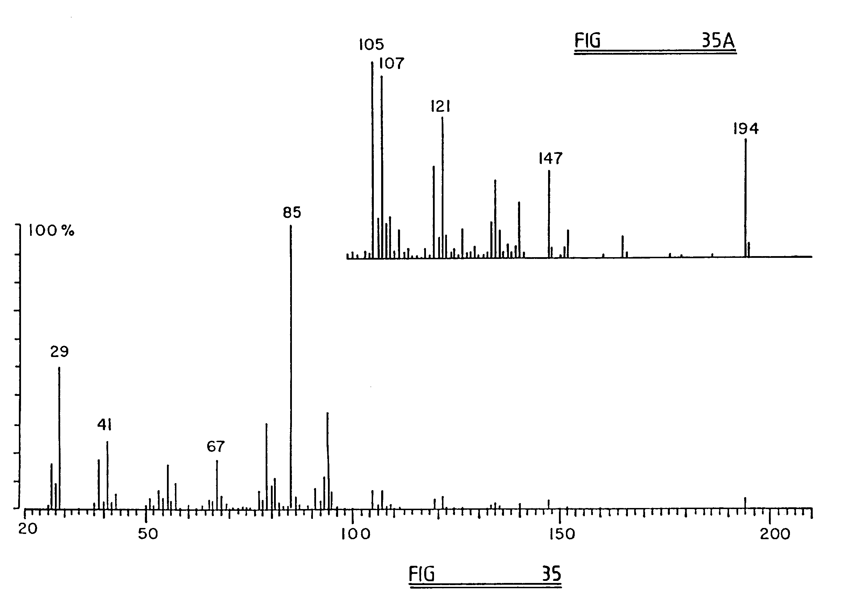

Figure 35 is the GC mass spectrum for the compound having the structure: 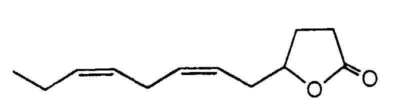 (Z,Z)-6,9-dodecadien-4-olide) prepared according to Example VII.

Figure 35A is an enlargement of that part of the GC mass spectrum of Figure 35 from peaks 105-194.

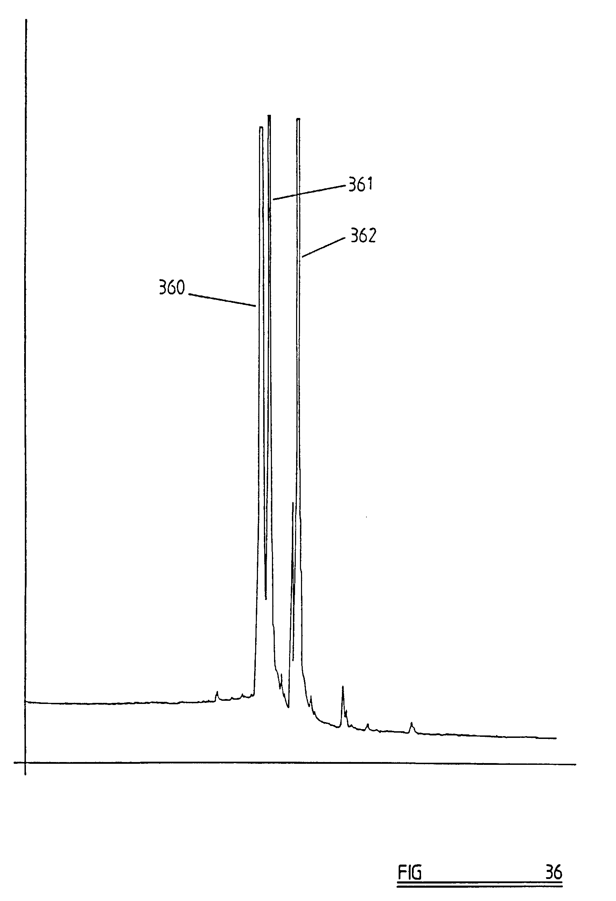

Figure 36 is the GLC profile for the distillation product of the reaction product of Example VII containing the compounds having the structures: 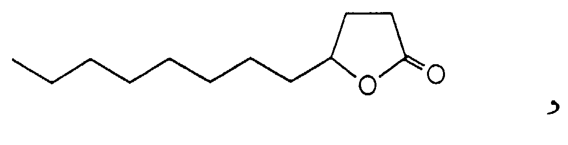 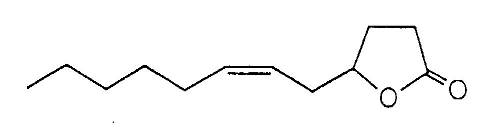 and

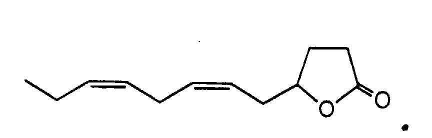

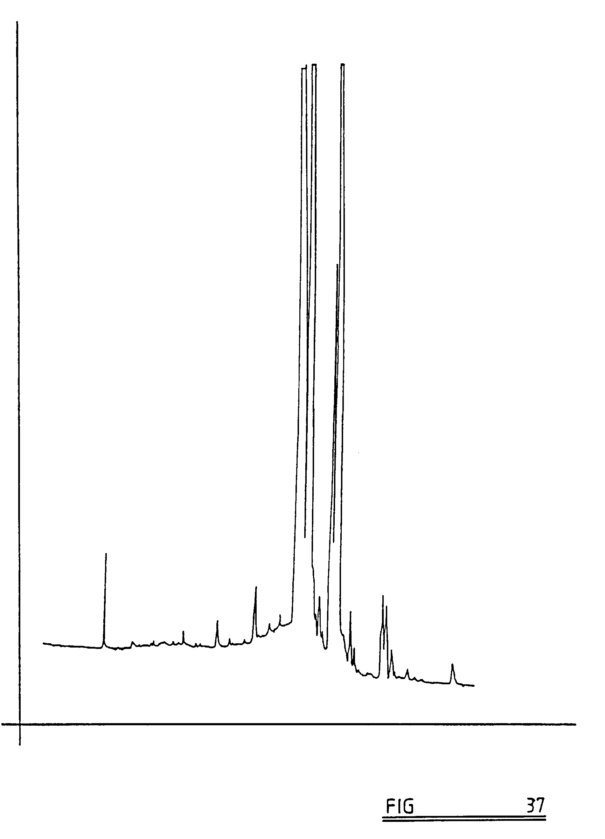

Figure 37 is the GLC profile for distillation Fraction 3 of the reaction product of Example VII.

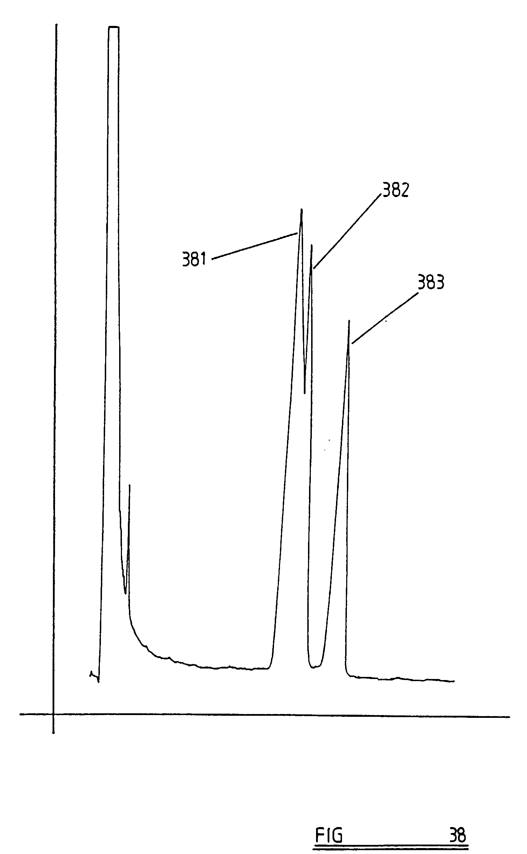

Figure 38 is the GLC profile for the reaction product of Example VII, Step 2 containing the compounds having the structures: 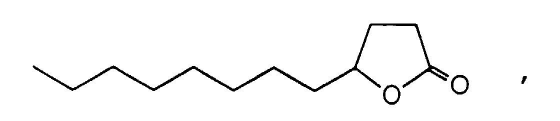 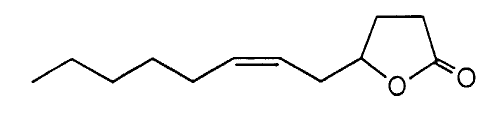 and 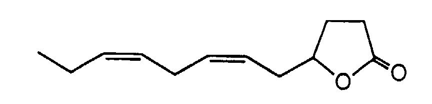 (Conditions: 10' x 0.125'' 10% carbowax column operated at 225°C, isothermal).

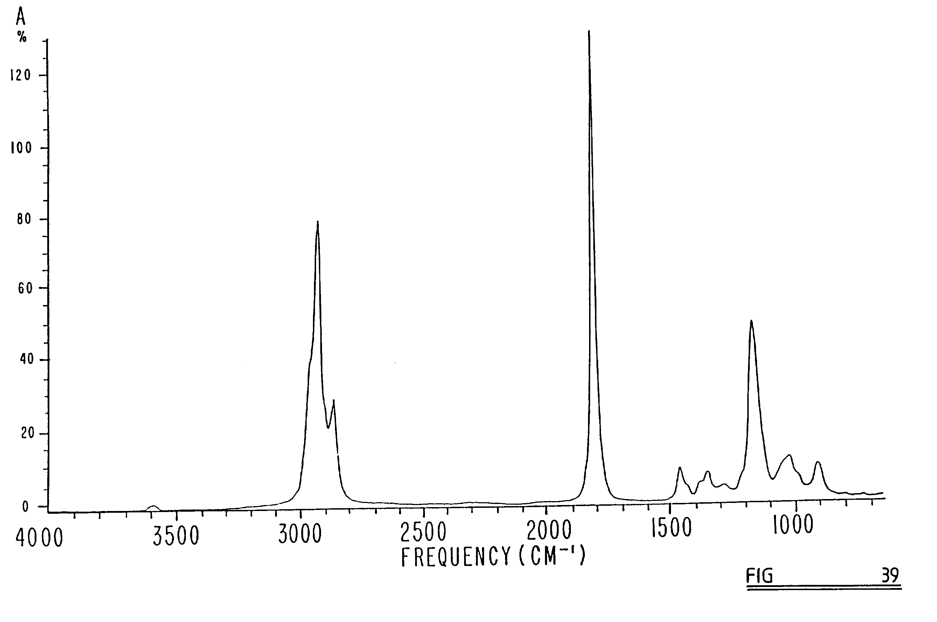

Figure 39 is the infra-red spectrum for the compound of the peak indicated by reference numeral 381 of Figure 38; for the compound having the structure:

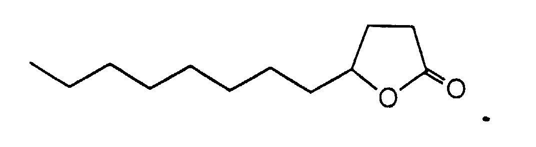

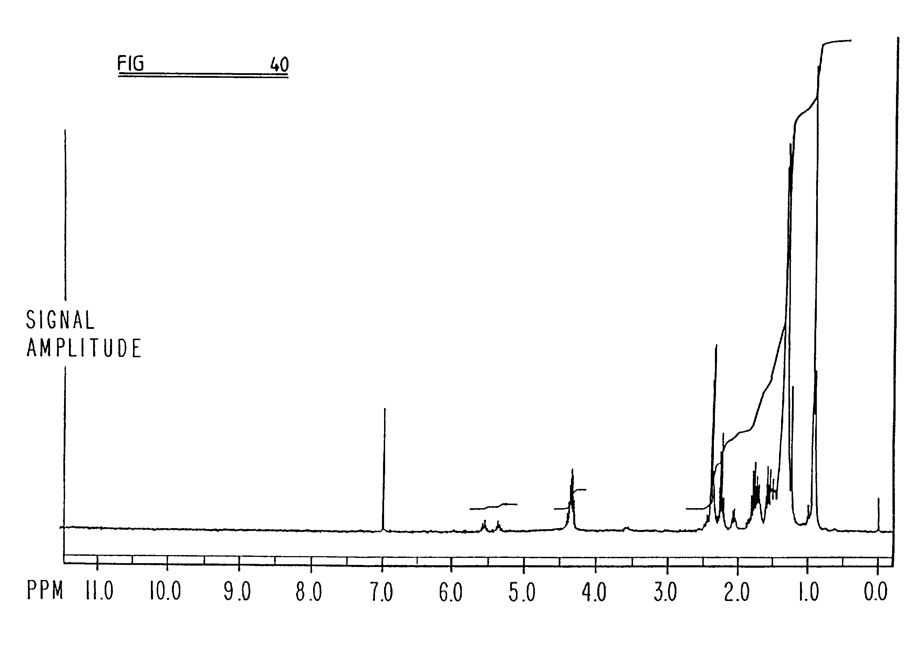

Figure 40 is the NMR spectrum for the peak indicated by reference numeral 381 of Figure 38, for the compound having the structure:

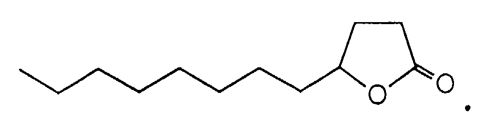

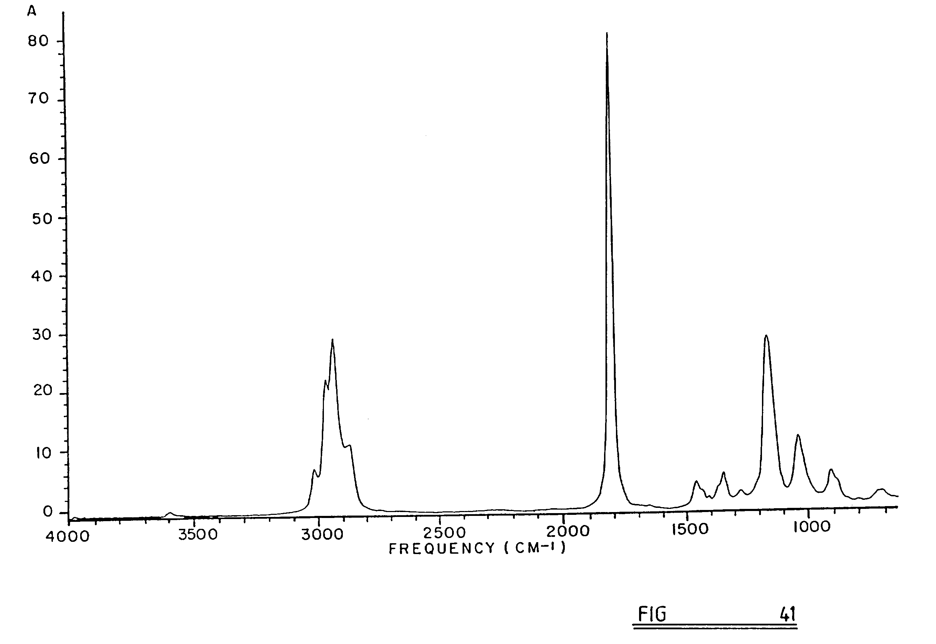

Figure 41 is the infra-red spectrum for the peak indicated by reference numeral 382 of Figure 38, for the compound having the structure:

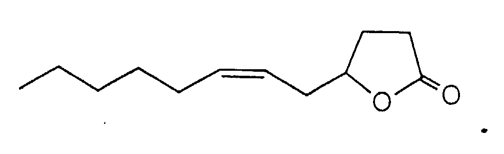

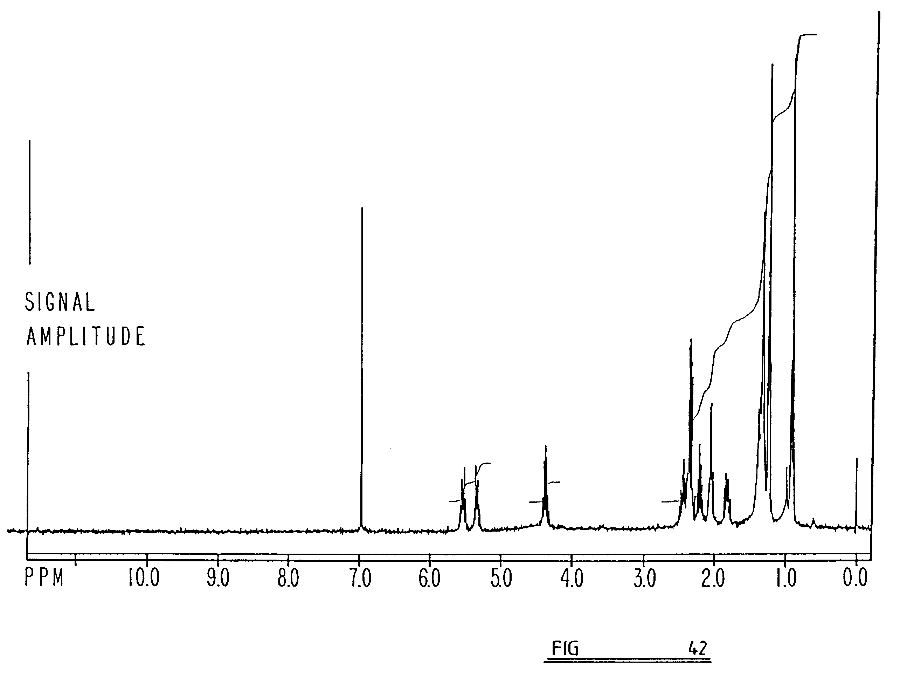

Figure 42 is the NMR spectrum for the peak indicated by reference numeral 382 of Figure 38, for the compound having the structure:

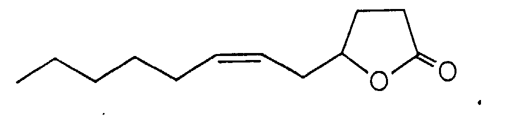

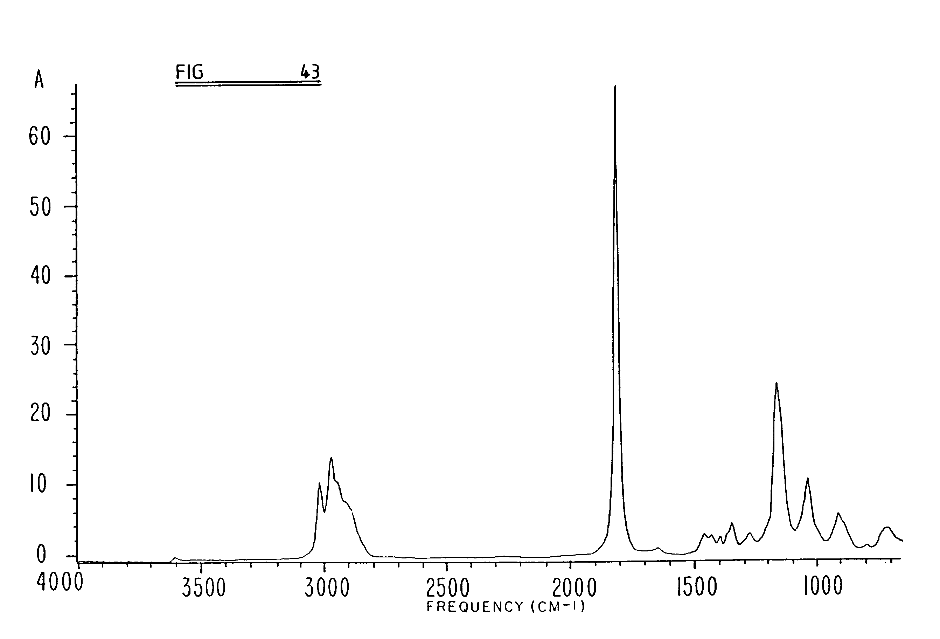

Figure 43 is the infra-red spectrum for the peak indicated by reference numeral 383 of Figure 38, for the compound having the structure:

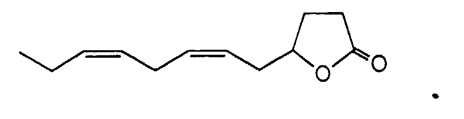

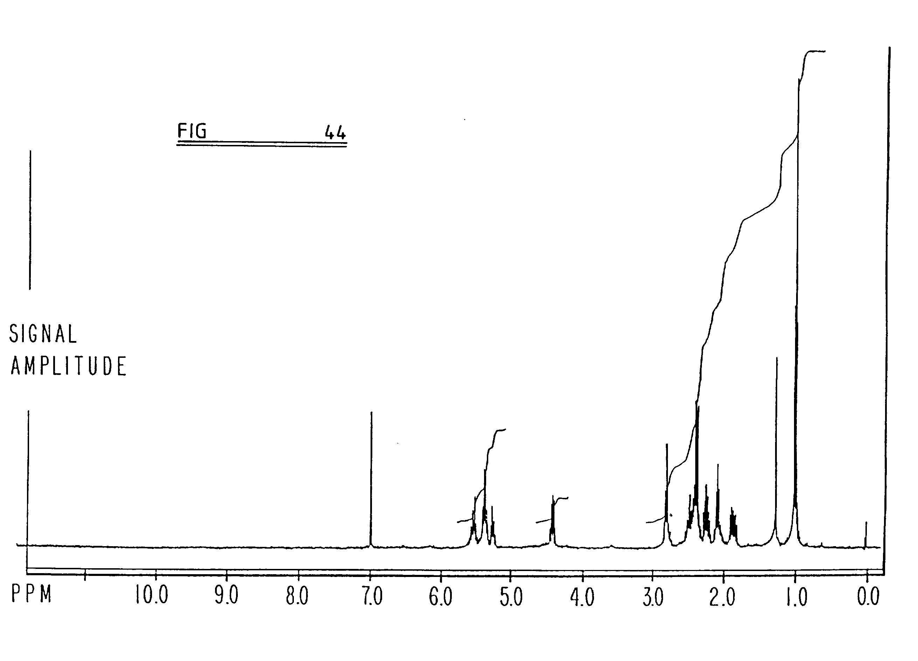

Figure 44 is the NMR spectrum for the peak indicated by reference numeral 383 of Figure 38, for the compound having the structure:

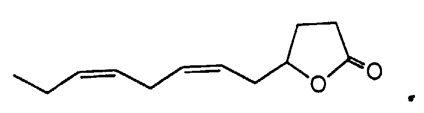

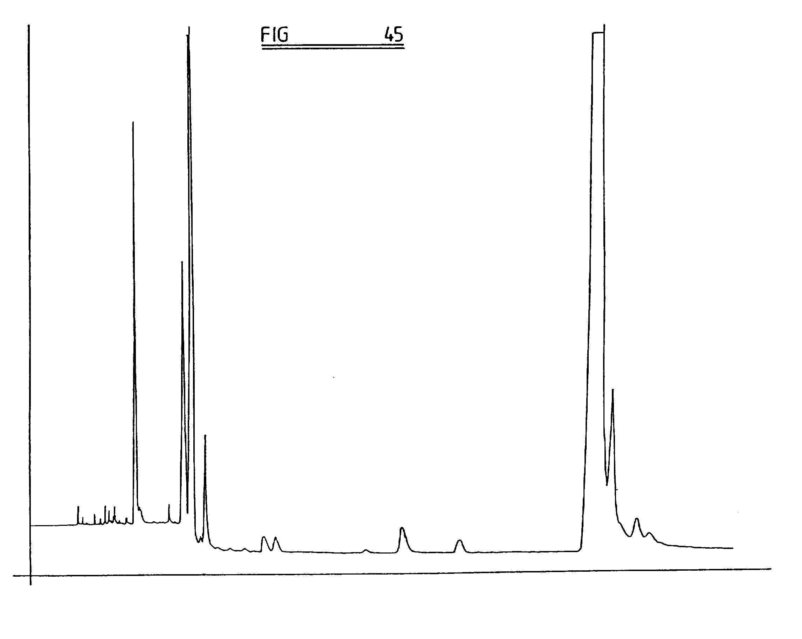

Figure 45 is the GLC profile for the reaction product of Example IX containing the compound having the structure:

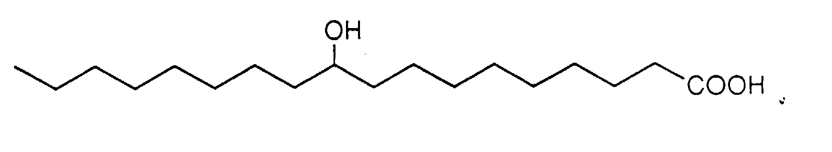

### DETAILED DESCRIPTION OF THE DRAWINGS

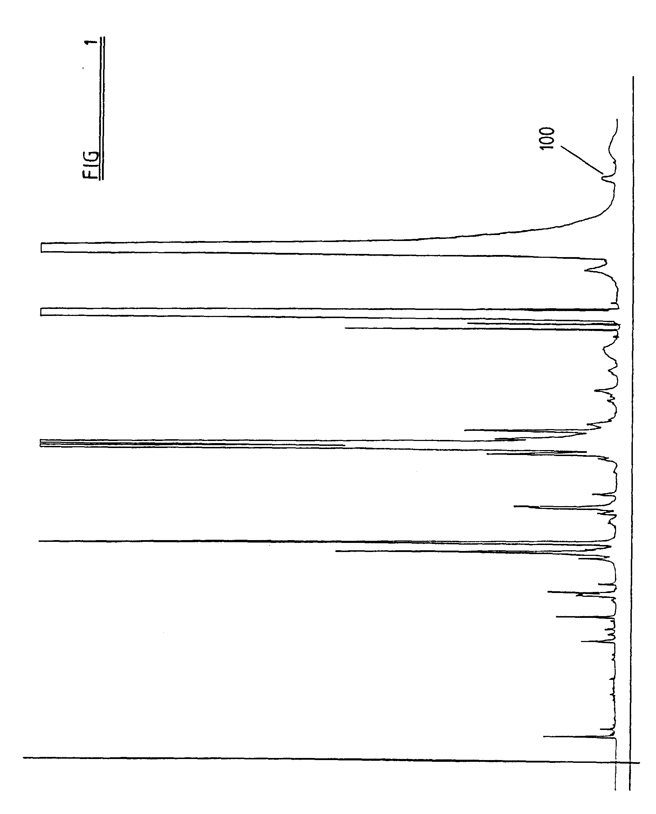

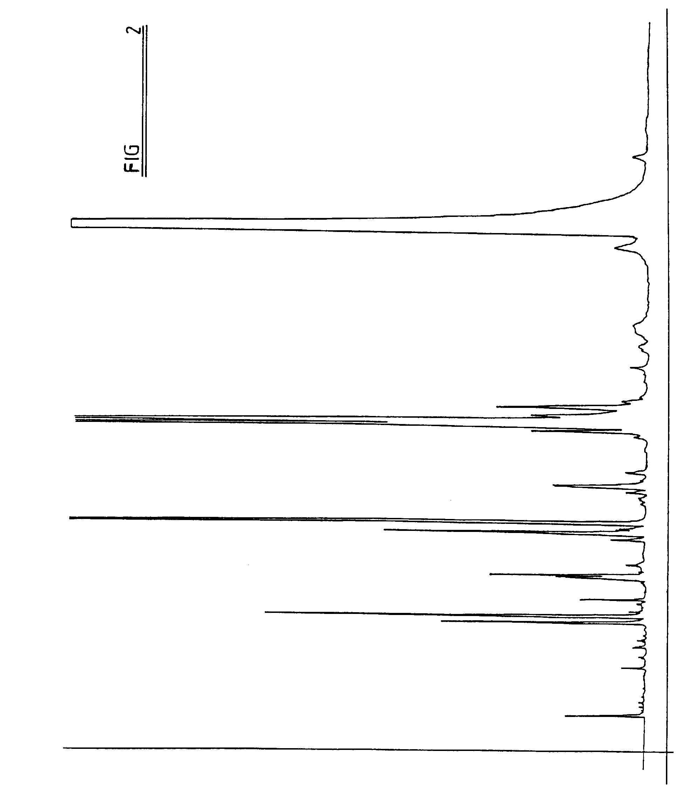

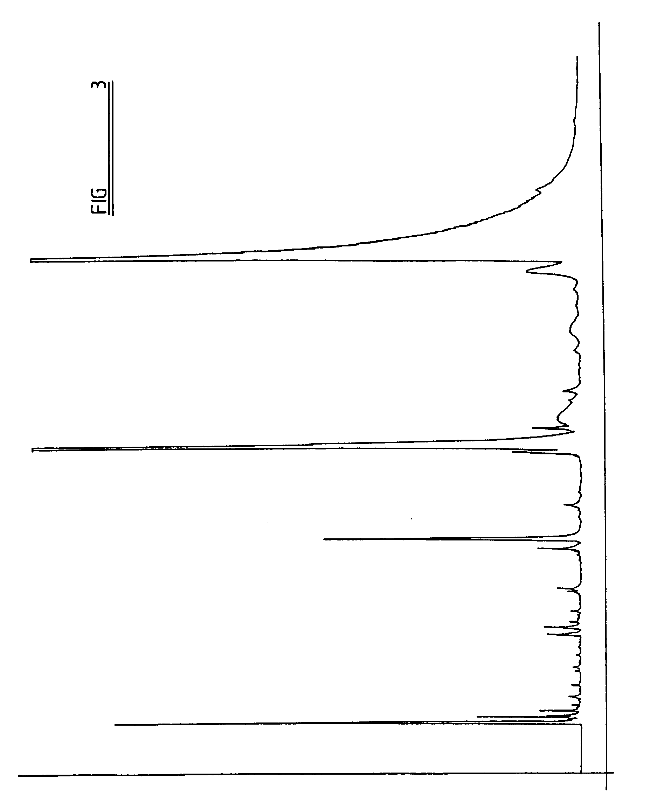

Figure 1 is the GLC profile for the reaction product of Example I(a). The peak indicated by reference numeral 100 is the peak for the compound having the structure:

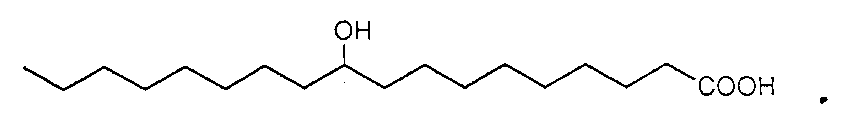

Figure 28 is the GLC profile for the crude reaction product of Example VI. The peak indicated by reference numeral 280 is the peak for the compound having the structure:

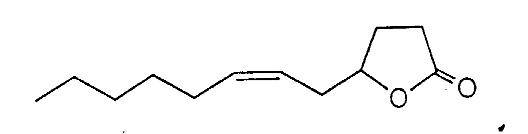

The peak indicated by reference numeral 282 is the peak for the compound having the structure:

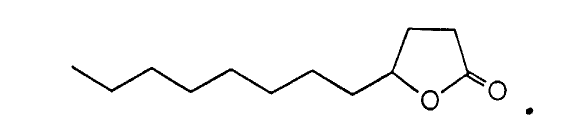

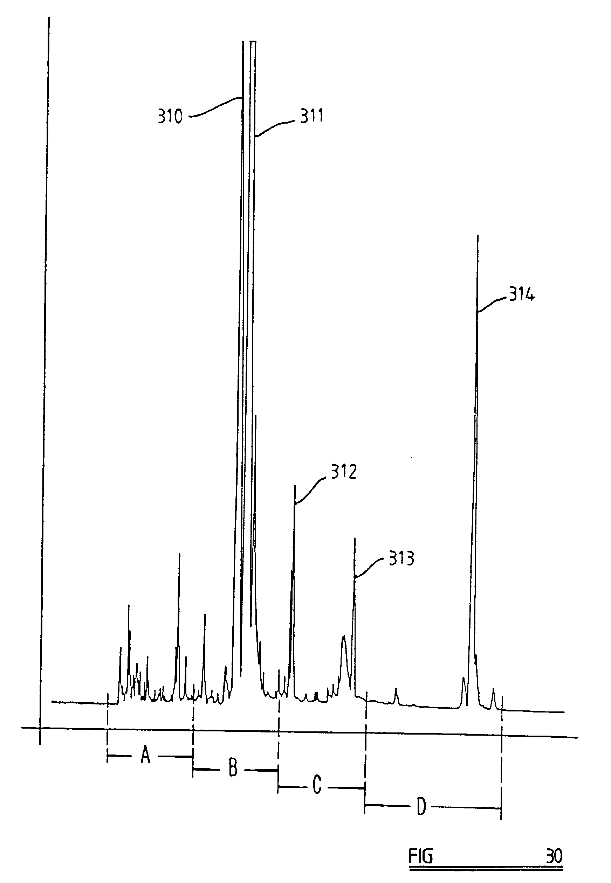

Figure 30 is the GLC profile for the reaction product of Example VIII. The peaks of the GLC profile are grouped into four groups, Group "A", Group "B", Group "C" and Group "D".

The peaks of Group "A" include the compounds:
Methanol;
Chloroform;
Methyl isobutyrate;
2-Methyl-2-propenoate;
Methyl butyrate;
Methyl crotonate;
Methyl isovalerate;
Methyl-2-methyl butyrate;
Hexanol;
Methyl valerate;
Methyl senecioate;
Methyl tiglate;
Methyl-4-methyl valerate;
Methyl hexanoate;
Methyl-3-hexenoate;
Methyl-4-methyl-4-pentenoate;
3-Methyl-2-oxo-valeric acid, methyl ester;
Methyl-2-furoate;
Methyl-2,4-dimethyl pentanoate;
4-Methyl-2-oxo-valeric acid, methyl ester;
Methyl heptanoate;
Dimethyl fumarate;
Dimethyl succinate;
Methyl-4-methylene hexanoate;
Phenyl Acetaldehyde;
Methyl-cyclohexane carboxylate;
Dimethyl ester of methyl maleic acid;
Methyl-3-octenoate;
Methyl benzoate;
Nonanal;
Phenyl ethyl alcohol;
Methyl-3-octenoate;
Methyl octanoate;
Methyl-2,2-dimethoxy-3-methyl pentanoate;
Methyl phenyl acetate;
n-Decanal;
1,1-Dimethoxy-2-phenyl ethane;
Methyl nonanoate;
Benzyl acetone;
Gamma octalactone; and
Methyl decanoate.

The group of peaks including peaks indicated by reference numerals 310 and 311 included in Group "B" includes the compounds:
Gamma nonalactone;
Methyl-4-oxo-nonanoate;
Methyl undecanoate;
Gamma decalactone;
Methyl-4-oxo-decanoate;
Methyl-3-dodecenoate;
Ionol;
Gamma undecalactone;
Methyl-3,6-dodecadienoate;
The compound having the structure: 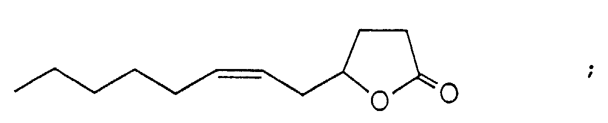 Methyl-4-oxo-dodecanoate;
Gamma dodecalactone having the structure: 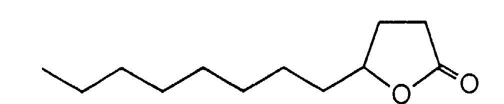 (indicated by the peak having the reference numeral 311); and
Methyl myristate.

The group of peaks indicated by Group "C" and containing the peaks indicated by reference numerals 312 and 313 are as follows:
Methyl palmitate;
Methyl-9-hexadecenoate; and
Methyl heptadecanoate.

The group of peaks indicated by Group "D" and containing the peak indicated by reference numeral 314 covers the following compounds:
Methyl-10,13-octadecadienoate;
Methyl-9-octadecenoate; and
Methyl stearate.

Figure 32 is the GC spectrum for the reaction product of Example VII. The peak indicated by reference numeral 320 is the peak for the compound having the structure: 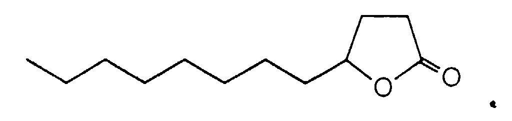 The peak indicated by reference numeral 321 is the peak for the compound having the structure: 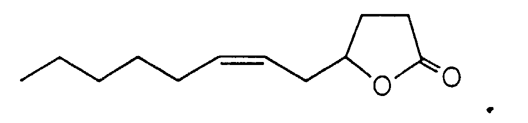 The peak indicated by reference numeral 322 is the peak for the compound having the structure:

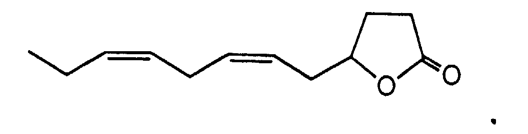

Figure 36 is the GLC profile for the reaction product of Example VII (distillation product). The peak indicated by reference numeral 360 is the peak for the compound having the structure: 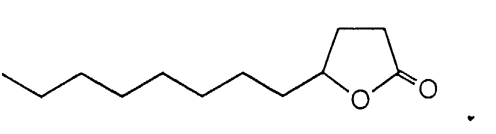 The peak indicated by reference numeral 361 is the peak for the compound having the structure: 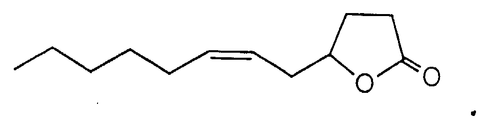 The peak indicated by reference numeral 362 is the peak for the compound having the structure:

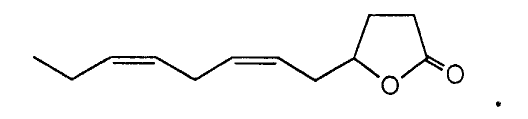

Figure 38 is the GLC profile for the reaction product of Example VII (conditions: 10' x 0.125" 10% carbowax column programmed at 225°C). The peak indicated by reference numeral 381 is the peak for the compound having the structure: 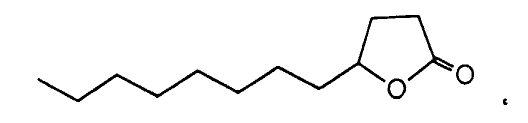 The peak indicated by reference numeral 382 is the peak for the compound having the structure: 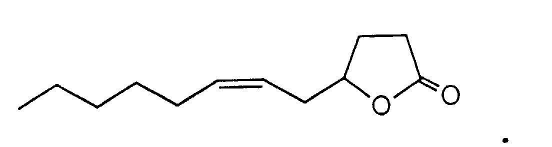 The peak indicated by reference numeral 383 is the peak for the compound having the structure:

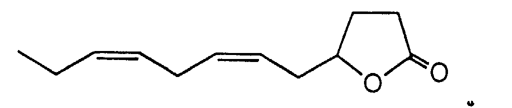

### EXAMPLE I

### GAMMA DODECALACTONE SCREENING PROCEDURE

The following inoculum medium was prepared under the following conditions:

| Inoculum Medium: | | Parameters: |
|---|---|---|
| 2% | Difco Beef Extract | Temperature: 25°C |
| 0.01% | KH₂PO₄ | Agitation: 150 RPM |
| 0.005% | MgSO₄.7H₂O | Duration: 24 Hours |
| 0.02% | TWEEN® 80 | |
| 1% | Olive Oil | |
| pH=7 | | |

The following screening medium was prepared using the following conditions:

| Screening Medium: | | Parameters: |
|---|---|---|
| 2% | Difco Beef Extract | Temperature: 25°C |
| 0.01% | KH₂PO₄ | Agitation: 150 RPM |
| 0.005% | MgSO₄.7H₂O | Inoculum: 5% |
| 0.02% | TWEEN® 80 | Duration: 48 hours |
| 1.5% | Olive Oil | |
| 0.6% | 10-Hydroxy stearic acid | |
| pH=7 | | |

### Process:

Five hundred ml flasks containing 100 ml of screening medium as prepared, supra, were sterilized at 121°C for 20 minutes. Each flask was inoculated using 5% of 24 hour grown culture of each organism as set forth in Table I, infra. Each flask was incubated in a shaker (150 RPM) at 25°C for 48 hours.

### Termination:

At the end of the fermentation, fermentation is terminated by adjusting the pH to 2 using 50% H₂SO₄. Flasks are then heated to 100°C for 10 minutes and cooled to room temperature. The broth is extracted three times with an equal volume of ethyl acetate. The combined extracts are washed twice with saturated NaCl. The solvent is evaporated under vacuum. The oil is treated with BF₃-Methanol and analyzed by GC.

### Results:

The results of these screens are summarized in Table I as follows:

| Example No. | Organism | Organism I.D. | Grams Crude Gamma-Dodecalactone Per 100 ml | Percent Gamma-Dodecalactone |
|---|---|---|---|---|
| I(a) | Candida brumptii | IFO 0731 | 2.92 | 0.03 |
| I(b) | Candida deformans | ATCC 22969 | 3.16 | 2.00 |
| I(c) | Candida ethanotermophilum | ATCC 20380 | 2.46 | 0.33 |
| I(d) | Candida iberica | ATCC 26318 | 3.27 | 0.30 |
| I(e) | Candida kefyr | ATCC 42265 | 3.43 | 0.05 |
| I(f) | Candida oleophila | ATCC 20177 | 2.74 | 1.90 |
| I(g) | Candida rugosa | ATCC 14830 | 2.01 | 0.04 |
| I(h) | Candida sp. | ATCC 20473 | 2.61 | 0.28 |
| I(i) | Candida sp. | ATCC 20504 | 2.54 | 0.14 |
| I(j) | Candida utilis | ATCC 9226 | 2.19 | 0.10 |
| I(k) | Candida utilis | ATCC 15239 | 1.97 | 0.54 |
| I(l) | Candida utilis | ATCC 20248 | 1.97 | 0.25 |
| I(m) | Candida utilis | ATCC 42181 | 1.96 | 1.40 |
| I(n) | Candida zeylanoides | ATCC 15585 | 2.58 | 0.12 |
| I(o) | Yarrowia lipolytica | ATCC 8661 | 3.04 | 1.70 |
| I(p) | Yarrowia lipolytica | ATCC 8662 | 2.38 | 2.40 |
| I(q) | Yarrowia lipolytica | ATCC 16617 | 2.68 | 2.20 |
| I(r) | Yarrowia lipolytica | ATCC 16618 | 3.69 | 1.10 |
| I(s) | Yarrowia lipolytica | ATCC 20255 | 2.56 | 2.40 |
| I(t) | Yarrowia lipolytica | ATCC 20324 | 2.49 | 1.80 |
| I(u) | Yarrowia lipolytica | ATCC 20341 | 2.59 | 1.60 |
| I(v) | Yarrowia lipolytica | ATCC 46483 | 2.93 | 1.30. |

The GLC profiles for the reaction products corresponding to each example are as follows:

| Figure No. | Example No. |
|---|---|
| 1 | I(a) |
| 2 | I(b) |
| 3 | I(c) |
| 4 | I(d) |
| 5 | I(e) |
| 6 | I(f) |
| 7 | I(g) |
| 8 | I(h) |
| 9 | I(i) |
| 10 | I(j) |
| 11 | I(k) |
| 12 | I(l) |
| 13 | I(m) |
| 14 | I(n) |
| 15 | I(o) |
| 16 | I(p) |
| 17 | I(q) |
| 18 | I(r) |
| 19 | I(s) |
| 20 | I(t) |
| 21 | I(u) |
| 22 | I(v). |

### EXAMPLE II

### PRODUCTION OF 10-HYDROXY STEARIC ACID AND NATURAL GAMMA DODECALACTONE

### STEP 1: PRODUCTION OF 10-HYDROXY STEARIC ACID FROM OLEIC ACID Reaction:

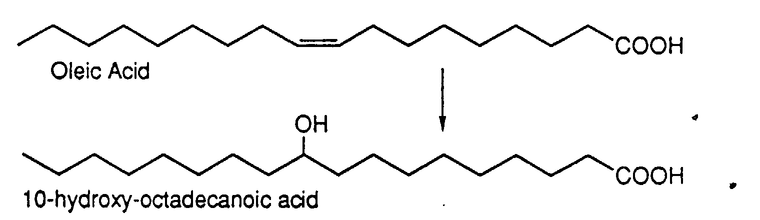

The following inoculum medium is prepared:

| Inoculum Medium: | |
|---|---|
| Difco Yeast Extract | 0.5% |
| KH₂PO₄ | 0.4% |
| MgSO₄.7H₂O | 0.05% |
| TWEEN® 80 | 0.02% |
| pH = 7.0, adjusted before sterilization with 50% aqueous KOH. | |

Fernbach flasks with 500 ml of medium are sterilized at 121°C for 30 minutes. After sterilization 0.5 ml of sterile 50% dextrose per 100 ml of medium is added. Flasks are inoculated from a slant of Pseudomonas sp NRRL B-2994 and incubated at 25°C, 100 RPM, for 24 hours to form an "inoculum culture".

### Production Medium:

The following production medium is prepared:

| | |
|---|---|
| TASTONE® 900 (Primary Grown Yeast Extract manufactured by the Universal Foods Inc. of 433 East Michigan Street, Milwaukee, Wisconsin 53202 (Trademark of Universal Foods Inc.)) | 0.5% |
| KH₂PO₄ | 0.4% |
| MgSO₄.7H₂O | 0.05% |
| TWEEN® 80 | 0.02% |
| Oleic Acid | 0.1% |
| pH = 6.5, maintained during fermentation with 25% NAOH | |

8 Liters of medium are sterilized in 14 liter fermenters at 121°C for 30 minutes. After sterilization, 5 ml of sterile 50% dextrose per liter of medium are added.

### Parameters:

Inoculum: 500 ml of inoculum culture (are added to each 8 liter production medium batch)
Temperature: 26°C
Aeration: 0.1 v/v/m
Agitation: 420 RPM

### Process:

After 24 hours of growth, aeration is stopped, agitation is increased to 900 RPM, and oleic acid pumping begins as shown in Table II, infra.

**TABLE 2.**

| 10-HYDROXY STEARIC ACID PRODUCTION FROM OLEIC ACID | | | | | |
|---|---|---|---|---|---|
| Incubation Time (Hrs) | Total Oleic Acid Pumped (grams) | Crude 10-Hydroxy Acid (g/l) | Oleic Acid % | 10-Hydroxy Acid % | Yield (10-Hydroxy Acid) Grams Per Liter |
| 24 | 108 | 8.5 | 38.7 | 27.0 | 2.3 |
| 48 | 199 | 18.6 | 40.9 | 31.0 | 5.8 |
| 120 | 300 | 32.5 | 26.0 | 45.0 | 14.4 |

### STEP 2: Conversion of 10-hydroxy stearic acid to gamma-dodecalactone.

### Reaction :

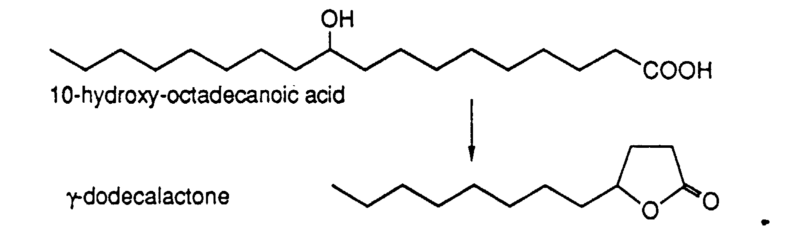

The following inoculum medium was prepared:

| Inoculum medium: | |
|---|---|
| Beef Extract | 2.0% |
| KH₂PO₄ | 0.01% |
| MgSO₄.7H₂ | 0.005% |
| TWEEN® 80 | 0.02% |
| Olive Oil | 1.0% |
| pH = 7 | |

100 ml of Medium were sterilized in 500 ml flasks for 20 minutes at 121°C. Flasks were inoculated from a slant of Yarrowia lipolytica ATCC 34088, and incubated at 25°C, 150 RPM for 24 hours.

2% TASTONE®900 was added to the fermenter from Step 1 containing the previously-produced 10-hydroxy stearic acid and sterilized at 121°C for 20 minutes. The fermenter was then cooled to 28°C and inoculated with 200 ml of inoculum. After 20 hours incubation 30 g of olive oil was added to the fermenter.

### Fermenter Parameters:

Inoculum: 200 ml of inoculum culture
Temperature: 28°C
Aeration: 0.5 v/v/m
Agitation: 620 RPM
pH: 7, maintained with 25% NaOH
Back pressure: 7.5 psi
Antifoam: Mazu DF 100, automatically added as needed.

### Results of Step 2:

The broth samples were analyzed as in step one. The results of Step 2 are summarized in Table 3 as follows:

**TABLE 3.**

| CONVERSION OF 10-HYDROXY STEARIC ACID TO GAMMA-DODECALACTONE. | | | | |
|---|---|---|---|---|
| Incubation Time (Hrs) | Crude Gamma-Dodecalactone (g/l) | 10-Hydroxy Acid % | Gamma-Dodecalactone % In Crude | Gamma-Dodecalactone yield (grams per liter) |
| 18 | 18.8 | 49.8 | 18.6 | 3.5 |
| 20 | 16.6 | 45.0 | 17.3 | 2.9 |
| 23 | 17.1 | 37.0 | 11.2 | 1.9. |

### Product Recovery:

At the end of the fermentation, the pH was adjusted to 4 using citric acid and the broth was then heated to 100°C for 30 minutes. The entire broth (6.5 liters) was extracted 3 times with 1/3 volume ethyl acetate. The extracts were combined and the solvent was removed under reduced pressure. The crude extract (110 grams) was fractionally distilled, resulting in 12.7 grams of product having a purity of 90.2%. The distillation data is as follows:

| Fraction No. | Vapor Temp. (°C) | Liquid Temp. (°C) | Vacuum mm/Hg. Pressure |
|---|---|---|---|
| 1 | 31/76 | 116/144 | 0.1 |
| 2 | 133 | 154 | 0.1 |
| 3 | 135 | 158 | 0.1 |
| 4 | 135 | 166 | 0.1 |
| 5 | 152 | 195 | 0.1. |

Fractions 3 and 4 were bulked for subsequent evaluation of their organoleptic qualities.

### EXAMPLE III

### PRODUCTION OF NATURAL GAMMA-DODECALACTONE

### Reaction :

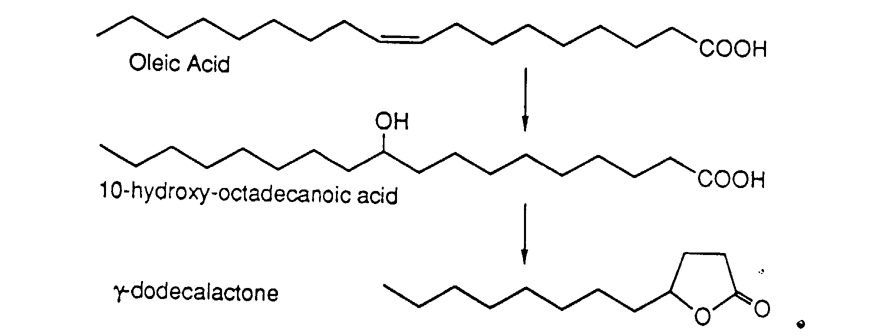

An experimental procedure was carried out identical to that of Example II, supra, with the exception that 3% olive oil was added to the fermenter before sterilization in Step 2, and no additional olive oil was added.

The results of Steps 1 and 2 are summarized in Tables 4 and 5 as follows:

**TABLE 4.**

| 10-HYDROXY STEARIC ACID PRODUCTION FROM OLEIC ACID | | | | | |
|---|---|---|---|---|---|
| Incubation Time (hrs) | Total Oleic Acid Pumped(g) | Crude 10-Hydroxy Acid(grams/liter) | Oleic Acid % | 10-Hydroxy Acid % | Yield of 10-Hydroxy Acid (gram/liter) |
| 24 | 113 | 13.2 | 34.8 | 38.3 | 5.1 |
| 48 | 161 | 20.0 | 32.9 | 39.0 | 7.8 |
| 120 | 300 | 27.0 | 25.5 | 47.2 | 12.8. |

**TABLE 5.**

| CONVERSION OF 10-HYDROXY STEARIC ACID TO GAMMA DODECALACTONE | | | | |
|---|---|---|---|---|
| Incubation Time (hrs) | Crude Gamma-Dodecalactone (g/l) | 10-Hydroxy Acid % | Gamma-Dodecalactone % in Crude | Gamma-Dodecalactone Yield (g/l) |
| 18 | 40.3 | 14.0 | 7.4 | 3.0 |
| 21 | 32.9 | 4.5 | 14.3 | 4.7 |
| 24 | 18.2 | - | 27.3 | 5.0. |

### Product Recovery:

A total of 5.7 liters of broth yielded 173 grams of crude gamma-dodecalactone. Fractional distillation of the crude resulted in 29.4 grams of product having a purity of 97.9%.

### EXAMPLE IV

### PRODUCTION OF NATURAL GAMMA-DODECALACTONE

### Reaction :

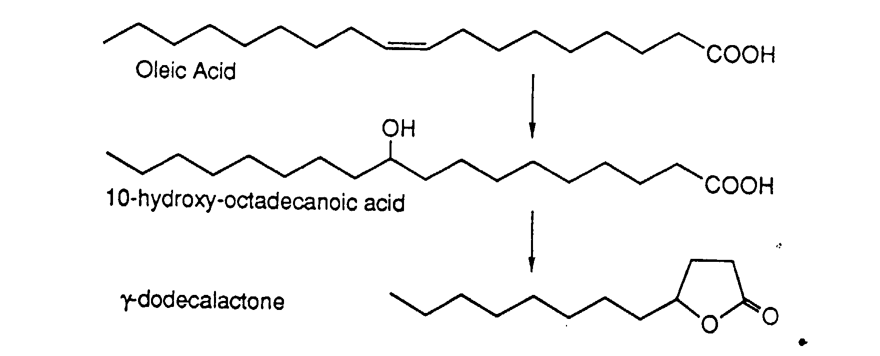

An experimental procedure was carried out identical to that of Example II except that 1.5% olive oil was added to the fermenter before sterilization in Step 2, and no additional olive oil was added

The results of Steps 1 and 2 are summarized in the following Tables 6 and 7:

**TABLE 6.**

| 10-HYDROXY STEARIC ACID PRODUCTION FROM OLEIC ACID | | | | | |
|---|---|---|---|---|---|
| Incubation Time (Hrs) | Total Oleic Acid Pumped(g) | Crude 10-Hydroxy Acid (g/l) | Oleic Acid % | 10-Hydroxy Acid % | Yield of 10-Hydroxy Acid (g/l) |
| 24 | 129 | 12.4 | 50.1 | 21.5 | 2.7 |
| 48 | 236 | 23.5 | 58.1 | 13.3 | 3.1 |
| 120 | 300 | 25.0 | 56.5 | 11.1 | 2.8 |
| 144 | 300 | 34.4 | 56.2 | 23.3 | 8.0. |

**TABLE 7.**

| CONVERSION OF 10-HYDROXY STEARIC ACID TO GAMMA DODECALACTONE | | | | |
|---|---|---|---|---|
| Incubation Time (Hrs) | Crude Gamma-Dodecalactone (g/l) | 10-Hydroxy Acid % | Gamma-Dodecalactone % In Crude | Gamma-Dodecalactone Yield (g/l) |
| 18 | 33.9 | - | 10.8 | 3.7 |
| 21 | 27.6 | 1.5 | 15.8 | 4.3. |

### Product Recovery:

A total of 6.2 liters of broth yielded 177 grams of crude gamma-dodecalactone. Fractional distillation of the crude resulted in 28.1 grams of product having a purity of 95%.

### EXAMPLE V

### PRODUCTION OF NATURAL GAMMA DODECALACTONE

### Reaction :

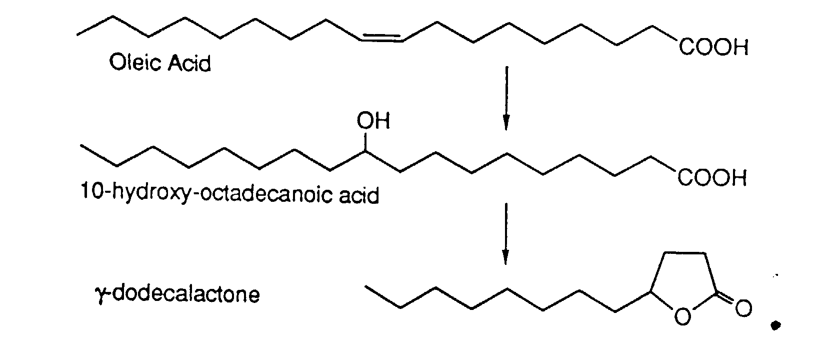

An experimental procedure was carried out identical to that of Example II except that in Step 2:
1. Candida petrophilum ATCC 20226 was used as the inoculum.
2. 2% Olive oil and 0.5% coconut oil was added to the fermenter before sterilization, and no additional co-oxidant oil was added.

The results of Steps 1 and 2 are summarized in the following Tables 8 and 9.

**TABLE 8.**

| 10-HYDROXY STEARIC ACID PRODUCTION FROM OLEIC ACID | | | | | |
|---|---|---|---|---|---|
| Incubation Time (Hrs) | Total Oleic Acid Pumped(g) | Crude 10-Hydroxy Acid (g/l) | Oleic Acid % | 10-Hydroxy Acid % | Yield of 10-Hydroxy Acid (g/l) |
| 24 | 92 | 11.3 | 33.8 | 34.0 | 3.9 |
| 120 | 284 | 46.2 | 38.0 | 38.0 | 17.6. |

**TABLE 9.**

| CONVERSION OF 10-HYDROXY STEARIC ACID TO GAMMA-DODECALACTONE | | | | |
|---|---|---|---|---|
| Incubation Time (Hrs) | Crude Gamma-Dodecalactone (g/l) | 10-Hydroxy Acid % | Gamma-Dodecalactone % In Crude | Gamma-Dodecalactone Yield (g/l) |
| 18 | 30.6 | 16.0 | 7.1 | 2.2 |
| 20.5 | 33.3 | 5.0 | 10.8 | 3.6 |
| 23 | 22.7 | - | 15.6 | 3.5. |

### Product Recovery:

A total of 6.0 liters of broth yielded 136.2 grams of crude. Fractional distillation of the crude resulted in 22.6 grams of product having a purity of 93.4%.

### EXAMPLE VI

### PRODUCTION OF NATURAL GAMMA-DODECALACTONE AND (Z) 6-DODECEN-4-OLIDE

### Reactions :

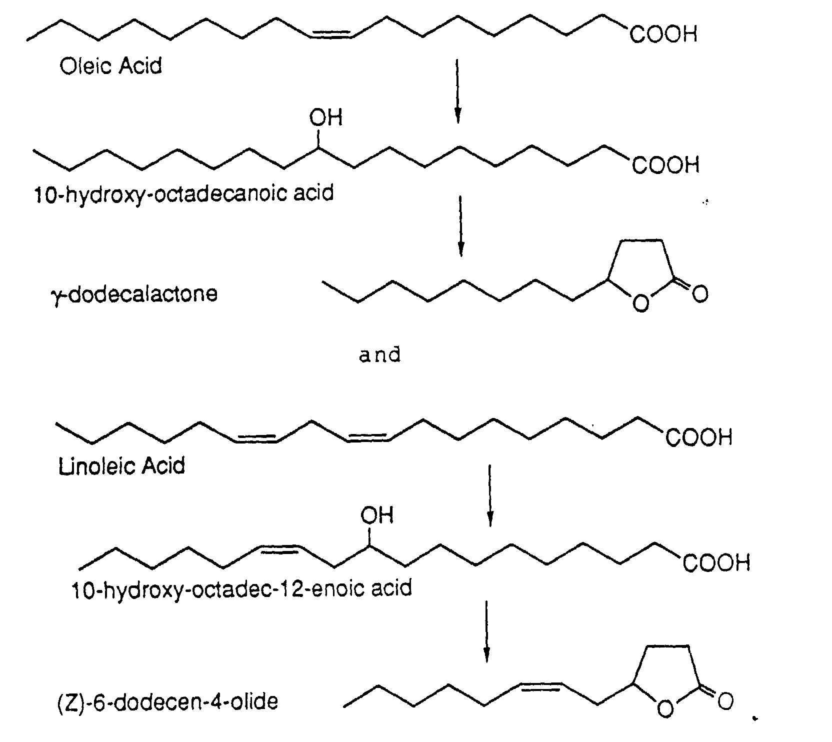

An experimental procedure was carried out identical to that of Example II, supra, except that:
1. A 50/50 mixture of oleic acid/linoleic acid was used as the substrate in Step 1.
2. 2% Olive oil was added to the fermenter before sterilization in Step 2 and no additional co-oxidant oil was added.

The results of Steps 1 and 2 are summarized in Table 10 and 11 as follows:

**TABLE 10.**

| PRODUCTION OF 10-HYDROXY ACIDS FROM OLEIC ACID AND LINOLEIC ACID | | | | | | |
|---|---|---|---|---|---|---|
| Time (Hrs) | Total Oleic/linoleic Acid Pumped(g) | Crude 10-Hydroxy Acids (g/l) | 10-Hydroxy Stearic Acid % In Crude | 10-Hydroxy Stearic Acid Yield (g/l) | 10-Hydroxy octadec-12 enoic Acid % In Crude | 10-Hydroxy octadec-12-enoic Acid Yield (g/l) |
| 72 | 400 | 42.1 | 8.4 | 3.5 | 7.3 | 3.1 |
| 96 | 400 | 47.2 | 10.8 | 5.1 | 9.6 | 4.5 |
| 144 | 400 | 46.1 | 10.1 | 4.7 | 9.7 | 4.5. |

**TABLE 11.**

| CONVERSION OF 10-HYDROXY ACIDS TO GAMMA LACTONES | | | | | |
|---|---|---|---|---|---|
| Incubation Time (Hrs) | Crude Gamma-Lactones (g/l) | Gamma-Lactones % In Crude | Gamma-Dodecalactone Yield(g/l) | 6-Dodecen-4-olide Yield;% In Crude | 6-Dodecen-4-olide (g/l) |
| 18 | 43.7 | 4.3 | 1.9 | 3.5 | 1.5. |

### Product Recovery:

A total of 8.3 liters of broth yielded 358 grams of crude containing a total of 7.8% of mixed lactones. The crude extract (358 grams) was fractionally distilled, resulting in 28.1 grams (fractions 4-6) of product. The combined fractions were topped (5% removed) under vacuum resulting in 26.7 grams product containing 47.9% gamma-dodecalactone and 47.9% 6-Dodecen-4-olide.

The distillation data is as follows:
(1/2" x 3" Vigreux column):

| Fraction No. | Vapor Temp. (°C) | Liquid Temp. (°C) | Vacuum mm/Hg. Pressure |
|---|---|---|---|
| 1 | 35/95 | 83/125 | 0.1 |
| 2 | 128 | 137 | 0.1 |
| 3 | 130 | 140 | 0.1 |
| 4 | 130 | 142 | 0.1 |
| 5 | 131 | 149 | 0.1 |
| 6 | 135 | 160 | 0.1 |
| 7 | 110 | 180 | 0.1. |

Fractions 4, 5 and 6 were bulked for further organoleptic evaluation.

### EXAMPLE VII

### PRODUCTION OF NATURAL GAMMA-DODECALACTONE, (Z)-6-DODECEN-4-OLIDE AND (Z,Z )-6-9-DODECADIEN-4-OLIDE

### Reactions :

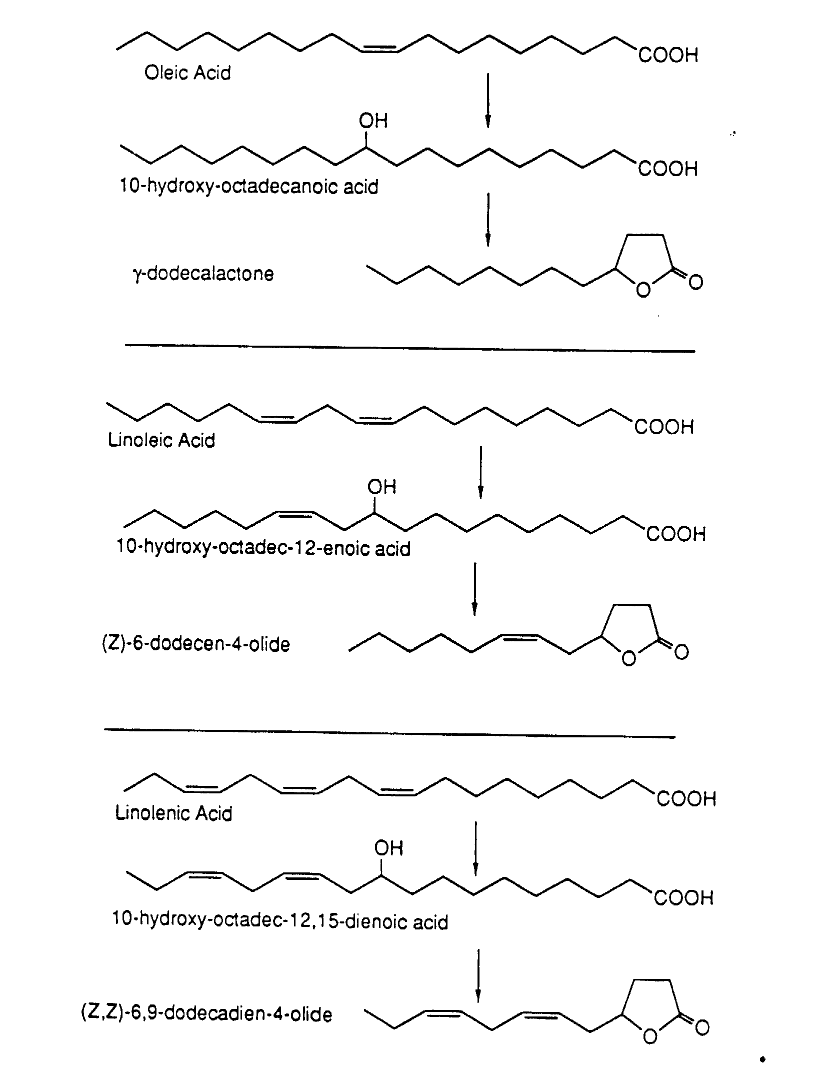

An experimental procedure was carried out identical to that of Example II with the exception that:
1. A 40/30/30 mixture of oleic acid/linoleic acid/linolenic acid was used as a substrate in Step 1.
2. 2% Olive oil was added to the fermenter before sterilization in Step 2, and no additional oil was added.

The results of Steps 1 and 2 are summarized in the following Tables 12 and 13:

**TABLE 12.**

| PRODUCTION OF 10-HYDROXY ACIDS FROM OLEIC ACID, LINOLEIC ACID AND LINOLENIC ACID | | | |
|---|---|---|---|
| Incubation Time (Hrs) | Total (grams) Oleic/Linoleic/Linolenic Acid Pumped | Crude Hydroxy Acids (g/l) | 10-Hydroxy-Octadecanoic Acids Yield (g/l) |
| 72 | 400 | 35.1 | 4.4 |
| 96 | 400 | 39.7 | 6.0 |
| 144 | 400 | 40.1 | 7.5. |

**TABLE 13.**

| CONVERSION OF 10-HYDROXY ACIDS TO GAMMA LACTONES | | | | |
|---|---|---|---|---|
| Incubation Time (Hrs) | Crude Gamma-Lactones (g/l) | Gamma-Dodecalactone % In Crude | 6-Dodecen-4-Olide % In Crude | 6,9-Dodecadien-4-Olide % In Crude |
| 19 | 45.7 | 3.7 | 1.7 | 4.2. |

### Product Recovery:

A total of 8.8 liters of broth yielded 238 grams of crude containing a total 14.5% of mixed lactones. The crude extract (238 grams) was fractionally distilled, resulting in 21 grams (fractions 3-5) of product. The combined fractions were topped (5% removed) under vacuum resulting in 20.0 grams product containing 48.9% gamma-dodecalactone, 22.1% (Z)6-Dodecen-4-Olide and 22.2% (Z,Z)-6,9-Dodecadien-4-Olide.

### EXAMPLE VIII

### PRODUCTION OF GAMMA-DODECALACTONE AND ANALYTICAL PROCEDURE FOR ANALYSIS OF CRUDE DODECALACTONE AS METHYL ESTER

### Reactions :

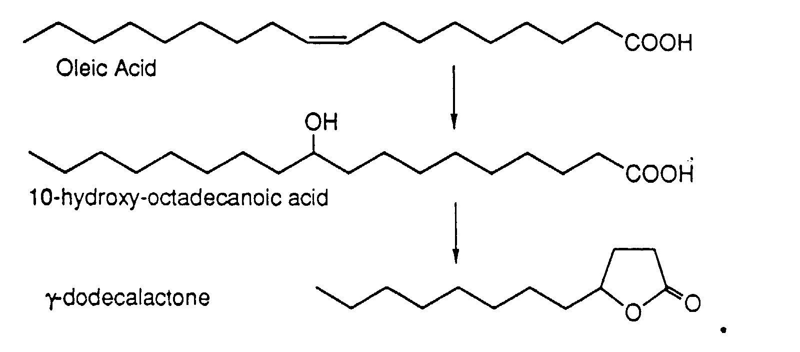

An inoculum medium is prepared as follows:

| Inoculum Medium: | |
|---|---|
| Difco Yeast Extract | 0.5% |
| KH₂PO₄ | 0.4% |
| MgSO₄.7H₂O | 0.05% |
| TWEEN®80 | 0.02% |
| pH = 7.0, adjusted before sterilization with 50% aqueous KOH. | |

Fernbach flasks with 500 ml of medium are sterilized at 121°C for 30 minutes. After sterilization 0.5 ml of sterile 50% dextrose per 100 ml of medium is added. Flasks are inoculated from a slant of Pseudomonas sp. NRRL B-2994 and incubated at 25°C, 100 RPM, for 24 hours to form an "inoculum culture".

### Production Medium:

A production medium is prepared as follows:

| | |
|---|---|
| TASTONE®900 | 0.5% |
| KH₂PO₄ | 0.4% |
| MgSO₄.7H₂O | 0.05% |
| TWEEN®80 | 0.02% |
| Oleic Acid | 0.1% |
| pH = 6.5, maintained during fermentation with 25% NaOH | |

8 Liters of medium are sterilized in 14 liter fermenters at 121°C for 30 minutes. After sterilization, 5 ml of sterile 50% dextrose per liter of medium are added.

### Parameters:

Inoculum: 500 ml of inoculum culture (are added to each 8 liter production medium batch)
Temperature: 26°C
Aeration: 0.1 v/v/m
Agitation: 420 RPM

### Process:

After 24 hours of growth, aeration is stopped, agitation is increased to 900 RPM, and oleic acid pumping begins as shown in Table 14, infra.

**TABLE 14.**

| 10-HYDROXY STEARIC ACID PRODUCTION FROM OLEIC ACID | | | | | |
|---|---|---|---|---|---|
| Incubation Time (Hrs) | Total Oleic Acid Pumped (grams) | Crude 10-Hydroxy Acid (g/l) | Oleic Acid % | 10-Hydroxy Acid % | Yield (10-Hydroxy Acid) Grams Per Liter |
| 24 | 108 | 8.5 | 38.7 | 27.0 | 2.3 |
| 48 | 199 | 18.6 | 40.9 | 31.0 | 5.8 |
| 120 | 300 | 32.5 | 26.0 | 45.0 | 14.4. |

### Sample Analysis:

A sample of broth (250 ml) is acidified to a pH of 2 using 50% H₂SO₄. The broth is extracted three times with an equal volume of ethyl acetate. The combined extracts are washed twice with saturated aqueous sodium chloride. The solvent is evaporated under vacuum. The oil is treated with BF₃-methanol and analyzed by GC.

### Results of Step 1:

The results of Step 1 are summarized in Table 14, set forth, supra.

### Step 2: Conversion of 10-Hydroxy stearic acid to gamma-dodecalactone.

The following inoculum medium was prepared:

| Inoculum medium: | |
|---|---|
| Beef Extract | 2.0% |
| KH₂PO₄ | 0.01% |
| MgSO₄.7H₂O | 0.005% |
| TWEEN® 80 | 0.02% |
| Olive Oil | 1.0% |
| pH = 7 | |

100 ml of Medium were sterilized in 500 ml flasks for 20 minutes at 121°C. Flasks were inoculated from a slant of Yarrowia lipolytica ATCC 34088, and incubated at 25°C, 150 RPM for 24 hours.

2% TASTONE® was added to the fermenter from Step 1 containing the previously-produced 10-hydroxy stearic acid, and sterilized at 121°C for 20 minutes. The fermenter was then cooled to 28°C and inoculated with 200 ml of inoculum. After 20 hours incubation 30 grams of olive oil were added to the fermenter.

### Fermenter Parameters:

Inoculum: 200 ml of inoculum culture
Temperature: 28°C
Aeration: 0.5 v/v/m
Agitation: 620 RPM
pH: 7, maintained with 25% NaOH
Back pressure: 7.5 psi
Antifoam: Mazu DF 100, automatically added as needed.

### Results of Step 2:

The results of Step 2 are summarized in Table 15 as follows:

**TABLE 15.**

| CONVERSION OF 10-HYDROXY STEARIC ACID TO GAMMA DODECALACTONE. | | | | |
|---|---|---|---|---|
| Incubation Time (Hrs) | Crude Gamma-Dodecalactone (g/l) | % 10-Hydroxy Acid In Crude | Gamma-Dodecalactone per Crude | Gamma-Dodecalactone yield (g/l) |
| 18 | 18.8 | 49.8 | 18.6 | 3.5 |
| 20 | 16.6 | 45.0 | 17.3 | 2.9 |
| 23 | 17.1 | 37.0 | 11.2 | 1.9. |

### Analysis:

### Reactions (exemplary):

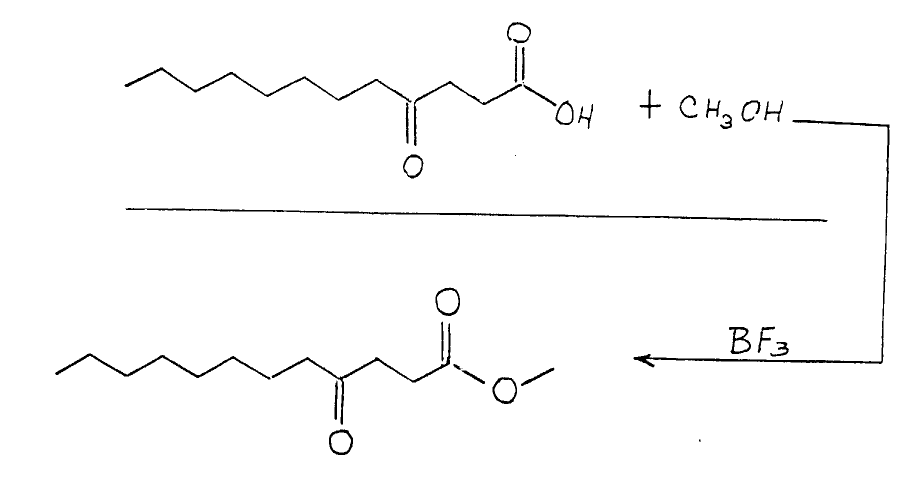

and 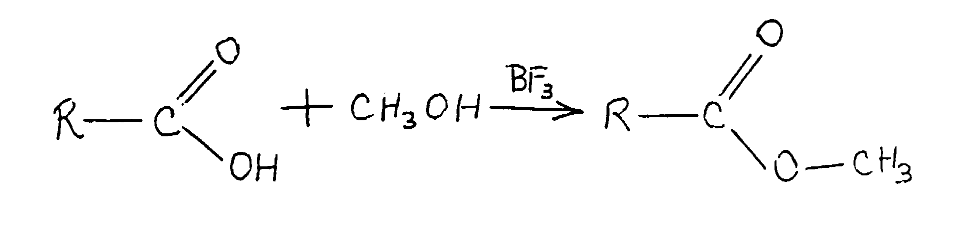 wherein R represents carboxylic acid residues.

At the end of the fermentation, the pH was adjusted to 4 using citric acid and the broth was then heated to 100°C for 30 minutes. The entire broth (6.5 liters) was extracted 3 times with 1/3 volume ethyl acetate. The extracts were combined and the solvent was removed under reduced pressure.

One gram of the crude extract is weighed into a vial. 5 ml of BF₃/methanol reagent (Supelco, Inc. Cat. No. 3-302l) is added to the vial. The vial is capped and heated in a boiling water bath for 5 minutes. The reaction mixture is then transferred to a small (60 ml) separatory funnel, washed two times with 10-20 ml portions of distilled water and the oil layer is removed for GLC analysis.

The GLC profile is set forth in Figure 30. The GLC profile of Figure 30 is described in detail in the section entitled "DETAILED DESCRIPTION OF THE DRAWINGS", supra.

### Product Recovery:

The crude extract (110 grams) was fractionally distilled resulting in 12.7 grams of product having a purity of 90.2%. The distillation was carried out at 135°C vapor temperature and 158-166°C liquid temperature at a pressure of 0.1 mm/Hg.

### EXAMPLE IX

### PRODUCTION OF NATURAL 10-HYDROXY STEARIC ACID

### Reaction :

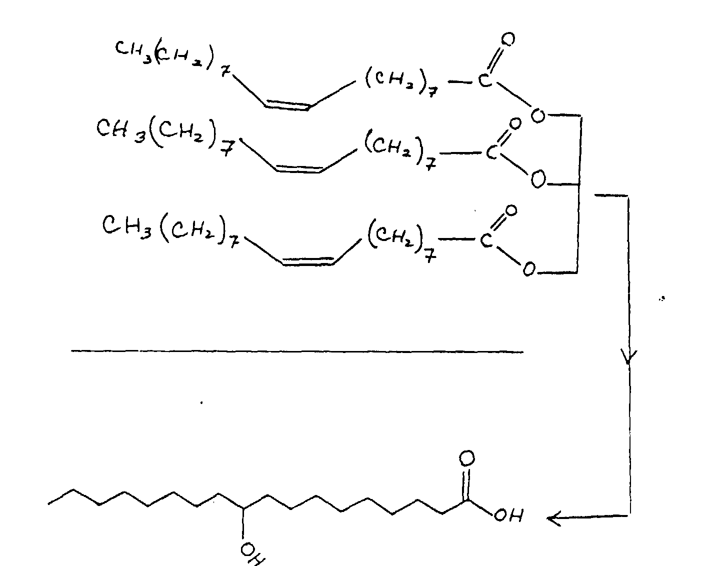

### STEP 1:

The experimental procedure is the same as that set forth in Example II, Step 1, supra, except for the following:
1. In the inoculum preparation two 500 ml flasks containing 100 ml of medium are sterilized at 121°C for a period of 30 minutes. After sterilization 0.5 ml of sterile 50% dextrose per 100 ml of medium is added. The flasks are inoculated from a slant of Pseudomonas sp. NRRL B-2994 and incubated at 25°C, 150 RPM, for 24 hours.
2. In the production medium, 1.0% TASTONE®900 is used.
3. Inoculum level: 200 ml of inoculum culture.
4. Aeration: 0.3 v/v/m.
5. During the process, sunflower oil (high oleic) shown according to the structure: 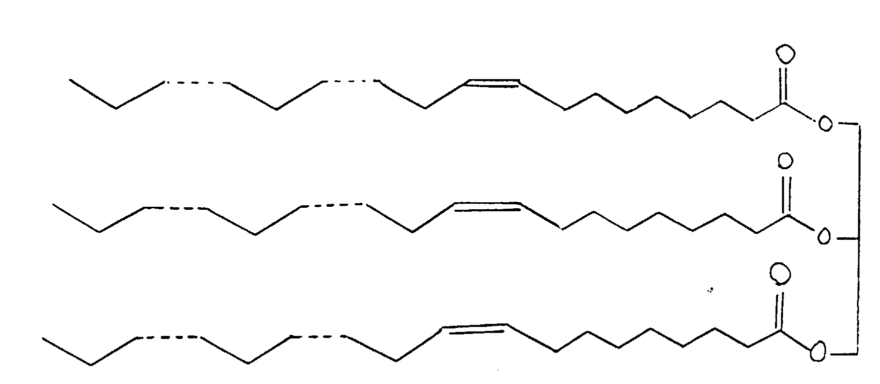 is used in place of oleic acid and 0.5 grams of of Lipase MY is added per liter of medium as pumping begins.

### Results of Step 1:

The results of Step 1 are summarized in Table 16 as follows:

**TABLE 16.**

| 10-HYDROXY STEARIC ACID PRODUCTION FROM SUNFLOWER OIL (HIGH OLEIC) | | | | | |
|---|---|---|---|---|---|
| Incubation Time (Hrs) | Total Oleic Acid Pumped (Contained In Sun flower Oil As Triglyceride) (g) | Crude 10-Hydroxy Acid (g/l) | Oleic Acid % | 10-Hydroxy Acid % | Yield of 10-Hydroxy Acid (g/l) |
| 24 | 300 | - | - | - | - |
| 48 | 300 | 31.0 | 10.3 | 74.3 | 23.0. |

### EXAMPLE X

### PATCHOULI PERFUME FORMULATION

The following mixture is prepared:

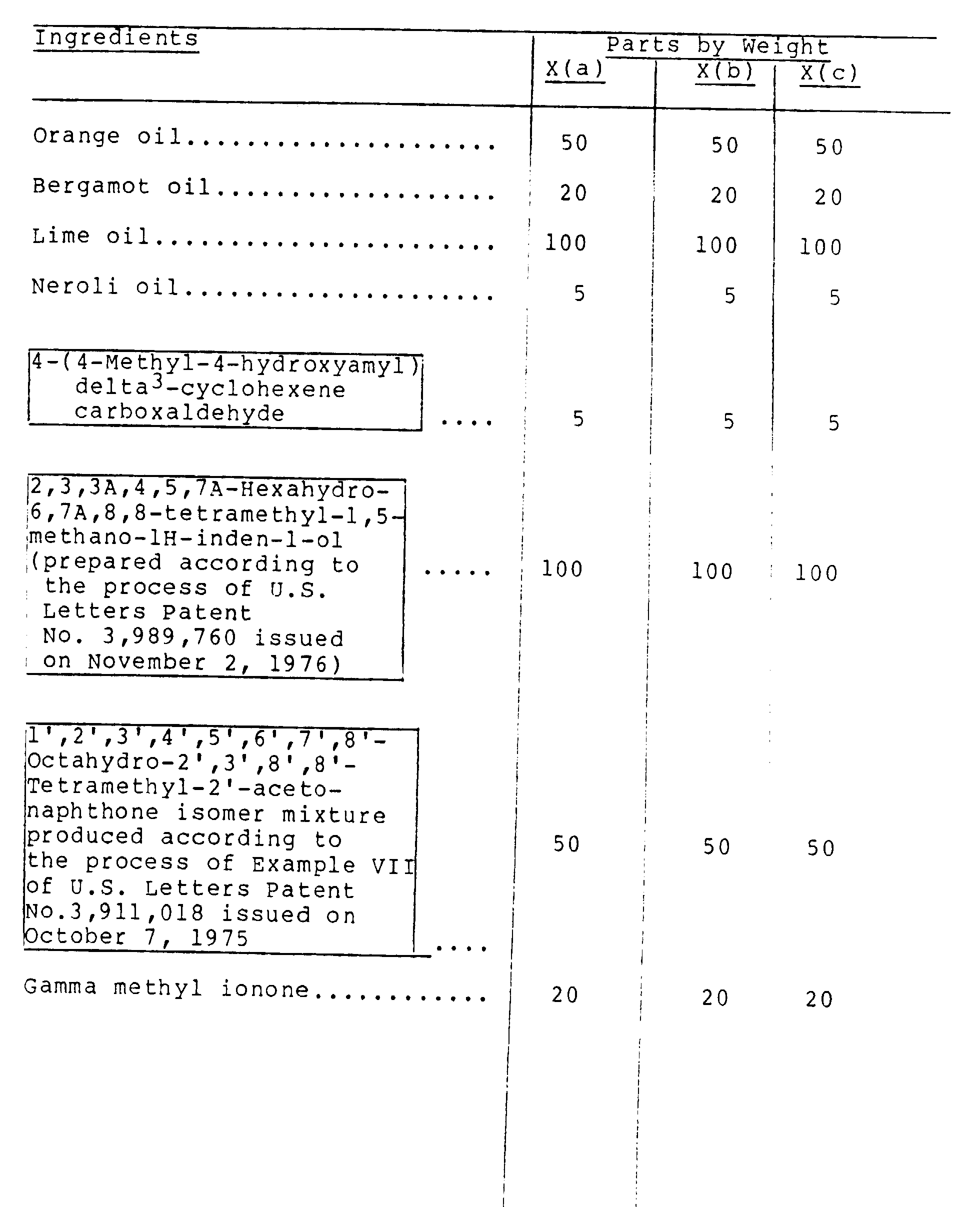

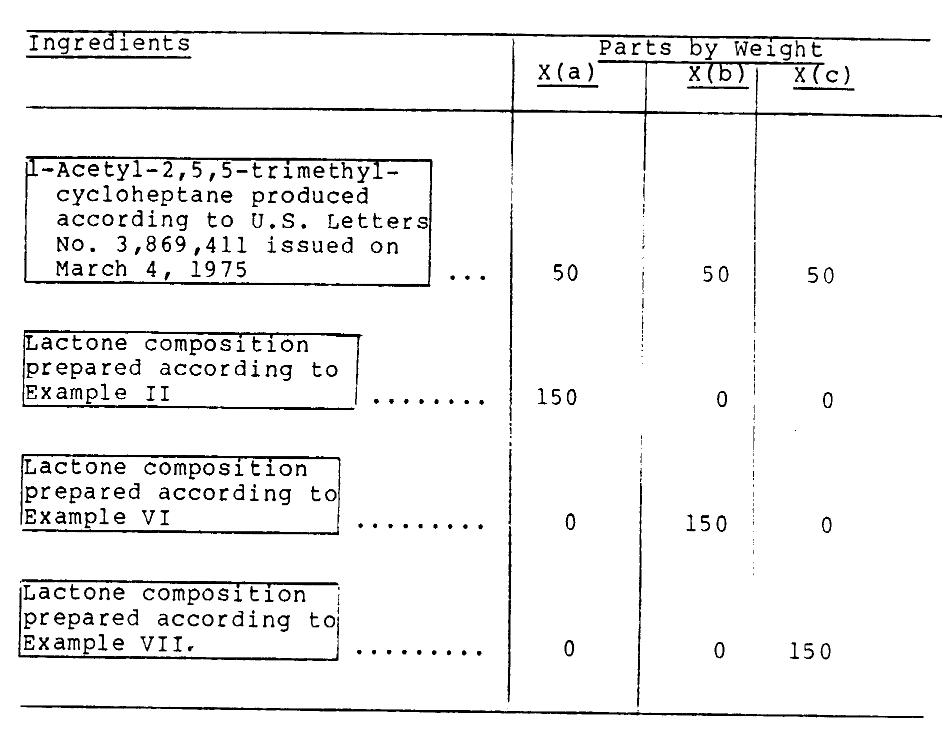

The mixtures of lactones prepared according to Examples II, VI and VII add to the formulations of Examples X(a), X(b) and X(c) sophisticated, sweet, fruity, peach-like undertones with green and herbaceous topnotes. Accordingly, the perfume formulations of each of Examples X(a), X(b) and X(c) can be described as "patchouli, with sweet, fruity and peach-like undertones, and green and herbaceous topnotes".

### EXAMPLE XI

### PREPARATION OF SOAP COMPOSITIONS

One hundred grams of soap chips are produced according to Example V of U.S. Patent No. 4,058,487 issued on November 15, 1977, the specification for which is incorporated herein by reference, as follows:

The sodium salt of an equal mixture of C₁₀-C₁₄ alkane sulfonate (95% active), 40 pounds, is dissolved in a mixture of 80 pounds of anhydrous isopropanol and 125 pounds of deionized water at 150°F. In this mixture is dissolved 10 pounds of partially hydrogenated coconut oil fatty acids and 15 pounds of sodium mono-C₁₄ alkyl maleate, and the pH of this solution is adjusted to 6.0 by the addition of a small amount of 50% aqueous solution of sodium hydroxide. The isopropanol is distilled off and the remaining aqueous solution is drum dried. The resulting solids are then blended in a chip mixture with 10 pounds of water, 0.2 pounds of titanium hydroxide and 0.7 pounds of one of the perfume ingredients set forth in Table 17 below. The chips are then plodded into logs, cut to size and finally stamped into bars having a pH of approximately 6.9.

Each of the perfumed soaps produced by means of the foregoing procedure manifests an excellent aroma as set forth in Table 17, infra.

**TABLE 17**

| Ingredient | Fragrance Profile |
|---|---|
| Lactone composition of Example II. | A peach and apricot aroma profile. |
| Lactone composition of Example VI. | A peach and apricot aroma profile. |
| Lactone composition of Example VII. | A peach and apricot aroma profile. |
| Perfume composition of Example X(a). | Patchouli, with sweet, fruity and peach-like undertones with green and herbaceous topnotes. |
| Perfume composition of Example X(b). | Patchouli, with sweet, fruity and peach-like undertones, and green and herbaceous topnotes. |
| Perfume composition of Example X(c). | Patchouli, with sweet, fruity and peach-like undertones, and green and herbaceous topnotes. |

### EXAMPLE XI

### TOACCO FLAVOR FORMULATION

Cigarettes are produced using the following tobacco formulations:

| Ingredients | Parts by Weight |
|---|---|
| Bright | 40.1 |
| Burley | 24.9 |
| Maryland | 1.1 |
| Turkish | 11.6 |
| Stem (flue cured) | 14.2 |
| Glycerine | 2.8 |
| H₂O | 5.3. |

At the rate of 0.2%, the following tobacco formulation is applied to all of the cigarettes produced with the above tobacco formulation:

| Ingredients | Parts by Weight |
|---|---|
| Ethyl butyrate | 0.05 |
| Ethyl valerate | 0.05 |
| Maltol | 2.00 |
| Cocoa extract | 26.00 |
| Coffee extract | 10.00 |
| Ethyl alcohol (95%) | 20.00 |
| H₂O | 41.900. |

To portions of 50% of the cigarettes at levels of 10 and 20 ppm, a mixture of lactones produced according to Example VII is added.

These cigarettes are hereinafter called "experimental" cigarettes. The cigarettes without the mixture of lactones are hereinafter called "control" cigarettes. The control and experimental cigarettes are then evaluated by paired comparison and the results are as follows:
(a) In aroma, the experimental cigarettes are all found to be more aromatic with Turkish tobacco-like nuances.
(b) In smoke flavor, the experimental cigarettes are all found to be more aromatic, more sweet with Turkish tobacco, oriental-like nuances than the control cigarettes.

The experimental cigarettes containing the mixture of lactones are found to be fruity and have pleasant aesthetically pleasing fruity notes in addition.

### EXAMPLE XII

### PUDDING

At the rate of 0.8 ppm the mixture of lactones produced according to Example II are added to a royal butterscotch pudding. Pleasant aesthetically pleasing peach nuances were added to the butterscotch pudding causing a panel of 30 members to prefer the butterscotch pudding with the mixture of lactones added thereto.

## Claims

1. A process for the preparation of one or more gamma dodecalactone derivatives defined according to the structure: 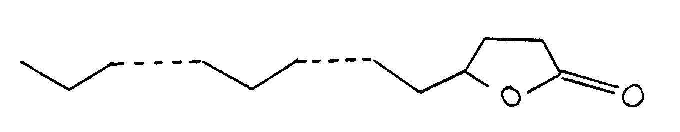 wherein the dashed lines represent the same or different carbon-carbon bonds; with each of the dashed lines representing a carbon-carbon single bond or a carbon-carbon double bond consisting of the sequential steps of:
(i) fermenting at a pH in the range of from about about 5.5 up to about 9.0 and at a temperature in the range of from about 20°C up to about 35°C at least one carboxylic acid derivative defined according to a structure selected from the group consisting of: 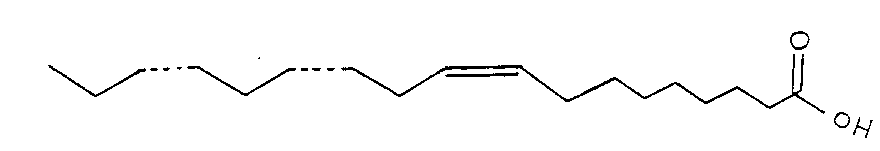 and 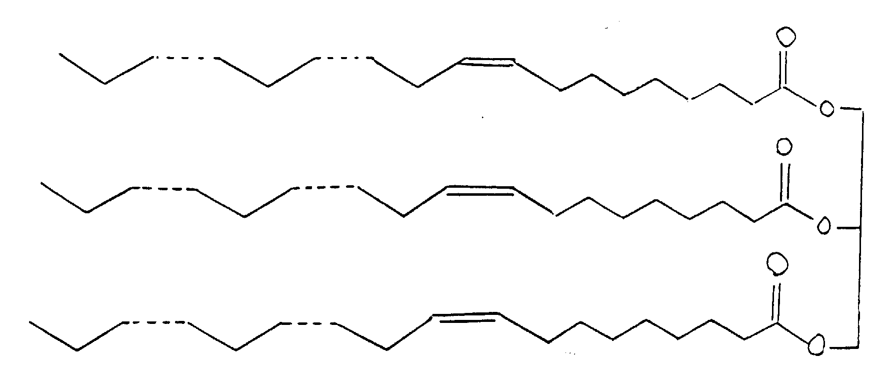 with the microorganism, Pseudomonas sp. NRRL R-2994 or a 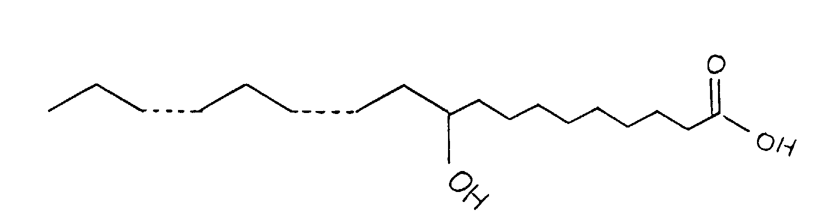 -producing mutant thereof for a period of time sufficient to produce one or more hydroxy carboxylic acids defined according to the structure: 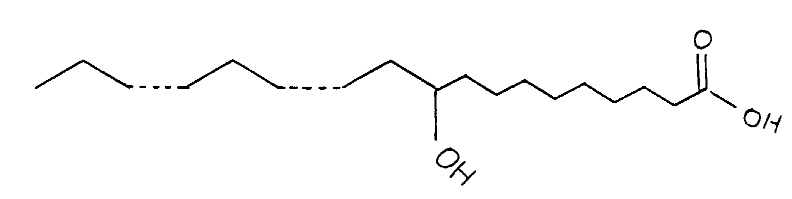 according to the reactions: 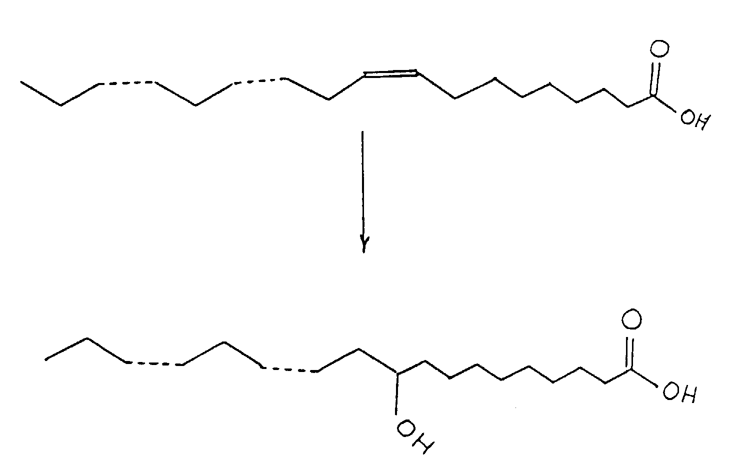 and/or 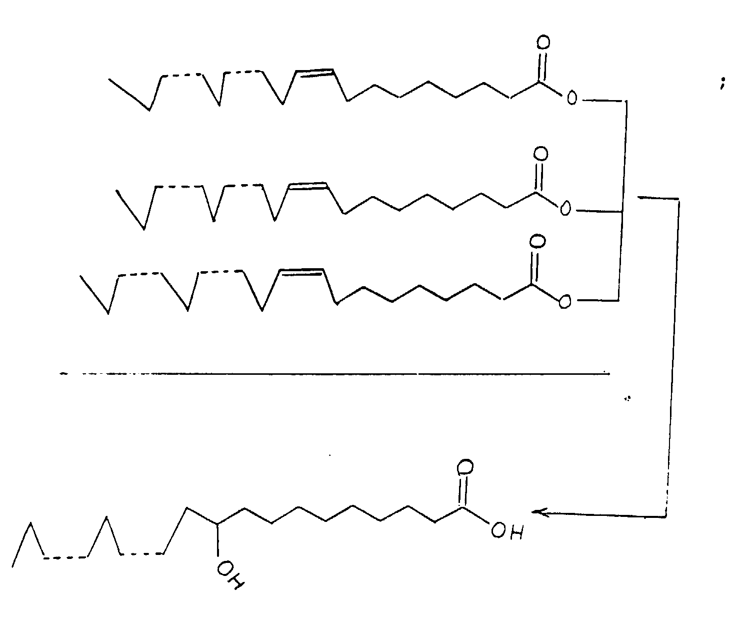 and
(ii) fermenting at least one of the hydroxy carboxylic acids defined according to the structure: 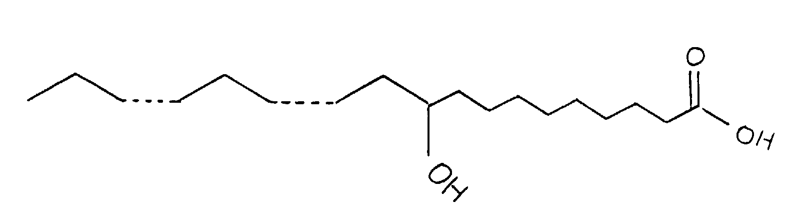 at a pH of from about 5.0 up about 7.5 at a temperature in the range of from about 20°C up to about 35°C with a microorganism selected from the group consisting of:
Candida brumptii IFO 0731;
Candida deformans ATCC 22969;
Candida ethanothermophilum ATCC 20380;
Candida iberica ATCC 26318;
Candida kefyr ATCC 42265;
Candida oleophila ATCC 20177;
Candida rugosa ATCC 14830;
Candida sp. ATCC 20473;
Candida sp. ATCC 20504;
Candida utilis ATCC 9226;
Candida utilis ATCC 15239;
Candida utilis ATCC 20248;
Candida utilis ATCC 42181;
Candida zeylanoides ATCC 15585;
Yarrowia lipolytica ATCC 8661;
Yarrowia lipolytica ATCC 8662;
Yarrowia lipolytica ATCC 16617;
Yarrowia lipolytica ATCC 16618;
Yarrowia lipolytica ATCC 20255;
Yarrowia lipolytica ATCC 20324;
Yarrowia lipolytica ATCC 20341;
Yarrowia lipolytica ATCC 46483;
Candida petrophilum ATCC 20226; and
Yarrowia lipolytica ATCC 34088
or a 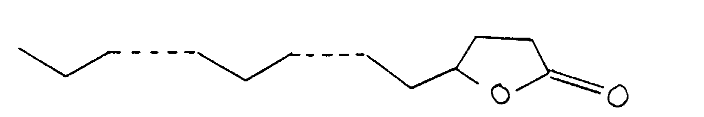 -producing mutant thereof for a period of time to produce one or more lactones defined according to the structure: 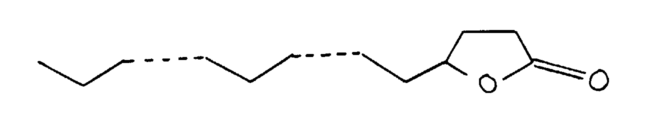 according to the reaction:

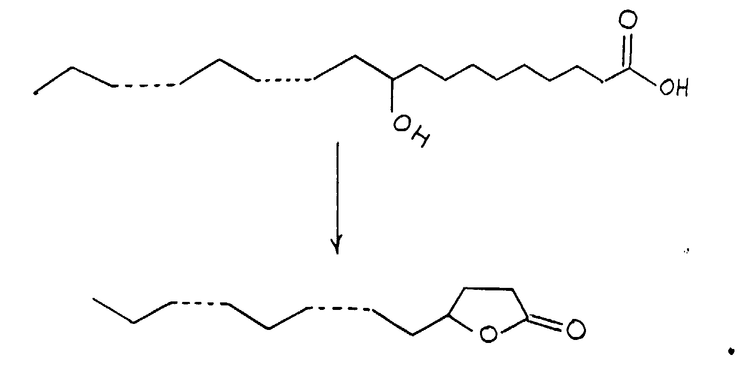

2. The process of Claim 1 wherein the time of reaction for Step 1 is from about 36 up to about 150 hours; and the time of reaction for Step 2 is from about 18 up to about 30 hours.

3. The process of Claim 1 wherein Step 2 is carried out in the presence of an olive oil co-oxidant.

4. A process for the preparation of a hydroxy carboxylic acid having the structure: 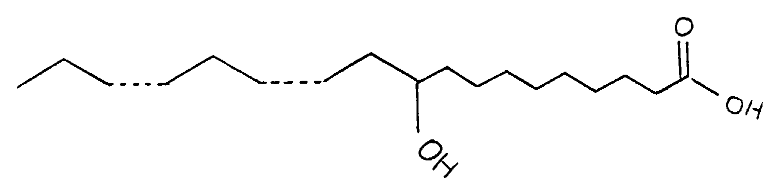 wherein the dashed lines represent the same or different carbon-carbon bonds; with each of the dashed lines representing a carbon-carbon single bond or a carbon-carbon double bond consisting of the step of fermenting at a pH in the range of from about 5.5 up to 9.0 and at a temperature in the range of from about 20°C up to about 35°C at least one carboxylic acid derivative defined according to a structure selected from the group consisting of: 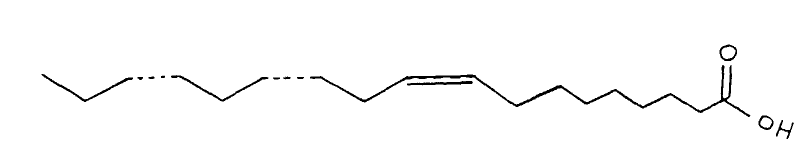 and 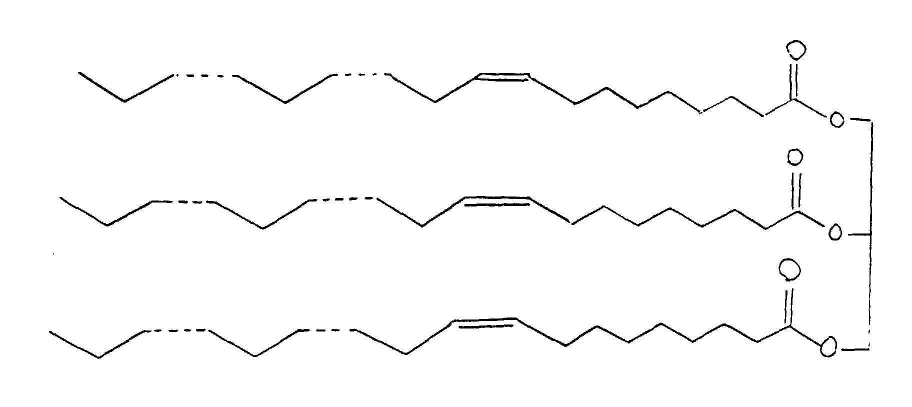 with the microorganism, Pseudomonas sp. NRRL B-2994 or a 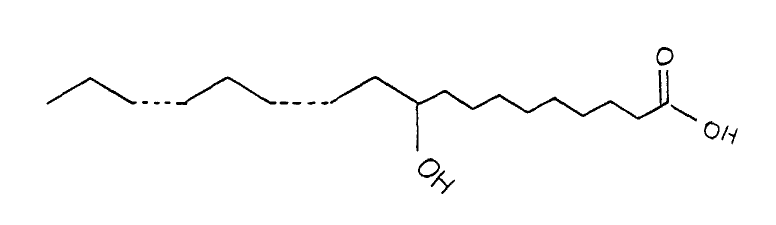 -producing mutant thereof for a period of time sufficient to produce one or more hydroxy carboxylic acids defined according to the structure: 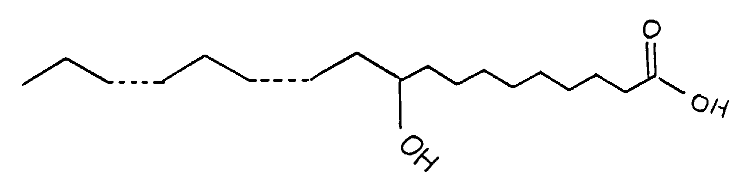 according to the reaction: 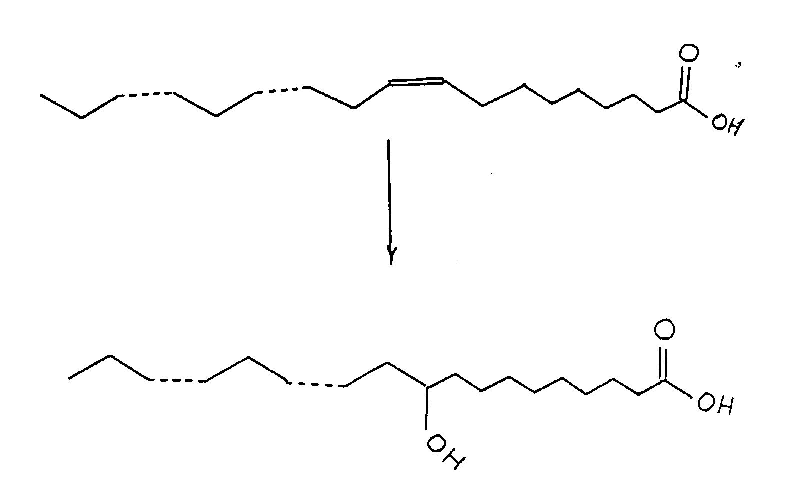 and/or

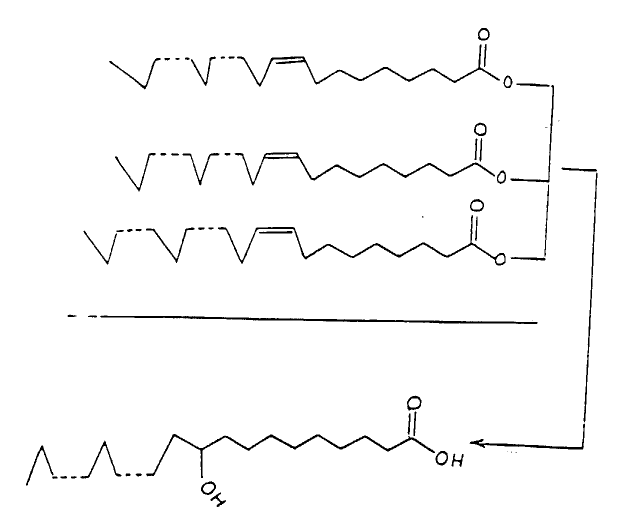

5. The process of Claim 4 wherein the time of reaction is from about 36 up to 150 hours.

6. The process of Claim 1 wherein the microorganism used in Step 2 is Yarrowia lipolytica ATCC 34088.

7. The process of Claim 1 wherein in Step 2 the microorganism used is Candida petrophilum ATCC 20226.

8. The process of Claim 4 wherein the carboxylic acid derivative employed has the structure:

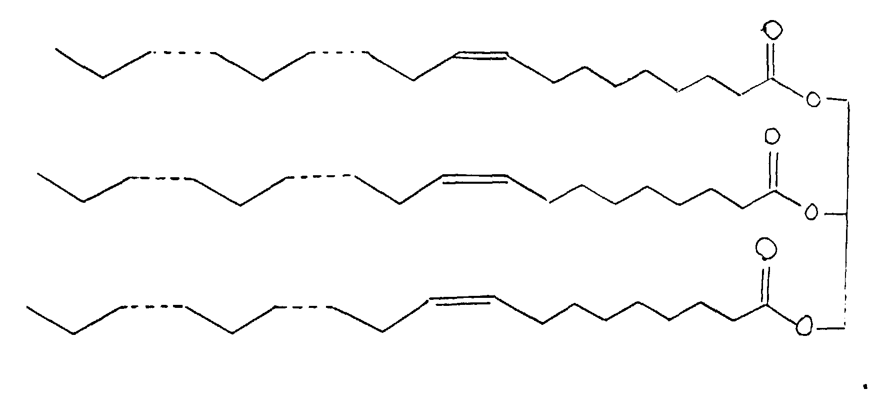

9. The process of Claim 8 wherein the carboxylic acid derivative is employed in the form of sun flower oil.

10. The process of Claim 4 wherein the carboxylic acid derivative employed as the reactant is defined according to the structure:

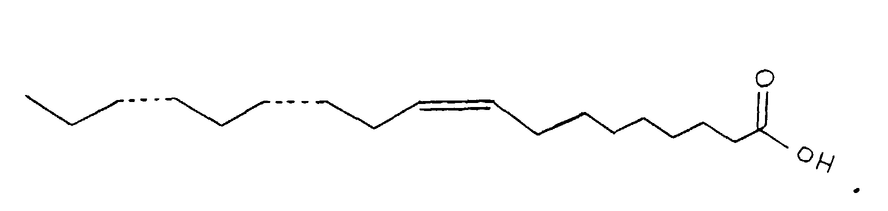

11. The process of Claim 10 wherein in the structure: 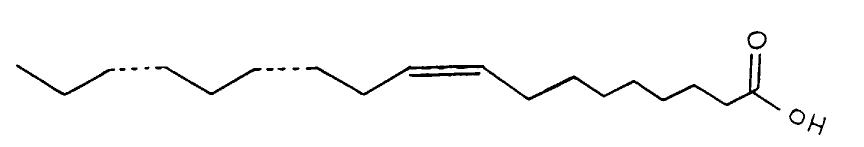 both dashed lines are carbon-carbon single bonds.

12. The process of Claim 10 wherein the carboxylic acid derivative employed is a mixture of oleic acid and linoleic acid.

13. The process of Claim 10 wherein the carboxylic acid derivative employed is a mixture of oleic acid, linoleic acid and linolenic acid.

14. The process of Claim 1 wherein in Step 1 the carboxylic acid derivative employed as a reactant is oleic acid.

15. The process of Claim 1 wherein in Step 1 the carboxylic acid derivative employed as a reactant is a mixture of oleic and linoleic acid.

16. The process of Claim 1 wherein in Step 1 the carboxylic acid derivative employed is a mixture of oleic acid, linoleic acid and linolenic acid.

17. A composition of matter comprising Lipase and Pseudomonas sp. NRRL B-2994.

## Patentansprüche

1. Verfahren zur Herstellung eines oder mehrerer gamma-Dodecalactonderivate der folgenden Struktur: 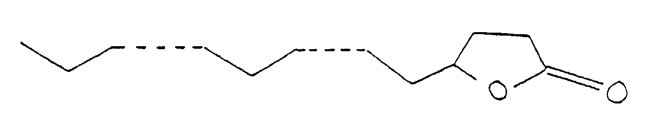 worin die gestrichelten Linien die gleiche oder verschiedene Kohlenstoff-Kohlenstoff-Bindungen bedeuten und jede der gestrichelten Linien eine Kohlenstoff-Kohlenstoff-Einfachbindung oder eine Kohlenstoff-Kohlenstoff-Doppelbindung bedeutet, bestehend aus folgenden Stufen:
(i) Fermentieren bei einem pH von etwa 5,5 bis etwa 9,0 und bei einer Temperatur von etwa 20 °C bis etwa 35 °C mindestens eines Carbonsäurederivats der folgenden Struktur ausgewählt aus der Gruppe bestehend aus 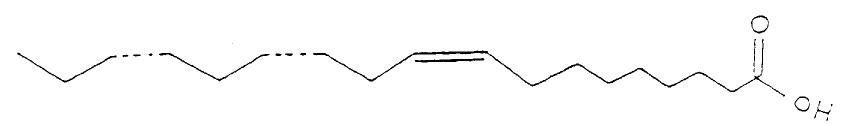 und 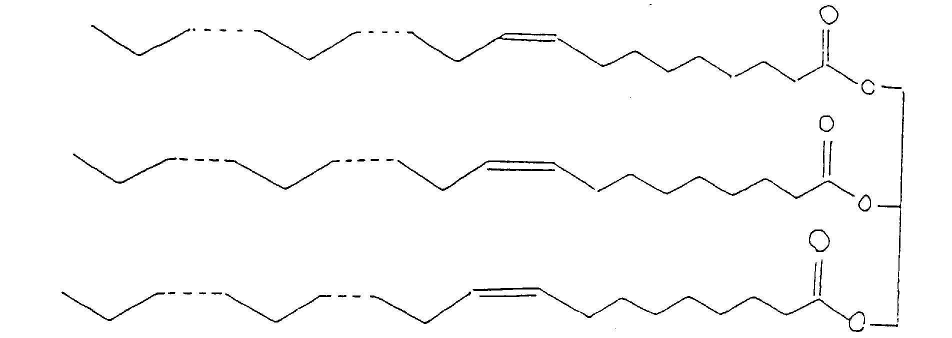 mit dem Mikroorganismus Pseudomonas sp. NRRL R-2994 oder eines 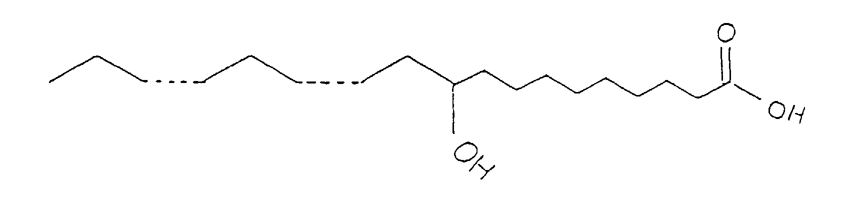 produzierenden Mutanten davon während einer solchen Zeitspanne, daß eine oder mehrere Hydroxycarbonsäuren der folgenden Struktur 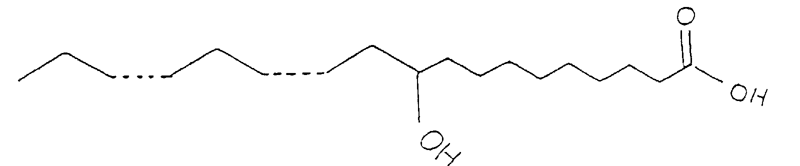 gemäß den Reaktionen: 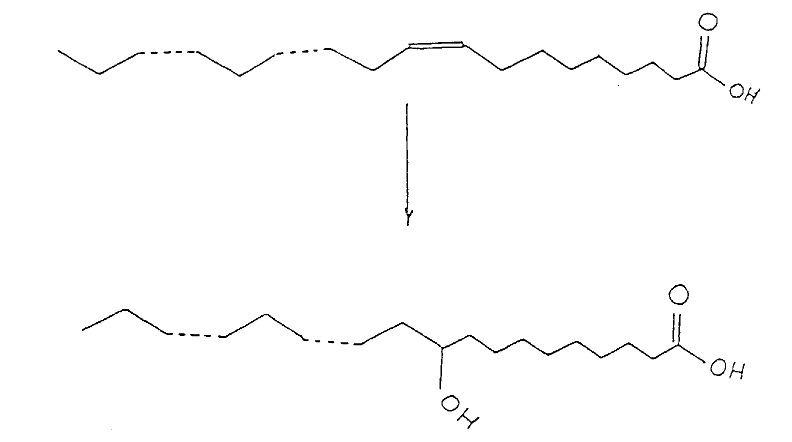 und/oder 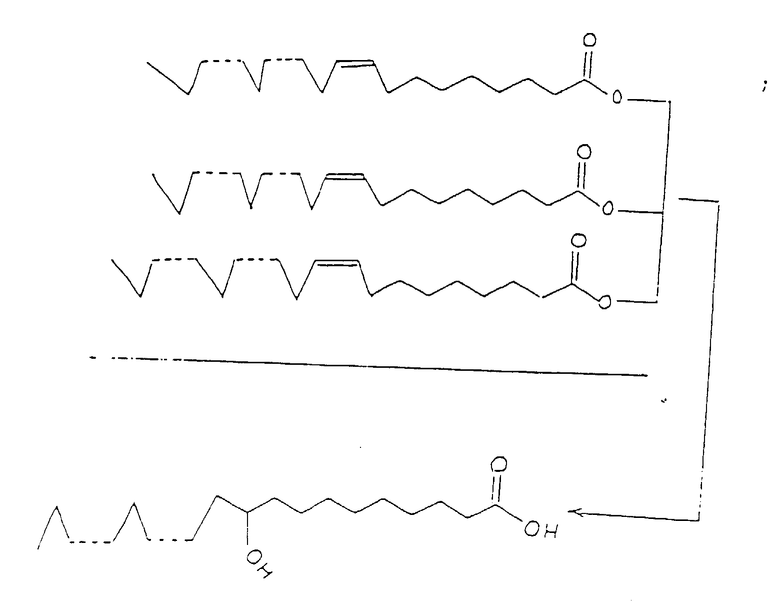 hergestellt wird und
(ii) Fermentieren mindestens einer der Hydroxycarbonsäuren der folgenden Struktur: 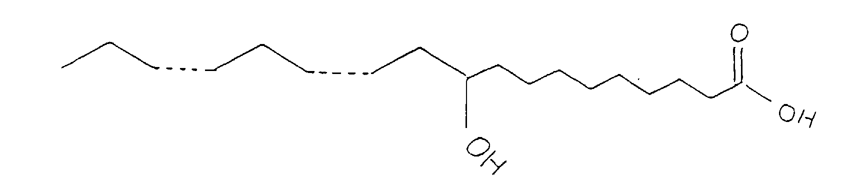 bei einem pH von etwa 5,0 bis etwa 7,5 bei einer Temperatur von über 20°C bis etwa 35 °C mit einem Mikroorganismus ausgewählt aus der Gruppe bestehend aus: Candida brumptii IFO 0731;
Candida deformans ATCC 22969;
Candida ethanothermophilum ATCC 20380;
Candida iberica ATCC 26318;
Candida kefyr ATCC 42265;
Candida oleophila ATCC 20177;
Candida rugosa ATCC 14830;
Candida sp. ATCC 20473:
Candida sp. ATCC 20504:
Candida utilis ATCC 9226;
Candida utilis ATCC 15239;
Candida utilis ATCC 20248;
Candida utilis ATCC 42181;
Candida zeylanoides ATCC 15585;
Yarrowia lipolytica ATCC 8661;
Yarrowia lipolytica ATCC 8662;
Yarrowia lipolytica ATCC 16617;
Yarrowia lipolytica ATCC 16618;
Yarrowia lipolytica ATCC 20255;
Yarrowia lipolytica ATCC 20324;
Yarrowia lipolytica ATCC 20341;
Yarrowia lipolytica ATCC 46483;
Candida petrophilum ATCC 20226; und
Yarrowia lipolytica ATCC 34088
oder eines 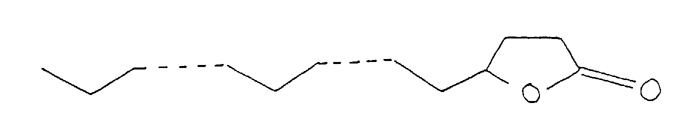 produzierenden Mutanten davon während einer solcher Zeitspanne, daß ein oder mehrere Lactone der folgenden Struktur; 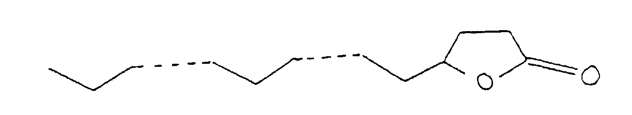 gemäß der Umsetzung: 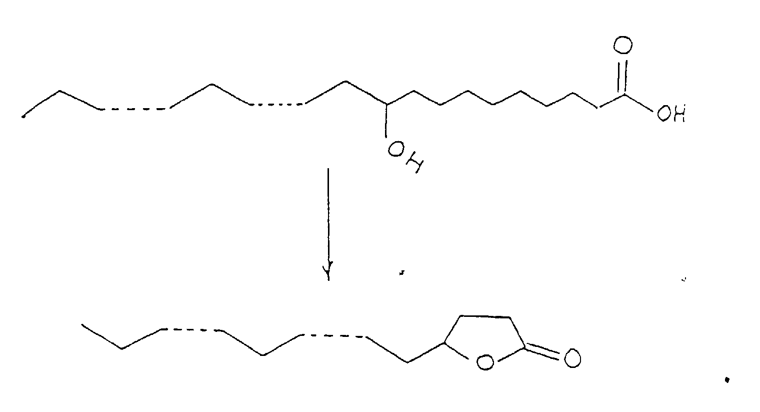 erhalten werden.

2. Verfahren nach Anspruch 1,
bei dem die Reaktionszeit in der Stufe 1 etwa 36 bis etwa 150 h und die Reaktionszeit in der Stufe 2 etwa 18 bis etwa 30 h beträgt.

3. Verfahren nach Anspruch 1,
bei dem Stufe 2 in Anwesenheit eines Olivenöl Co-Oxidanten durchgeführt wird.

4. Verfahren zur Herstellung einer Hydroxycarbonsäure mit der Struktur: 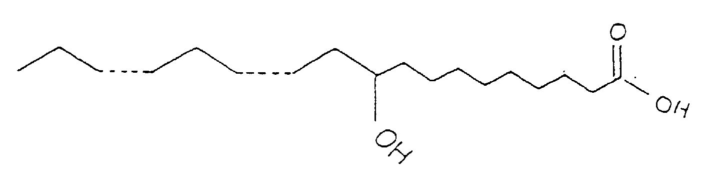 worin die gestrichelten Linien die gleichen oder verschiedenen Kohlenstoff-Kohlenstoff-Bindungen bedeuten und jede der gestrichelten Linien eine Kohlenstoff-Kohlenstoff-Einfachbindung oder eine Kohlenstoff-Kohlenstoff-Doppelbindung bedeutet, bestehend aus der Stufe des Fermentierens bei einem pH von etwa 5,5 bis 9,0 bei einer Temperatur von etwa 20 °C bis etwa 35 °C mindestens eines Carbonsäurederivats der folgenden Struktur ausgewählt aus der Gruppe bestehend aus 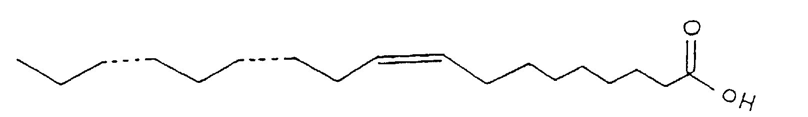 und 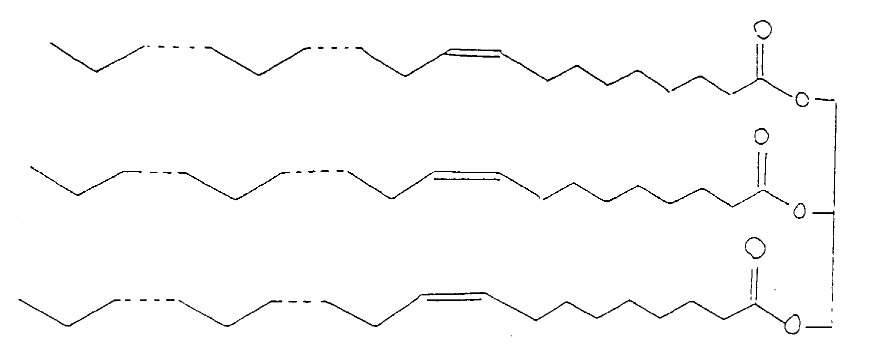 mit dem Mikroorganismus Pseudomonas sp. NRRL B-2994 oder eines 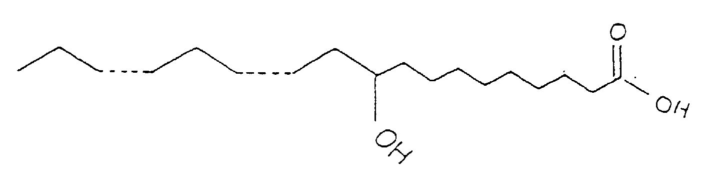 produzierenden Mutanten davon während einer solchen Zeitspanne, daß eine oder mehrere Hydroxycarbonsäuren der folgenden Struktur 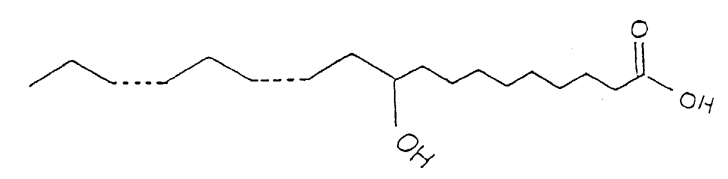 gemäß der Umsetzung 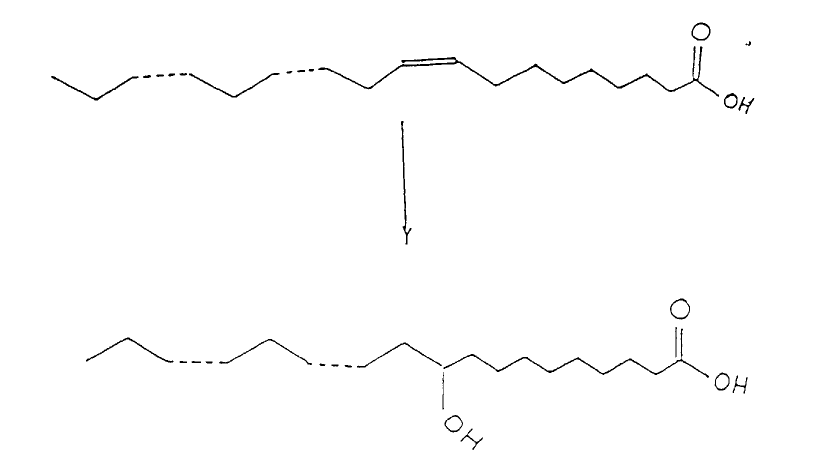 und/oder 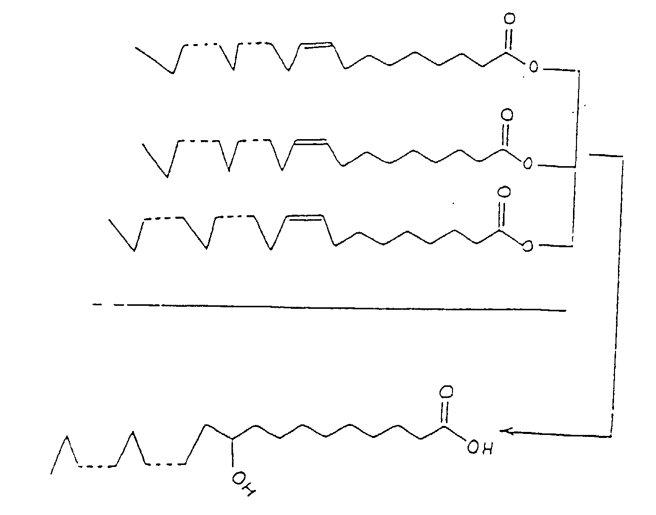 erhalten werden.

5. Verfahren nach Anspruch 4, bei dem die Reaktionszeit etwa 36 bis 150 h beträgt.

6. Verfahren nach Anspruch 1, bei dem es sich bei dem in Stufe 2 eingesetzten Mikroorganismus um Yarrowia lipolytica ATCC 34088 handelt.

7. Verfahren nach Anspruch 1, bei dem es sich bei dem in Stufe 2 eingesetzten Mikroorganismus um Candida petrophilum ATCC 20226 handelt.

8. Verfahren nach Anspruch 4, bei dem das eingesetzte Carbonsäurederivat folgende Struktur besitzt:

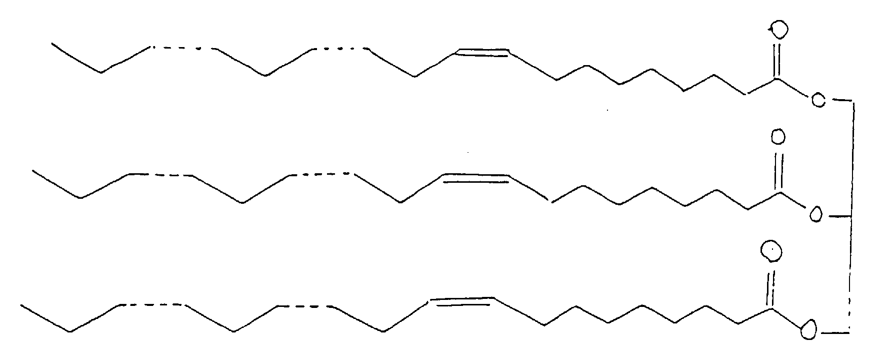

9. Verfahren nach Anspruch 8, bei dem das Carbonsäurederivat in Form des Sonnenblumenöls eingesetzt wird.

10. Verfahren nach Anspruch 4, bei dem das als Reaktant eingesetzte Carbonsäurederivat folgende Struktur besitzt:

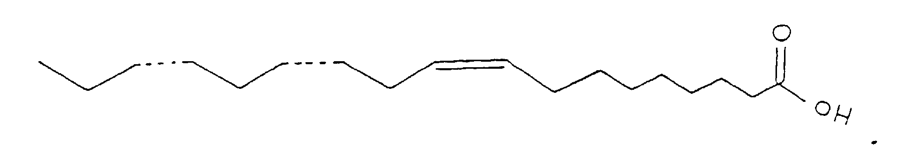

11. Verfahren nach Anspruch 10, bei dem bei der Struktur 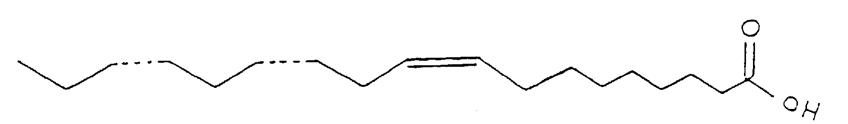 beide gestrichelten Linien Kohlenstoff-Kohlenstoff-Einfachbindungen bedeuten.

12. Verfahren nach Anspruch 10, bei dem das eingesetzte Carbonsäurederivat eine Mischung aus Ölsäure und Linolsäure ist.

13. Verfahren nach Anspruch 10, bei dem das eingesetzte Carbonäurederivat eine Mischung aus Ölsäure, Linolsäure und Linolensäure ist.

14. Verfahren nach Anspruch 1, bei dem das in der Stufe 1 als Reaktant eingesetzte Carbonsäurederivat Ölsäure ist.

15. Verfahren nach Anspruch 1, bei dem das in der Stufe 1 als Reaktant eingesetzte Carbonsäurederivat eine Mischung aus Ölsäure und Linolsäure ist.

16. Verfahren nach Anspruch 1, bei dem das in Stufe 1 eingesetzte Carbonsäurederivat eine Mischung aus Ölsäure, Linolsäure und Linolensäure ist.

17. Zusammensetzung enthaltend Lipase und Pseudomonas sp. NRRL B 2994.

## Revendications

1. Procédé pour la préparation d'un ou plusieurs dérivés de gamma dodécalactone définis conformément à la structure: 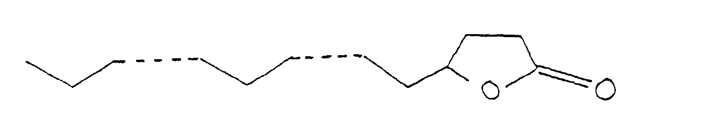 dans laquelle les lignes en traits interrompus représentent des liaisons carbone-carbone identiques ou différentes, chaque ligne en traits interrompus représentant une liaison simple carbone-carbone ou une liaison double carbone-carbone, composé des étapes consistant:
(i) à faire fermenter, à un pH dans l'intervalle d'environ 5,5 jusqu'à environ 9,0 et à une température dans l'intervalle d'environ 20°C jusqu'à environ 35°C, au moins un dérivé d'acide carboxylique défini conformément à une structure choisie dans le groupe consistant en ; 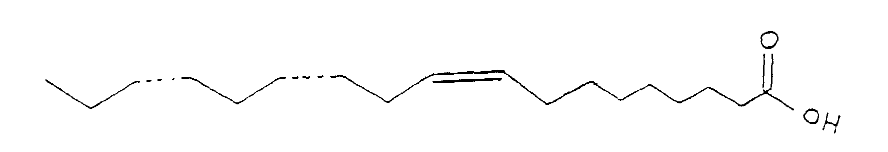 et en 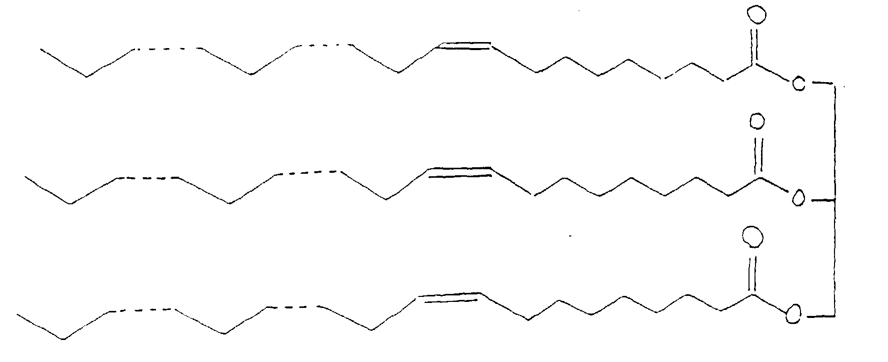 avec le microorganisme, Pseudomonas sp. NRRLR-2994 ou avec un mutant de celui-ci, producteur de: 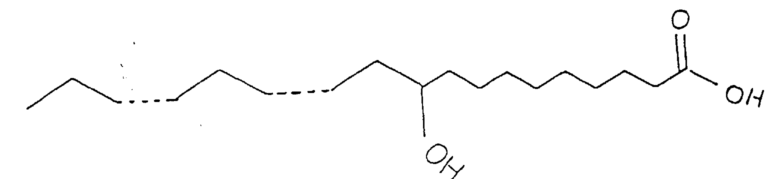 pendant une période de temps suffisante pour produire un ou plusieurs acides hydroxy carboxyliques définis conformément à la structure: 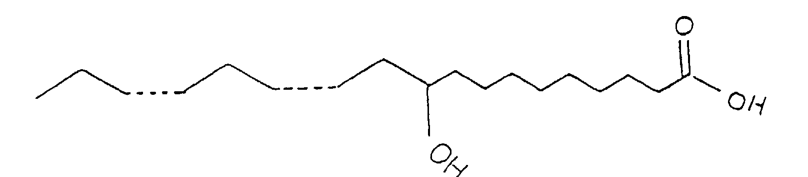 conformément aux réactions: 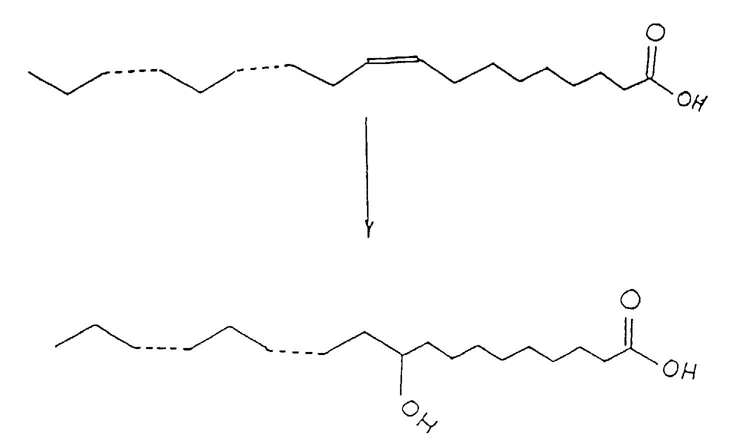 et/ou 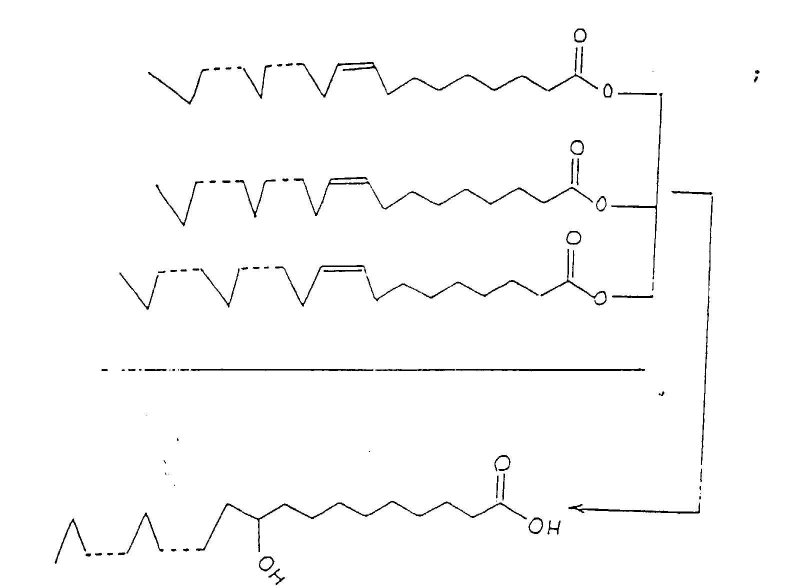 et
(ii) à faire fermenter au moins un des acides hydroxy carboxyliques définis conformément à la structure: 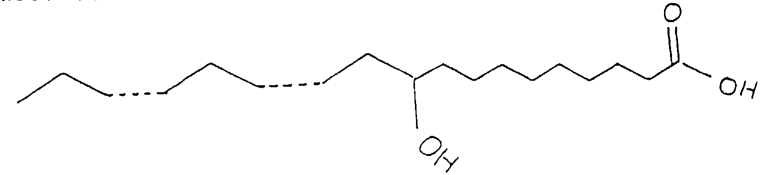 à un pH d'environ 5,0 jusqu'à environ 7,5 à une température dans l'intervalle d'environ 20°C jusqu'à environ 35°C, avec un microorganisme choisi dans le groupe composé de:
Candida brumptii IFO 0731;
Candida deformans ATCC 22969;
Candida ethanothermophilum ATCC 20380;
Candida iberica ATCC 26318;
Candida kefyr ATCC 42265;
Candida oleophila ATCC 20177;
Candida rugosa ATCC 14830;
Candida sp. ATCC 20473;
Candida sp. ATCC 20504;
Candida utilis ATCC 9226;
Candida utilis ATCC 15239;
Candida utilis ATCC 20248;
Candida utilis ATCC 42181;
Candida zeylanoides ATCC 15585;
Yarrowia lipolytica ATCC 8661;
Yarrowia lipolytica ATCC 8662;
Yarrowia lipolytica ATCC 16617;
Yarrowia lipolytica ATCC 16618;
Yarrowia lipolytica ATCC 20255;
Yarrowia lipolytica ATCC 20324;
Yarrowia lipolytica ATCC 20341;
Yarrowia lipolytica ATCC 46483;
Candida petrophilum ATCC 20226; et
Yarrowia lipolytica ATCC 34088
ou avec un mutant de celui-ci, producteur de: 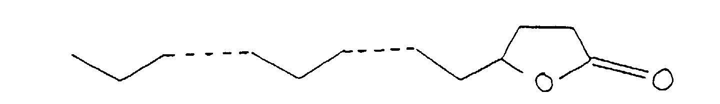 pendant une période de temps suffisante pour produire une ou plusieurs lactones définies conformément à la structure: 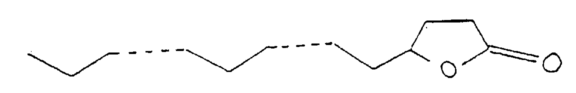 conformément à la réaction:

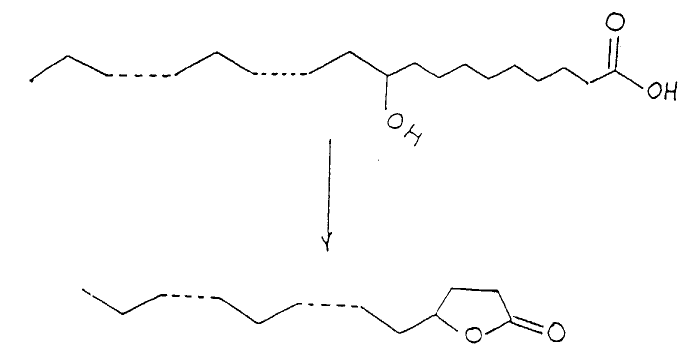

2. Procédé de la revendication 1 dans lequel la durée de réaction pour l'étape 1 est d'environ 36 heures à environ 150 heures et la durée de réaction pour l'étape 2 est d'environ 18 heures à environ 30 heures.

3. Procédé de la revendication 1 dans lequel l'étape 2 est effectuée en présence d'un co-oxydant d'huile d'olive.

4. Procédé de préparation d'un acide hydroxy carboxylique ayant la structure: 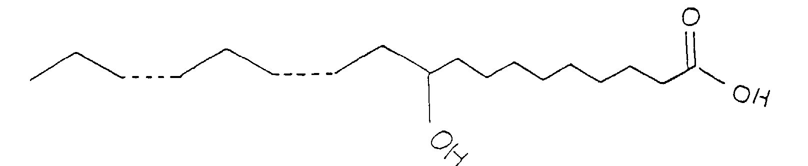 dans lequel les lignes en traits interrompus représentent des liaisons carbone-carbone identiques ou différentes, chacune des lignes en traits interrompus représentant une liaison simple carbone-carbone ou une liaison double carbone-carbone, consistant en l'étape dans laquelle on fait fermenter, à un pH dans l'intervalle d'environ 5,5 jusqu'à environ 9,0 et à une température d'environ 20°C jusqu'à environ 35°C, au moins un dérivé d'acide carboxylique défini conformément à une structure choisie dans le groupe consistant en: 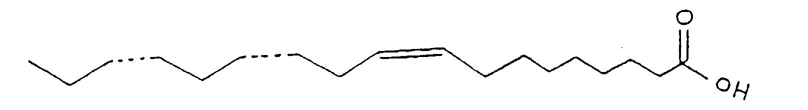 et en 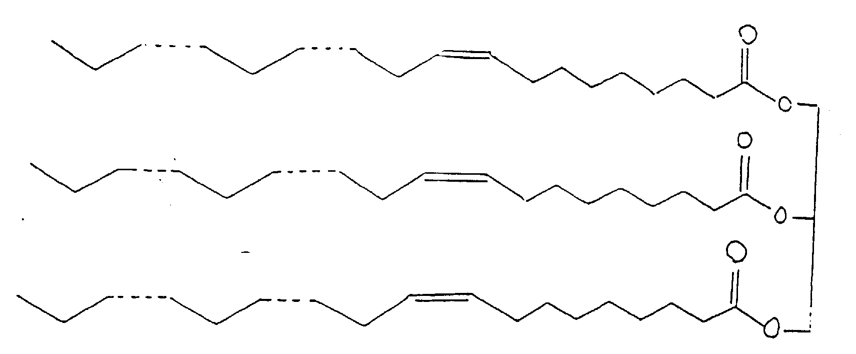 avec le microorganisme, Pseudomonas sp. NRRL B-2994 ou avec un mutant de celui-ci, producteur de: 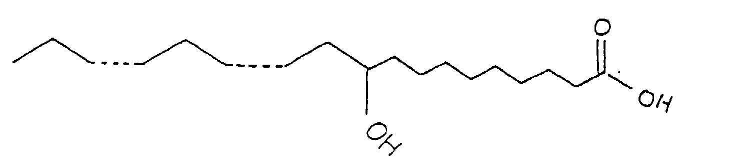 pendant une période de temps suffisante pour produire un ou plusieurs acides hydroxy carboxyliques définis conformément à la structure: 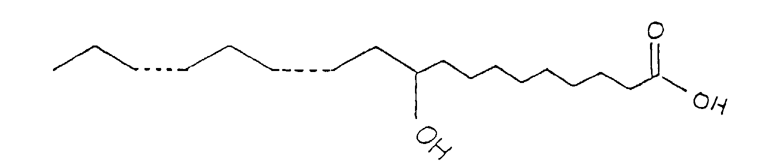 conformément à la réaction: 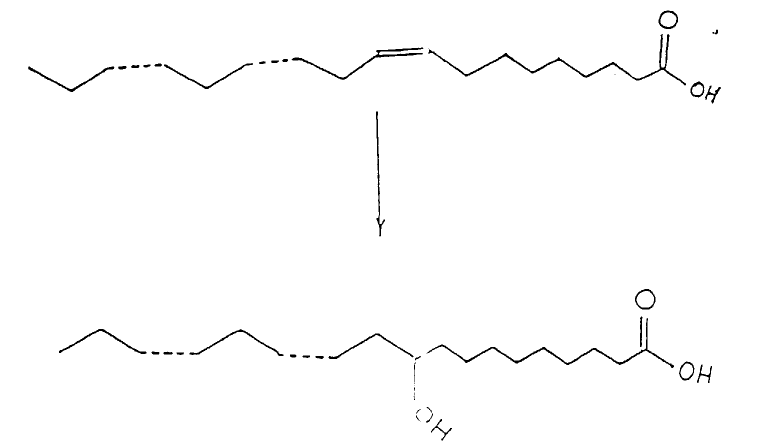 et/ou à la réaction:

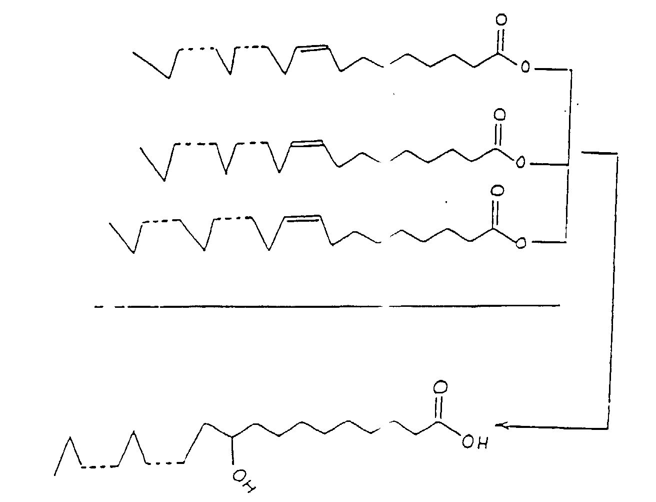

5. Procédé de la revendication 4 dans lequel la durée de réaction est d'environ 36 heures à environ 150 heures.

6. Procédé de la revendication 1 dans lequel le microorganisme utilisé dans l'étape 2 est le Yarrowia lipolytica ATCC34088.

7. Procédé de la revendication 1 dans lequel, dans l'étape 2, le microorganisme utilisé est le Candida petrophilum ATCC20226.

8. Procédé de la revendication 4 dans lequel le dérivé d'acide carboxylique utilisé possède la structure:

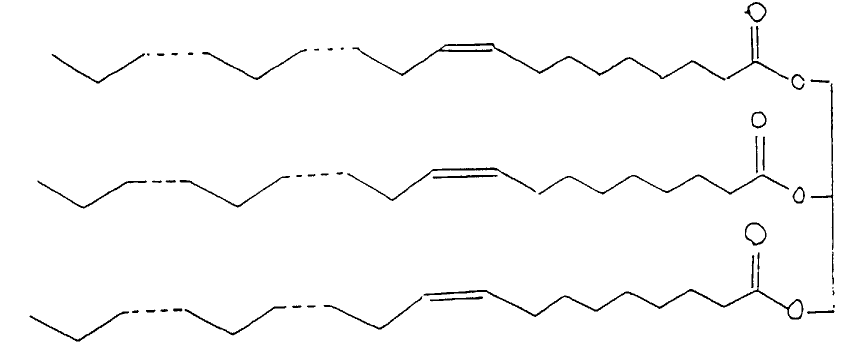

9. Procédé de la revendication 8 dans lequel le dérivé d'acide carboxylique est utilisé sous la forme d'huile de tournesol.

10. Procédé de la revendication 4 dans lequel le dérivé d'acide carboxylique utilisé comme réactant est défini conformément à la structure:

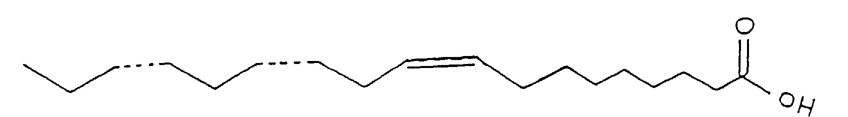

11. Procédé de la revendication 10 dans lequel, dans la structure: 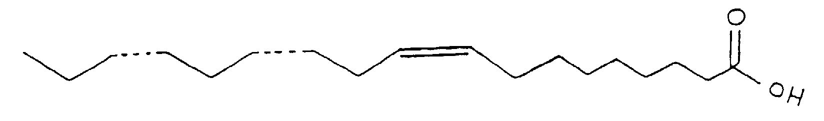 les deux lignes en traits interrompus sont des liaisons simples carbone-carbone.

12. Procédé de la revendication 10 dans lequel le dérivé d'acide carboxylique utilisé. est un mélange d'acide oléique et d'acide linoléique.

13. Procédé de la revendication 10 dans lequel le dérivé d'acide carboxylique utilisé est un mélange d'acide oléique, d'acide linoléique et d'acide linolénique.

14. Procédé de la revendication 1 dans lequel, dans l'étape 1, le dérivé d'acide carboxylique utilisé comme réactant est l'acide oléique.

15. Procédé de la revendication 1 dans lequel, dans l'étape 1, le dérivé d'acide carboxylique utilisé comme réactant est un mélange d'acides oléique et linoléique.

16. Procédé de la revendication 1 dans lequel, dans l'étape 1, le dérivé d'acide carboxylique utilisé est un mélange d'acide oléique, d'acide linoléique et d'acide linolénique.

17. Produit dont la composition comprend de la Lipase et un Pseudomonas sp. NRRL B-2994.
